# FASCICULE DE BREVET EUROPEEN

(11) **EP 1 179 078 B1**
(45) Date de publication et mention de la délivrance du brevet: **30.03.2016**
(21) Numéro de dépôt: 00929649.2
(22) Date de dépôt: 19.05.2000
(51) Int. Cl.: C12N 15/82, C12N 9/10, C12N 15/54, C12N 15/62, C12Q 1/68, C12N 1/21, A01H 5/00

(54) **GRAINS D'AMIDON CONTENANT UN POLYPEPTIDE RECOMBINANT D'INTERET, LEUR PROCEDE D'OBTENTION, ET LEURS UTILISATIONS**
STÄRKEKÖRNER, WELCHE EIN REKOMBINANTES POLYPEPTID NACH WAHL ENTHALTEN, VERFAHREN ZU DEREN GEWINNUNG UND ANWENDUNG
STARCH GRANULES CONTAINING A RECOMBINANT POLYPEPTIDE OF INTEREST, METHOD FOR OBTAINING SAME AND USES

(30) Priorité: 21.05.1999 FR 9906494
(43) Date de publication de la demande: 13.02.2002
(73) Titulaire: CENTRE NATIONAL DE LA RECHERCHE SCIENTIFIQUE -CNRS-, 75794 Paris Cedex 16 (FR)
(72) Inventeur: D'HULST, Christophe, F-59150 Wattrelos (FR); BALL, Steven, F-59830 Bourghelles (FR)
(74) Mandataire: Grosset-Fournier, Chantal Catherine
(86) Numéro de dépôt international: PCT/FR2000/001384
(87) Numéro de publication internationale: WO 2000/071734

(56) Documents cités:
- WO-A-97/45545
- WO-A-98/14601
- ISSHIKI M. ET AL.: "A naturally occurring functional allele of the rice waxy locus has a GT to mutation at the 5' splice site of the first intron." PLANT J 1998 JUL;15(1):133-, XP002130227
- DATABASE SWISSPROT [en ligne] 1 août 1998 (1998-08-01) D'HULST, C., ET AL. : "cloning of the cDNA encoding for the GBSSI in the green algae Chlamydomonas reinhardtii" XP002146121
- DATABASE EMBL SEQUENCE LIBRARY [en ligne] 2 juin 1998 (1998-06-02) D'HULST, C., ET AL. : "cloning of the cDNA encoding for the GBSSI in the green algae Chlamydomonas reinhardtii" XP002146137
- DELRUE B ET AL: "WAXY CHLAMYDOMONAS REINHARDTII: MONOCELLULAR ALGAL MUTANTS DEFECTIVE IN AMYLOSE BIOSYNTHESIS AND GRANULE-BOUND STARCH SYNTHASEACTIVITY ACCUMULATE A STRUCTURALLY MODIFIED AMYLOPECTIN" JOURNAL OF BACTERIOLOGY,US,WASHINGTON, DC, vol. 174, no. 11, 1 juin 1992 (1992-06-01), pages 3612-3620, XP000673929 ISSN: 0021-9193 cité dans la demande
- CHEN, L., ET AL.: "IMPROVED ADSORPTION TO STARCH OF A BETA-GALACTOSIDASE FUSION PROTEIN CONTAINING THE STARCH-BINDING DOMAIN FROM ASPERGILLUS GLUCOAMYLASE" BIOTECHNOLOGY PROGRESS, vol. 7, 1991, pages 225-229, XP002056940
- KUSNADI, A.R., ET AL.: "FUNCTIONAL STARCH-BINDING DOMAIN OF ASPERGILLUS GLUCOAMYLASE I IN ESCHERICHIA COLI" GENE, vol. 127, 1993, pages 193-197, XP002056413
- MU-FORSTER, C., ET AL .: "PHYSICAL ASSOCIATION OF STARCH BIOSYNTHETIC ENZYMES WITH STARCH GRANULES OF MAIZE ENDOSPERM" PLANT PHYSIOLOGY, vol. 111, 1996, pages 821-829, XP002056414
- DAUVILLEE D. ET AL.: "Novel, starch-like polysaccharides are synthesized by an unbound form of granule-bound starch synthase in glycogen-accumulating mutants of chlamydomonas reinhardtii." PLANT PHYSIOL 1999 JAN;119(1):321-30, XP000879046
- MADDELEIN ML. ET AL.: "toward an understanding of the biogenesis of the starch granule - determination of the granule-bound and soluble starch synthase functions in amylopectin synthesis" THE JOURNAL OF BIOLOGICAL CHEMISTRY, vol. 269, no. 40, 1994, pages 25150-25157, XP002130229 cité dans la demande

## Description

La présente invention a pour objet des grains d'amidon contenant un polypeptide recombinant d'intérêt, leur procédé d'obtention, ainsi que leurs utilisations, notamment dans des compositions pharmaceutiques.

L'amidon représente l'une des sources les plus importantes de polysaccharides présents sur terre, et peut notamment être trouvé dans les plantes (maïs, pomme de terre, blé, riz, orge...), les algues, les microalgues etc. L'amidon se présente sous forme de grains insolubles dans l'eau, dont la taille peut varier de 0,1 à plusieurs dizaines de µm de diamètre selon l'origine (plantes, algues ou microalgues) ou encore le génotype du végétal considéré. Ainsi, les tailles de ces grains varient de 0,1 µm de diamètre à plus de 50 µm de diamètre. De même, les degrés de cristallinité de ces grains oscillent entre 0% (pour les grains riches en amylose) à plus de 30%. Il existe trois à quatre types cristallins (A, B, C, V). Le grain croît par apposition de couches successivement amorphes et semi-cristallines à partir du centre du grain d'amidon.

L'amidon renferme plusieurs fractions polysaccharidiques distinctes, composées de glucanes liés en α-1,4 et ramifiés en α-1,6. Plus particulièrement, l'amidon est constitué de deux polymères de glucose : l'amylose d'une part, fraction minoritaire du grain (environ 20 à 30 % en poids), de faible poids moléculaire, modérément ramifié (< 1 % de liaisons α-1,6) et l'amylopectine d'autre part, fraction majoritaire du grain (70 à 80 % en poids), de haut poids moléculaire et fortement ramifiée (5 % de liaisons α-1,6). L'amylose n'est pas nécessaire à l'édification de la cristallinité du grain d'amidon ; on sait aujourd'hui que c'est l'amylopectine qui est responsable de la cristallinité du grain d'amidon.

Au plan biologique, l'amidon *sensu stricto* n'est retrouvé que dans le règne végétal, et, plus spécifiquement, dans les chloroplastes ou dans les plastes non photosynthétiques de la cellule végétale eucaryote. Deux types d'amidon peuvent être synthétisés par les végétaux : l'amidon transitoire ou photosynthétique (dont la synthèse se déroule au niveau des chloroplastes), et, l'amidon de réserve (dont la synthèse se déroule au niveau des amyloplastes).

La synthèse de l'amidon chez les plantes fait intervenir toute une panoplie d'enzymes impliquées dans la biosynthèse du précurseur ADP-glucose, l'échafaudage des molécules d'amylose et d'amylopectine et enfin, la dégradation du grain d'amidon.

La première étape de la biosynthèse de l'amidon est la production du précurseur ADP-glucose qui fait intervenir deux enzymes : la phosphoglucomutase (PGM) et l'ADP-glucose pyrophosphorylase (AGPase).

La deuxième étape de la biosynthèse du grain d'amidon fait également intervenir deux types d'enzymes, principalement impliquées dans la synthèse de l'amylose et de l'amylopectine : les amidon-synthétases (ou adénosine diphosphate glucose α-1,4-glucane α-4-glucosyltransférases) et les enzymes de branchement (ou α-1,4-glucane-6-glucosyltransférases). Les amidon-synthétases catalysent le transfert du résidu de glucose de l'ADP-glucose sur des chaînes de glucanes en élongation en créant une liaison O-glycosidique de type α-1,4. Puis, les enzymes de branchement hydrolysent une liaison α-1,4 d'un glucane en élongation, et soudent ensuite le fragment ainsi libéré sur le reste du glucane par l'intermédiaire d'une liaison α-1,6.

En ce qui concerne la dégradation de l'amidon, il existe deux familles majeures d'enzymes de dégradation : les enzymes hydrolytiques (hydrolases) d'une part, telles que les α-amylases (endomylases), les β-amylases (exomylases), les γ-amylases (amyloglucosidases), les D-enzymes (glucosyltransférases), les R-enzymes (enzymes de débranchement), les α-glucosidases (maltases), et, d'autre part, les enzymes phosphorolytiques (ou amidon-phosphorylases).

Plusieurs isoformes d'amidon-synthétases coexistent chez les plantes supérieures. La distinction majeure entre ces isoformes tient en leur caractère soluble (à savoir qu'elles sont en solution dans le stroma plastidial des plantes) ou lié au grain d'amidon.

Les amidon-synthétases liées au grain d'amidon (ou GBSS pour Granule Bound Starch Synthases) sont retrouvées en étroite association avec l'amidon. Plusieurs isoformes de GBSS ont été isolées chez le maïs, le pois, la pomme de terre ou encore le blé (*MacDonald and Preiss, 1985 ; Smith, 1990 ; Dry et al., 1992 ; Denyer et al., 1995*). Dans tous les cas, c'est la GBSSI qui représente l'isoforme majeure ; le rôle tenu par cette isoforme dans la biogenèse du grain d'amidon est la formation d'amylose (*Tsai, 1974 ; Hovenkamp-Hermelink et al., 1987 ; Delrue et al.,1992 ; Denyer et al.,* 1995). Une mutation aux loci WX des céréales, *AMF* de la pomme de terre, *LAM* du pois, associe la disparition de la GBSSI à un effondrement total de la fraction amylosique de l'amidon. Un ADNc correspondant à la « protéine Waxy » (par abus de langage, on utilise le terme « protéine Waxy »pour désigner la GBSSI chez les plantes, la distinguant ainsi des autres GBSS) a été isolé chez le blé, l'orge, le maïs, le riz, la pomme de terre et le pois. Les comparaisons des séquences protéiques relatives montrent une homologie très grande entre les diverses espèces (*Ainsworth et al., 1993*).

La GBSSI n'est pas la seule amidon-synthétase liée au grain d'amidon. D'autres isoformes sont retrouvées liées au grain d'amidon chez le pois, la pomme de terre, le maïs ou encore le blé (*Smith, 1990 ; Dry et al., 1992 ; Mu et al., 1994 ; Denyer et al., 1995*). Cependant, les rôles tenus par chacune de ces isoformes ne sont pas clairs jusqu'à présent. De plus, la plupart d'entre elles sont aussi retrouvées dans la phase soluble.

Les amidon-synthétases solubles (ou SS pour Soluble Starch Synthases) ne sont pas liées au grain d'amidon, mais sont retrouvées sous forme soluble dans le stroma plastidial des plantes. De même que pour les formes liées, de multiples formes d'amidon-synthétases solubles sont présentes chez les végétaux supérieurs. Ainsi, on a par exemple pu détecter trois isoformes d'amidon-synthétases solubles (SSI, SSII et SSIII) dans le tubercule de pomme de terre.

Des ADNc correspondants aux différentes formes d'amidon-synthétases solubles ont été clonés chez les végétaux supérieurs (*Baba et al., 1993 ; Dry et al, 1992 ; Edwards et al*., *1995 ; Abel et al*., *1996 ; Marshall et al*., *1996 ; Gao et al., 1998*). La comparaison des séquences qui en découle, montre clairement la présence de trois régions hautement conservées à travers les isoformes, que ce soit au sein d'une même espèce ou entre les espèces de plantes supérieures.

Des travaux de recherche récents menés par les Inventeurs ont permis d'établir que la l'amidon-synthétase soluble II (SSII) de *Chlamydomonas reinhardtii* est principalement impliquée dans la formation des cristaux de la molécule d'amylopectine.

En revanche, la GBSSI n'intervient pas dans l'édification des cristaux de l'amylopectine. L'activité GBSSI n'a jamais été détectée en phase soluble. La GBSSI est intimement associée au grain d'amidon. Cependant, contrairement à l'amylase, aucun motif de liaison à l'amidon n'a été mis en évidence dans les séquences de GBSSI décrites jusqu'à présent. Ainsi on ne connaît pas le mécanisme régissant la liaison de la GBSSI au granule d'amidon.

Les amidon-synthétases suscitent un intérêt particulier dans la mesure où ces enzymes pourraient permettre de véhiculer un peptide recombinant d'intérêt vers les plastes où a lieu la biosynthèse des grains d'amidon. Ainsi la transformation de plantes avec des séquences codant pour des peptides de fusion entre une amidon-synthétase et un peptide d'intérêt, permettrait d'obtenir des grains d'amidon en grande quantité à partir desquels ledit peptide d'intérêt pourrait être récupéré.

C'est dans cet objectif que les auteurs de la demande internationale WO 98/14601 (Exseed Genetics), ont décrit des séquences nucléotidiques codant des protéines de fusion dans lesquelles le polypeptide d'intérêt est lié à l'extrémité aminoterminale d'une amidon-synthétase choisie dans le groupe constitué par les amidon-synthétases solubles I, II et III (SSI, SSII, SSIII), les amidons-synthétases liées au grain (GBSS), les enzymes de branchement I, IIa et IIBb et les glucoamylases. Toutefois aucun procédé de transformation de plantes à l'aide des séquences décrites dans cette demande, et donc d'obtention de grains d'amidon transformés par lesdites séquences, n'est illustré de façon détaillée.

La présente invention découle de la mise en évidence par les inventeurs du fait que seule la transformation de plantes avec des séquences nucléotidiques codant des polypeptides de fusion dans lesquels le polypeptide d'intérêt est lié à l'extrémité carboxyterminale de l'amidon-synthétase, permet d'obtenir des grains d'amidon contenant ledit peptide d'intérêt.

Un des buts de la présente invention est de fournir de nouvelles séquences nucléotidiques codant des protéines de fusion capables de véhiculer un peptide d'intérêt vers le site de biosynthèse des grains d'amidon dans les cellules végétales (y compris les cellules d'algues ou de micro-algues).

Un autre but de la présente invention est de fournir des plantes transformées à l'aide des séquences nucléotidiques susmentionnées, lesdites plantes produisant de l'amidon contenant un polypeptide d'intérêt.

Un autre but de la présente invention est de fournir des grains d'amidon contenant un polypeptide d'intérêt.

Un autre but de la présente invention est de fournir un procédé de préparation de ces grains d'amidon.

Un autre but de la présente invention est de fournir un procédé de préparation d'un polypeptide recombinant d'intérêt à partir de ces grains d'amidon.

Un autre but de la présente invention est de fournir, des compositions notamment alimentaires ou pharmaceutiques, contenant les grains d'amidon susmentionnés.

Un autre but de la présente invention est de fournir un procédé de biotransformation des grains d'amidon lorsque ledit peptide d'intérêt utilisé est capable de transformer l'amidon.

L'invention sera illustrée dans ce qui suit à l'aide des figures suivantes :
- Figure 1 : ADNc codant pour la partie carboxyterminale de la GBSSI de *Chlamydomonas reinhardtii*,
- Figure 2 : ADNc codant pour la GBSSI de *Chlamydomonas reinhardtii,* et séquence peptidique de la GBSSI de *Chlamydomonas reinhardtii.* .

La présente invention a pour objet l'utilisation d'une séquence nucléotidique recombinante insérée dans un vecteur recombinant en un site non essentiel pour sa réplication, comprenant, dans le sens 5'→3', une séquence nucléotidique codant pour une adénosine diphosphate glucose α-1, 4-glucane α-4-glucosyltransférase ou amidon-synthétase EC 2.4.1.21, ou pour une protéine dérivée de cette enzyme, par suppression, ou substitution d'un ou plusieurs acides aminés, la séquence nucléotidique de ladite protéine dérivée ayant une homologie d'au moins 60% avec la séquence nucléotidique susmentionnés, ladite enzyme ou protéine dérivée ayant la propriété de migrer vers les sites de biosynthèse des grains d'amidon dans les cellules végétales et de s'associer aux grains d'amidon,
ladite séquence nucléotidique étant choisie parmi les séquences SEQ ID NO 6 ou SEQ ID NO 8,
ladite séquence, codant pour l'enzyme ou protéine susmentionnée, étant placée en amont d'une séquence nucléotidique codant pour un peptide ou polypeptide d'intérêt, pour la transformation de cellules végétales dans le cadre de la mise en oeuvre d'un procédé de préparation de grains d'amidon contenant un polypeptide de fusion codé par ladite séquence nucléotidique recombinante, ledit polypeptide de fusion comprenant l'enzyme ou protéine susmentionnée en position N-terminale et ledit peptide ou polypeptide d'intérêt en position C-terminale.

Dans un mode de réalisation avantageux, l'invention concerne l'utilisation précédente, pour la mise en oeuvre d'un procédé de préparation du polypeptide de fusion tel que défini ci-dessus, à partir des grains d'amidon contenant ledit polypeptide de fusion.

Dans un mode de réalisation avantageux, l'invention concerne l'utilisation précédente, pour la mise en oeuvre d'un procédé de préparation d'un peptide ou polypeptide d'intérêt à partir du polypeptide de fusion tel que défini précédement.

Dans un autre mode de réalisation avantageux, l'invention concerne l'utilisation précédente, pour la préparation d'une composition pharmaceutique comprenant des grains d'amidon contenant des polypeptides de fusion tels que définis ci-dessus, ou d'une composition pharmaceutique contenant des polypeptides de fusion tels que définis ci-dessus, lesdits grains d'amidon ou polypeptides de fusion étant le cas échéant en association avec un véhicule physiologiquement acceptable, le peptide ou polypeptide d'intérêt dans lesdits polypeptides de fusion possédant un effet thérapeutique déterminé.

Dans encore un autre mode de réalisation avantageux, l'invention concerne l'utilisation précédente, pour la préparation d'une composition alimentaire comprenant les grains d'amidon contenant des polypeptides de fusion tels que définis ci-dessus, le peptide ou polypeptide d'intérêt dans lesdits polypeptides de fusion étant utilisable dans le domaine agroalimentaire.

La présente invention est basée sur les résultats obtenus des inventeurs, permettant d'obtenir toute séquence nucléotidique recombinante caracterisee en ce qu'elle comprend, dans le sens 5'→3', une séquence nucléotidique codant pour une adénosine diphosphate glucose α-1,4-glucane α-4-glucosyltransférase ou amidon-synthétase (EC 2.4.1.21), ou pour une protéine dérivée de cette enzyme, notamment par suppression, addition ou substitution d'un ou plusieurs acides aminés, ladite amidon-synthétase ou protéine dérivée ayant la propriété de migrer vers les sites de biosynthèse des grains d'amidon dans les cellules végétales et de s'associer aux grains d'amidon, ladite séquence nucléotidique codant pour l'enzyme ou protéine susmentionnée étant placée en amont d'une séquence nucléotidique codant pour un peptide ou polypeptide d'intérêt.

Par amidon-synthétase, on entend dans ce qui précède et ce qui suit, toute protéine ayant la propriété de migrer vers les sites de biosynthèse des grains d'amidon dans les cellules végétales et de s'associer aux grains d'amidon, que cette amidon-synthétase ait conservé ou non son activité enzymatique au sein du polypeptide de fusion, codé par une séquence nucléotidique recombinante susmentionnée, entre ladite amidon-synthétase et ledit polypeptide d'intérêt.

De préférence, la séquence nucléotidique codant pour une amidon-synthétase, ou pour une protéine dérivée telle que définie ci-dessus, est choisie parmi celles codant pour une amidon-synthétase liée au grain d'amidon GBSS présente notamment chez les plantes, algues ou micro-algues, et plus avantageusement encore pour une isoforme GBSSI, ou pour une protéine dérivée de cette GBSS, ou GBSSI, telle que définie ci-dessus.

L'invention est aussi basée sur les résultats obtenus par les inventeurs, permettant d'obtenir toute séquence nucléotidique recombinante telle que define ci-dessus, caractérisée en ce que la séquence nucléotidique codant pour une amidon-synthétase, et plus particulièrement pour une GBSS, notamment pour une GBSSI, est telle qu'obtenue par criblage d'une banque d'ADNc préparée à partir de cellules susceptibles de contenir cette enzyme, notamment de cellules de plantes, algues ou micro-algues, à l'aide d'un antisérum contenant des anticorps reconnaissant spécifiquement ladite amidon-synthétase codée par un ou plusieurs ADNc de la banque, lorsque ladite amidon-synthétase est exprimée par un vecteur de clonage approprié, ledit antisérum étant obtenu par immunisation d'un animal, tel que le lapin, avec de l'amidon extrait des cellules susmentionnées.

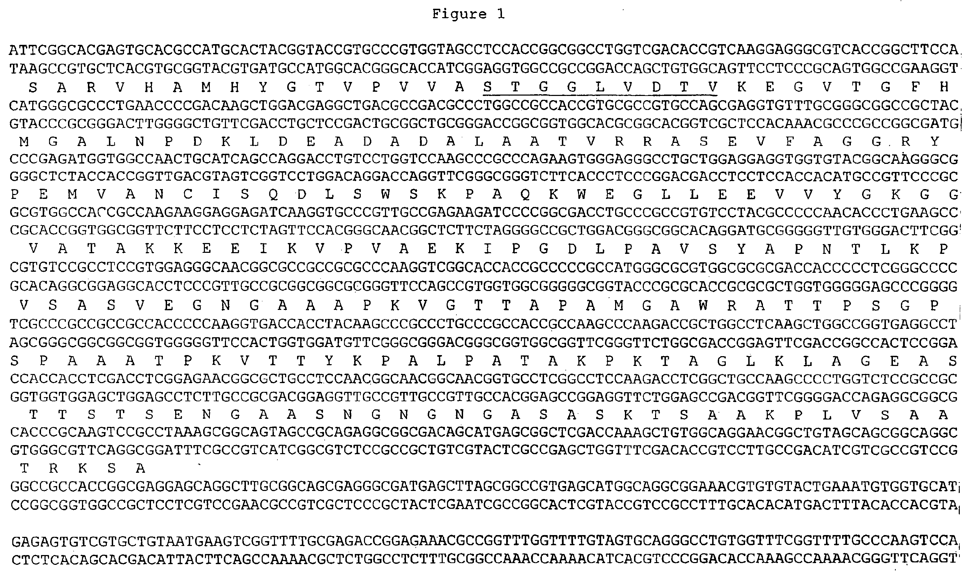

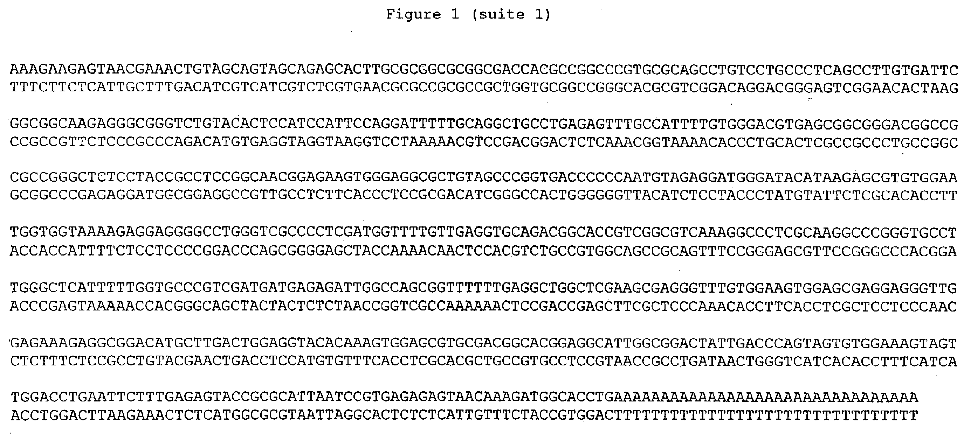

Plus particulièrement toute séquence nucléotidique recombinante telle que définie ci-dessus, caractérisée en ce que la séquence nucléotidique codant pour une amidon-synthétase, ou pour une protéine dérivée, est choisie parmi :
- la séquence nucléotidique de l'ADNc d'environ 2900 à 3100 paires de bases, et dont les 1696 paires de bases de l'extrémité 3' sont représentés sur la figure 1, ladite séquence nucléotidique :
   codant pour la GBSSI de *Chlamydomonas reinhardtii* d'environ 640 à 680 acides aminés, notamment d'environ 660 acides aminés, dont l'extrémité aminoterminale correspond à l'enchaînement d'acides aminés suivant ALDIVMVAAEVAPGGKTGGLGDV, ou ALDIVMVAAEVAPWSKTGGLGDV, et dont l'extrémité carboxyterminale correspond à l'enchaînement d'acides aminés représenté sur la figure 1,
   . et étant obtenue par criblage d'une banque d'ADNc préparée à partir de cellules de *Chlamydomonas reinhardtii*, à l'aide d'un antisérum obtenu par immunisation de lapins avec de l'amidon extrait des cellules susmentionnées de *Chlamydomonas reinhardtii*,
- ou un fragment nucléotidique de l'ADNc susmentionné, codant pour un fragment peptidique de la GBSSI de *Chlamydomonas reinhardtii*, ledit fragment peptidique comprenant l'intégralité de la partie aminoterminale de ladite GBSSI, et étant délimité à son extrémité carboxyterminale par l'acide aminé situé en l'une des positions 25 à 238, ou en l'une des positions 118 à 238, de la séquence en acides aminés représentée sur la figure 1,
- ou une séquence nucléotidique dérivée par dégénérescence du code génétique de la séquence nucléotidique de l'ADNc susmentionné, ou d'un fragment nucléotidique susmentionné de cette dernière, et codant pour la GBSSI de *Chlamydomonas reinhardtii* susmentionnée, ou pour un fragment peptidique susmentionné de cette dernière,
- ou une séquence nucléotidique dérivée d'une séquence ou d'un fragment nucléotidique susmentionnés, notamment par substitution, suppression ou addition d'un ou plusieurs nucléotides, et codant une séquence peptidique dérivée de la GBSSI de *Chlamydomonas reinhardtii* susmentionnée, ou dérivée d'un fragment peptidique susmentionné de cette dernière, et ayant la propriété de s'associer aux grains d'amidon, ladite séquence nucléotidique dérivée ayant de préférence une homologie d'au moins environ 50 %, et de préférence d'au moins environ 70 %, avec la séquence ou fragment nucléotidique susmentionnés,
- ou une séquence nucléotidique susceptible d'hybrider avec une des séquences ou fragments nucléotidiques susmentionnés, notamment dans les conditions stringentes d'hybridation définies ci-après,
la propriété que possède une amidon-synthétase, ou un fragment ou une protéine dérivée de cette dernière tels que définis ci-dessus, de pouvoir s'associer aux grains d'amidon, pouvant être mesurée selon la méthode suivante : extraction des protéines des grains d'amidon, par exemple selon le procédé détaillé ci-après, et détection de la
la propriété que possède une amidon-synthétase, ou un fragment ou une protéine dérivée de cette dernière tels que définis ci-dessus, de pouvoir s'associer aux grains d'amidon, pouvant être mesurée selon la méthode suivante : extraction des protéines des grains d'amidon, par exemple selon le procédé détaillé ci-après, et détection de la présence de ladite amidon-synthétase, ou d'un fragment ou d'une protéine dérivée de cette dernière tels que définis ci-dessus, notamment par électrophorèse en gel de polyacrylamide selon la méthode détaillée ci-après.

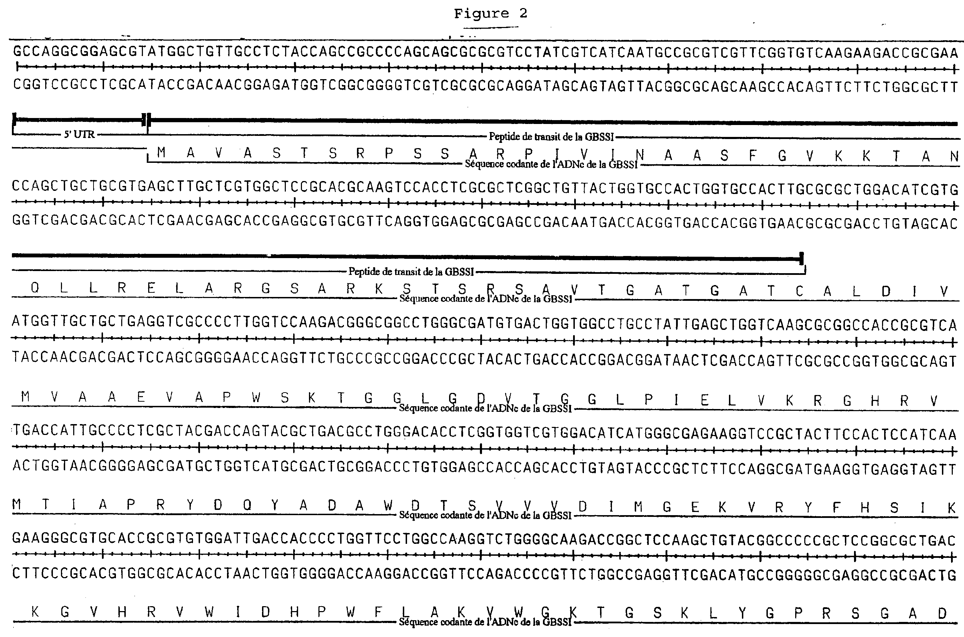

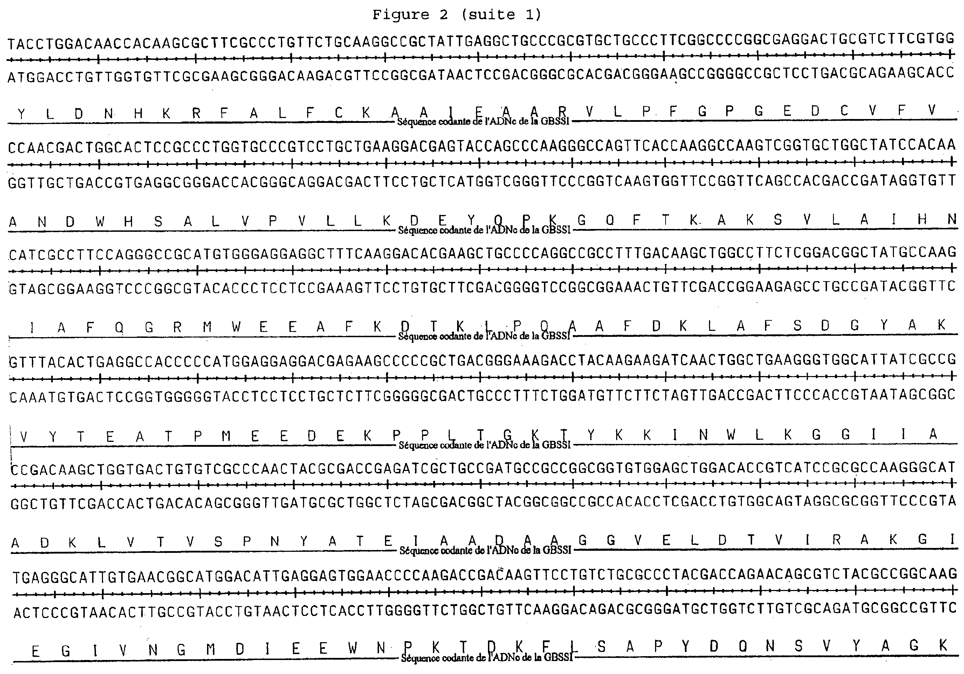

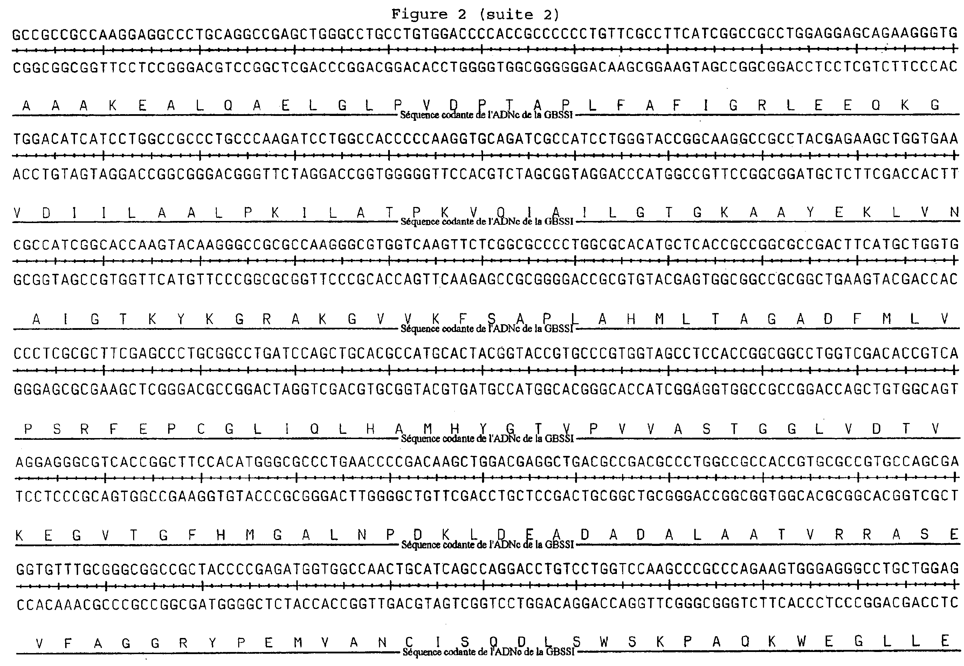

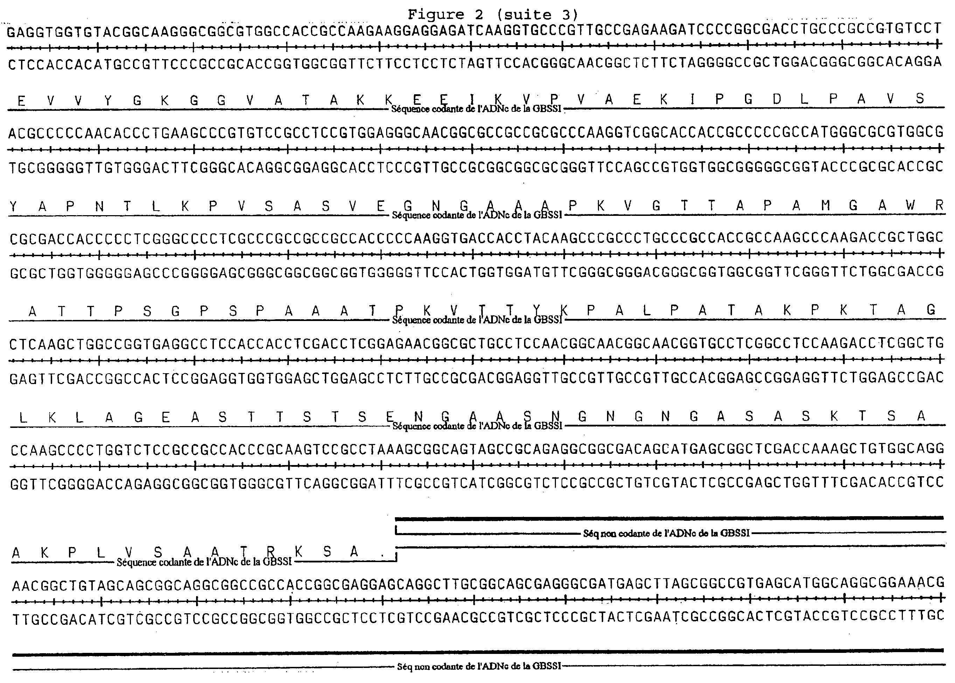

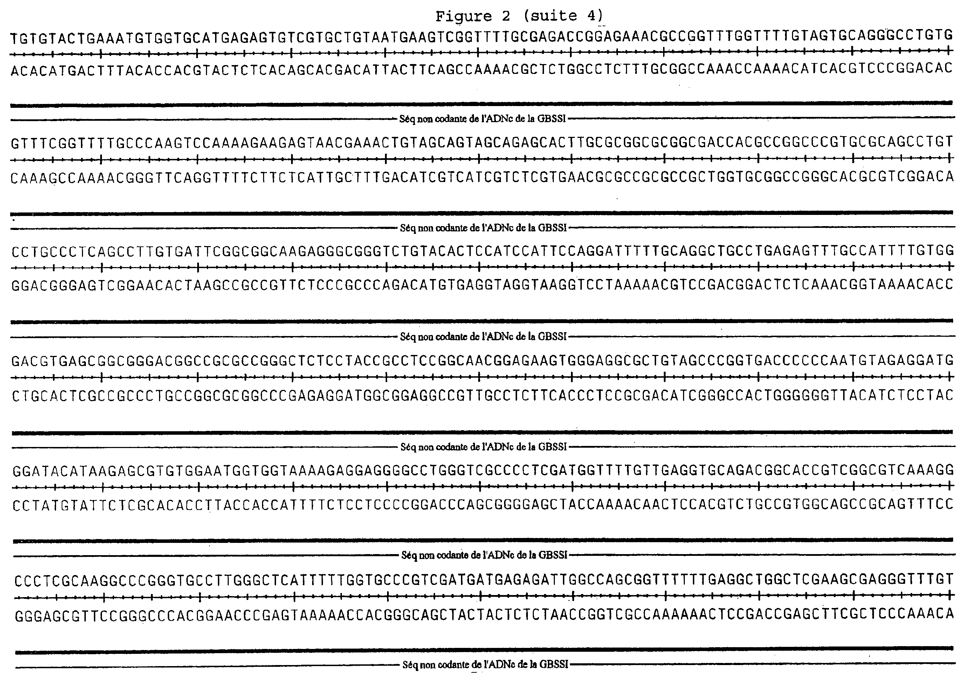

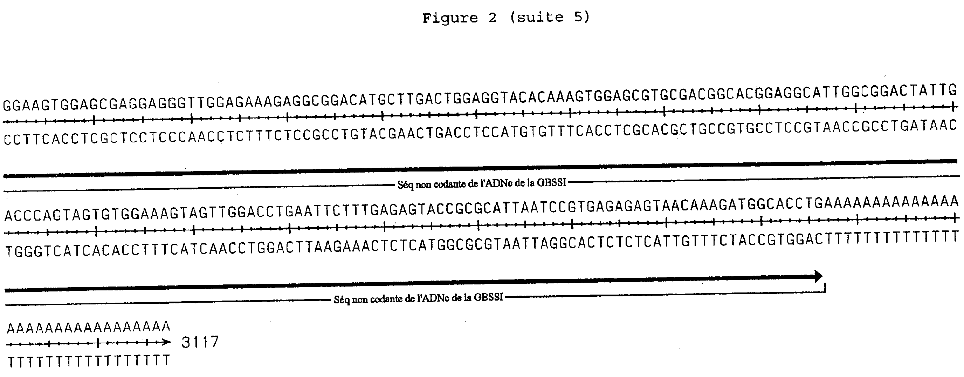

L'invention est basée sur les résultats obtenus par les inventeurs, permettant d'obtenir toute séquence nucleotidique recombinante telle que define ci-dessus, caractérisée en ce que la séquence nucléotidique susmentionnée codant pour une amidon-synthétase, ou pour une protéine dérivée, est plus particulièrement choisie parmi :
- la séquence nucléotidique de l'ADNc représenté sur la figure 2, correspondant à SEQ ID NO : 1 dans la liste de séquences ci-après, ladite séquence nucléotidique codant pour la GBSSI de *Chlamydomonas reinhardtii*,
- tout fragment tel que défini ci-dessus de la séquence nucléotidique SEQ ID NO : 1 représentée sur la figure 2, et plus particulièrement toute séquence dont le nucléotide de l'extrémité 5' correspond à celui situé en l'une des positions 1 à 186 de SEQ ID NO : 1, et dont le nucléotide de l'extrémité 3' correspond à celui situé en l'une des positions 1499 à 3117 de SEQ ID NO : 1, notamment :
   - la séquence SEQ ID NO : 2 délimitée par les nucléotides situés aux positions 15 à 2138 de SEQ ID NO : 1, codant pour la GBSSI de *Chlamydomonas reinhardtii* sous forme de pré-protéine de 708 acides aminés (SEQ ID NO : 3) délimitée par les acides aminés situés aux positions 1 et 708 de la séquence peptidique représentée sur la figure 2,
   - la séquence SEQ ID NO : 4 délimitée par les nucléotides situés aux positions 186 à 2138 de SEQ ID NO : 1, codant pour la GBSSI de *Chlamydomonas reinhardtii* sous forme de protéine mature de 651 acides aminés (SEQ ID NO : 5) délimitée par les acides aminés situés aux positions 58 et 708 de la séquence peptidique représentée sur la figure 2,
   - la séquence SEQ ID NO : 6 délimitée par les nucléotides situés aux positions 186 à 1499 de SEQ ID NO : 1, codant pour un fragment de 438 acides aminés (SEQ ID NO : 7) délimité par les acides aminés situés aux positions 58 et 495 de la séquence peptidique de la GBSSI de *Chlamydomonas reinhardtii* représentée sur la figure 2,
   - la séquence SEQ ID NO : 8 délimitée par les nucléotides situés aux positions 186 à 1778 de SEQ ID NO : 1, codant pour un fragment de 531 acides aminés (SEQ ID NO : 9) délimité par les acides aminés situés aux positions 58 et 588 de la séquence peptidique de la GBSSI de *Chlamydomonas reinhardtii* représentée sur la figure 2,
- ou une séquence nucléotidique dérivée par dégénérescence du code génétique des séquences nucléotidiques susmentionnées, et codant pour la GBSSI de *Chlamydomonas reinhardtii* susmentionnée, ou pour un fragment peptidique susmentionné de cette dernière,
- ou une séquence nucléotidique dérivée d'une séquence ou d'un fragment nucléotidique susmentionnés, notamment par substitution, suppression ou addition d'un ou plusieurs nucléotides, et codant une séquence peptidique dérivée de la GBSSI de *Chlamydomonas reinhardtii* susmentionnée, ou dérivée d'un fragment peptidique susmentionné de cette dernière, et ayant la propriété de s'associer aux grains d'amidon, ladite séquence nucléotidique dérivée ayant de préférence une homologie d'au moins environ 50 %, et de préférence d'au moins environ 70 %, avec la séquence ou fragment nucléotidique susmentionnés,
- ou une séquence nucléotidique susceptible d'hybrider avec une des séquences ou fragments nucléotidiques susmentionnés, notamment dans les conditions stringentes d'hybridation définies ci-dessus.

L'invention concerne toute séquence nucléotidique recombinante insérée dans un vecteur recombinant en un site non essentiel pour sa réplication, caractérisée en ce qu'elle comprend, dans le sens 5'→3', une séquence nucléotidique codant pour une adénosine diphosphate glucose α-1, 4-glucane α-4-glucosyltransférase ou amidon-synthétase EC 2.4.1.21, ladite enzyme ayant la propriété de migrer vers les sites de biosynthèse des grains d'amidon dans les cellules végétales et de s'associer aux grains d'amidon, ladite séquence nucléotidique codant pour l'enzyme ou protéine susmentionnée étant placée en amont d'une séquence nucléotidique codant pour un peptide ou polypeptide d'intérêt, la dite séquence recombinante étant choisie parmi :
- un fragment correspondant à:
   - la séquence SEQ ID NO: 6 codant pour un fragment de 438 acides aminés (SEQ ID NO : 7) de la GBSSI de *Chlamydomonas reinhardtii,* ou
   - la séquence SEQ ID NO : 8 codant pour un fragment de 531 acides aminés (SEQ ID NO : 9) de la GBSSI de *Chlamydomonas reinhardtii*,
- ou une séquence nucléotidique dérivée par dégénérescence du code génétique des séquences nucléotidiques correspondant à la séquence SEQ ID NO : 6 ou SEQ ID NO : 8, et codant pour la GBSSI de *Chlamydomonas reinhardtii* susmentionnée, ou pour un fragment peptidique susmentionné de cette dernière, ayant la propriété de s'associer au grain d'amidon,
- ou une séquence nucléotidique dérivée d'une séquence ou d'un fragment nucléotidique correspondant à la séquence SEQ ID NO : 6 ou SEQ ID NO : 8, par substitution ou suppression d'un ou plusieurs nucléotides, et codant une séquence peptidique dérivée de la GBSSI de *Chlamydomonas reinhardtii* susmentionnée, et ayant la propriété de s'associer aux grains d'amidon, ladite séquence nucléotidique dérivée ayant une homologie d'au moins 70%, avec la séquence ou fragment nucléotidique susmentionnés.

L'invention a plus particulièrement pour objet toute séquence nucléotidique recombinante telle que définie ci-dessus, caractérisée en ce que la séquence nucléotidique codant pour un peptide ou polypeptide d'intérêt est choisie parmi celles codant des peptides biologiquement actifs, notamment des peptides d'intérêt thérapeutique ou utilisables dans le domaine agroalimentaire.

L'invention concerne également toute séquence nucléotidique recombinante telle que définie ci-dessus, caractérisée en ce que la séquence nucléotidique codant pour un peptide ou polypeptide d'intérêt est choisie parmi celles codant des enzymes susceptibles de transformer l'amidon, telles que les enzymes interagissant avec les α-glucanes dont les hydrolases diverses, les phosphorylases, les α-1,4 glucanotransérases, les enzymes de branchement, les amylases, et notamment les hydrolases thermostables issues de bactéries extrémophiles telles que les archaebactéries actives à des températures supérieures à 40 °C.

L'invention a également pour objet toute séquence nucléotidique recombinante telle que définie ci-dessus, caractérisée en ce qu'elle comprend une séquence nucléotidique codant pour un site de clivage, ladite séquence nucléotidique étant placée entre la séquence nucléotidique codant pour une amidon-synthétase, ou une protéine dérivée de cette dernière, et la séquence nucléotidique codant le polypeptide d'intérêt.

A titre d'illustration, la séquence nucléotidique codant pour un site de clivage est choisie parmi les séquences codant pour une séquence peptidique de type aspartyl-proline très labile en pH acide, ou codant pour une petite séquence peptidique reconnue spécifiquement par une protéase, telles que la trypsine, la chymotrypsine, la pepsine, la collagénase, la thrombine, l'alasubtilisine, ou reconnue par des composés chimiques tel que le bromure de cyanogène.

L'invention a également pour objet toute séquence nucléotidique recombinante telle que définie ci-dessus, caractérisée en ce qu'elle comprend un promoteur situé en amont de la séquence nucléotidique codant pour une amidon-synthétase, ou une protéine dérivée de cette dernière, ainsi qu'une séquence codant pour des signaux de terminaison de la transcription située en aval de la séquence nucléotidique codant le polypeptide d'intérêt.

Parmi les promoteurs de transcription susceptibles de pouvoir être utilisés dans le cadre de la présente invention, on peut citer :
- pour les promoteurs procaryotes, les promoteurs Lac ou T7,
- pour les promoteurs eucaryotes de type végétaux supérieurs, le promoteur 35S CaMV, ou tout type de promoteur d'origine végétale,
- dans le cas de la transformation de microalgues, le promoteur utilisé peut être celui du gène *ARG7* codant l'arginosuccinate lyase ou encore le promoteur du gène *NIT1* codant la nitrate réductase.

L'invention a également pour objet tout vecteur recombinant, notamment du type plasmide, cosmide ou phage, caractérisé en ce qu'il contient une séquence nucléotidique recombinante selon l'invention telle que définie ci-dessus, insérée en un site non essentiel pour sa réplication.

L'invention concerne également tout hôte cellulaire, transformé par un vecteur recombinant tel que défini ci-dessus, notamment toute bactérie telle que *Agrobacterium tumefaciens,* et comprenant au moins une séquence nucléotidique recombinante selon l'invention telle que définie ci-dessus, insérée en un site non essentiel pour sa réplication.

L'invention concerne également tout hôte cellulaire, transformé par un vecteur recombinant tel que défini ci-dessus, notamment toute bactérie telle que *Agrobacterium tumefaciens,* et comprenant au moins une séquence nucléotidique recombinante selon l'invention.

L'invention concerne également tout polypeptide de fusion caractérisé en ce qu'il comprend :
- en position N-terminale une amidon-synthétase, ou une protéine dérivée de cette enzyme, notamment par suppression, addition ou substitution d'un ou plusieurs acides aminés, ladite amidon-synthétase ou protéine dérivée ayant la propriété de migrer vers les sites de biosynthèse des grains d'amidon dans les cellules végétales et de s'associer aux grains d'amidon,
- et, en position C-terminale, un peptide ou polypeptide d'intérêt,
la partie C-terminale de la séquence en acides aminés de l'amidon-synthétase, ou de la protéine dérivée, étant ainsi liée à la partie N-terminale de la séquence peptidique d'intérêt, ledit polypeptide de fusion étant codé par une séquence nucléotidique recombinante telle que définie ci-dessus selon l'invention.

Particulièrement les inventeurs ont décrit tout polypeptide de fusion tel que défini ci-dessus, caractérisé en ce qu'il comprend en position N-terminale une GBSS présente notamment chez les plantes, algues ou micro-algues, et plus particulièrement une isoforme GBSSI, ou une protéine dérivée de cette dernière telle que définie ci-dessus.

L'invention repose sur la caractérisation par les Inventeurs de tout polypeptide de fusion tel que défini ci-dessus, caractérisé en ce que l'amidon-synthétase est choisie parmi :
- la GBSSI de *Chlamydomonas reinhardtii* d'environ 640 à 680 acides aminés, dont l'extrémité aminoterminale correspond à l'enchaînement d'acides aminés suivant: ALDIVMVAAEVAPGGKTGGLGDV, ou ALDIVMVAAEVAPWSKTGGLGDV, et l'extrémité carboxyterminale correspond à l'enchaînement d'acides aminés représenté sur la figure 1, ladite GBSSI étant codée par la séquence nucléotidique obtenue par criblage d'une banque d'ADNc préparée à partir de cellules de *Chlamydomonas reinhardtii,* à l'aide d'un antisérum obtenu par immunisation de lapins avec de l'amidon extrait des cellules susmentionnées de *Chlamydomonas reinhardtii*,
- ou un fragment peptidique de la GBSSI de *Chlamydomonas reinhardtii,* ledit fragment peptidique comprenant l'intégralité de la partie aminoterminale de ladite GBSSI, et étant délimité à son extrémité carboxyterminale par l'acide aminé situé en l'une des positions 25 à 238, ou en l'une des positions 118 à 238, de la séquence en acides aminés représentée sur la figure 1,
- ou une séquence peptidique dérivée d'une séquence ou d'un fragment peptidique susmentionnés, notamment par substitution, suppression ou addition d'un ou plusieurs acides aminés, et ayant la propriété de s'associer aux grains d'amidon, ladite séquence peptidique dérivée ayant de préférence une homologie d'au moins environ 60%, avantageusement d'au moins environ 80%, avec la séquence ou fragment peptidique susmentionnés,
la propriété que possède la GBSSI de *Chlamydomonas reinhardtii*, ou un fragment ou une protéine dérivée de cette dernière tels que définis ci-dessus, de pouvoir s'associer aux grains d'amidon, pouvant être mesurée selon la méthode décrite ci-dessus.

Plus particulièrement, les inventeurs ont décrit tout polypeptide de fusion tel que défini ci-dessus, caractérisé en ce que l'amidon-synthétase définie ci-dessus, est plus particulièrement choisie parmi :
- la séquence peptidique SEQ ID NO : 3 délimitée par les acides aminés situés aux positions 1 à 708 de la figure 2, correspondant à la GBSSI de *Chlamydomonas reinhardtii* sous forme de pré-protéine de 708 acides aminés,
- tout fragment tel que défini ci-dessus de la séquence peptidique SEQ ID NO : 3 représentée sur la figure 2, et plus particulièrement toute séquence dont l'acide aminé de l'extrémité aminoterminale correspond à celui situé en l'une des positions 1 à 58 de SEQ ID NO : 3, et dont l'acide aminé de l'extrémité carboxyterminale correspond à celui situé en l'une des positions 495 à 708 de SEQ ID NO : 3, notamment :
   - la séquence SEQ ID NO : 5 délimitée par les acides aminés situés aux positions 58 à 708 de SEQ ID NO : 3, correspondant à la GBSSI de *Chlamydomonas reinhardtii* sous forme de protéine mature de 651 acides aminés,
   - la séquence SEQ ID NO : 7 délimitée par les acides aminés situés aux positions 58 à 495 de SEQ ID NO : 3, correspondant à un fragment de 438 acides aminés de la séquence peptidique de la GBSSI de *Chlamydomonas reinhardtii* représentée sur la figure 2,
   - la séquence SEQ ID NO : 9 délimitée par les acides aminés situés aux positions 58 à 588 de SEQ ID NO : 3, correspondant à un fragment de 531 acides aminés de la séquence peptidique de la GBSSI de *Chlamydomonas reinhardtii* représentée sur la figure 2,
- ou une séquence peptidique dérivée d'une séquence ou d'un fragment peptidique susmentionnés, notamment par substitution, suppression ou addition d'un ou plusieurs acides aminés, et ayant la propriété de s'associer aux grains d'amidon, ladite séquence peptidique dérivée ayant de préférence une homologie d'au moins environ 60%, avantageusement d'au moins environ 80%, avec la séquence ou fragment peptidique susmentionnés,
la propriété que possède la GBSSI de *Chlamydomonas reinhardtii*, ou un fragment ou une protéine dérivée de cette dernière tels que définis ci-dessus, de pouvoir s'associer aux grains d'amidon, pouvant être mesurée selon la méthode décrite ci-dessus.

L'invention concerne plus particulièrement tout polypeptide de fusion défini précédemment, caractérisé en ce que l'amidon-synthétase est choisie parmi :
- un polypeptide choisi parmi:
   - la séquence SEQ ID NO : 7 correspondant à un fragment de 438 acides aminés de la séquence peptidique de la GBSSI de *Chlamydomonas reinhardtii,*
   - la séquence SEQ ID NO : 9 correspondant à un fragment de 531 acides aminés de la séquence peptidique de la GBSSI de *Chlamydomonas reinhardtii*,
- ou une séquence peptidique dérivée d'une séquence ou d'un fragment peptidique correspondant à la séquence SEQ ID NO: 7 ou SEQ ID NO : 9, par substitution ou suppression d'un ou plusieurs acides aminés, et ayant la propriété de s'associer aux grains d'amidon, ladite séquence peptidique dérivée ayant une homologie d'au moins environ 60%, avantageusement d'au moins environ 80%, avec la séquence ou fragment peptidique correspondant à la séquence SEQ ID NO : 7 ou SEQ ID NO : 9.

L'invention a plus particulièrement pour objet tout polypeptide de fusion tel que défini ci-dessus, caractérisé en ce que le polypeptide d'intérêt est choisi parmi les peptides biologiquement actifs, notamment les peptides d'intérêt thérapeutique ou utilisables dans le domaine agroalimentaire.

L'invention concerne également tout polypeptide de fusion tel que défini ci-dessus, caractérisé en ce que le polypeptide d'intérêt est choisi parmi les enzymes susceptibles de transformer l'amidon, telles que les enzymes interagissant avec les α-glucanes dont les hydrolases diverses, les phosphorylases, les α-1,4 glucanotransférases, les enzymes de branchement, les amylases, et notamment les hydrolases thermostables issues de bactéries extrémophiles telles que les archaebactéries actives à des températures supérieures à 40 °C.

L'invention a également pour objet tout polypeptide de fusion tel que défini ci-dessus, caractérisé en ce qu'il comprend un site de clivage, tel que décrit ci-dessus, placé entre d'une part l'amidon-synthétase, ou une protéine dérivée de cette dernière, et, d'autre part, le polypeptide d'intérêt.

L'invention concerne également les cellules végétales transformées génétiquement, contenant une ou plusieurs séquences nucléotidiques recombinantes telles que décrites ci-dessus, intégrées dans leur génome ou maintenues de manière stable dans leur cytoplasme, lesdites cellules végétales étant choisies parmi les cellules de plantes, d'algues ou de micro-algues, capables de fabriquer de l'amidon.

L'invention vise également les cellules végétales transgéniques telles que décrites ci-dessus contenant un ou plusieurs polypeptides de fusion définis ci-dessus au sein des grains d'amidon contenus dans lesdites cellules végétales.

L'invention a plus particulièrement pour objet les cellules végétales transgéniques susmentionnées, transformées avec une séquence nucléotidique recombinante contenant la séquence nucléotidique de l'ADNc d'environ 2900 à 3100 paires de bases, et dont les 1696 paires de bases de l'extrémité 3' sont représentées sur la figure 1, ladite séquence nucléotidique codant pour la GBSSI de *Chlamydomonas reinhardtii* décrite ci-dessus, ou contenant un fragment ou une séquence dérivée tels que décrits ci-dessus de l'ADNc susmentionné.

L'invention concerne plus particulièrement encore les cellules végétales transgéniques susmentionnées, transformées avec :
- la séquence nucléotidique de l'ADNc représenté sur la figure 2, ladite séquence nucléotidique codant pour la GBSSI de *Chlamydomonas reinhardtii,*
- tout fragment tel que défini ci-dessus de la séquence nucléotidique représentée sur la figure 2,
- ou une séquence nucléotidique dérivée, telle que définie ci-dessus, des séquences nucléotidiques susmentionnées,
- ou une séquence nucléotidique susceptible d'hybrider avec une des séquences ou fragments nucléotidiques susmentionnés, notamment dans les conditions stringentes d'hybridation définies ci-dessus.

L'invention a également pour objet les plantes, algues ou micro-algues transformées génétiquement, ou parties, notamment fleurs, fruits, feuilles, tiges, racines, semences, ou fragments de ces plantes, algues ou micro-algues, comprenant au moins une séquence nucléotidique recombinante telle que définie ci-dessus intégrée dans le génome ou maintenue de manière stable dans le cytoplasme des cellules les composant.

L'invention vise également les plantes, algues ou micro-algues transformées génétiquement, ou parties, ou fragments de ces plantes, algues ou micro-algues, tels que définis ci-dessus, contenant un ou plusieurs polypeptides de fusion tels que décrits ci-dessus au sein des grains d'amidon contenus dans les cellules végétales les composant.

Parmi les plantes, algues ou micro-algues transformées dans le cadre de la présente invention, on peut citer principalement le blé, le maïs, la pomme de terre, le riz, l'orge, l'amarante, les algues du genre *Chlamydomonas,* notamment *Chlamydomonas reinhardtii*, les algues du genre *Chlorella,* notamment *Chlorella vulgaris,* ou les algues unicellulaires du genre *Dunaliella* (telles que décrites dans l'ouvrage *"*Dunaliella : Physiology, Biochemistry, and Biotechnology (1992), Mordhay Avron and Ami Ben-Amotz Editeurs, CRC Press Inc., Boca Raton, Florida, USA").

L'invention a plus particulièrement pour objet les plantes, algues ou micro-algues transgéniques susmentionnées, transformées avec une séquence nucléotidique recombinante contenant la séquence nucléotidique de l'ADNc d'environ 2900 à 3100 paires de bases, et dont les 1696 paires de bases de l'extrémité 3' sont représentées sur la figure 1, ladite séquence nucléotidique codant pour la GBSSI de *Chlamydomonas reinhardtii* décrite ci-dessus, ou contenant un fragment ou une séquence dérivée tels que décrits ci-dessus de l'ADNc susmentionné.

L'invention concerne plus particulièrement encore les plantes, algues ou micro-algues transgéniques susmentionnées, transformées avec :
- la séquence nucléotidique de l'ADNc représenté sur la figure 2, ladite séquence nucléotidique codant pour la GBSSI de *Chlamydomonas reinhardtii*,
- tout fragment tel que défini ci-dessus de la séquence nucléotidique représentée sur la figure 2,
- ou une séquence nucléotidique dérivée, telle que définie ci-dessus, des séquences nucléotidiques susmentionnées,
- ou une séquence nucléotidique susceptible d'hybrider avec une des séquences ou fragments nucléotidiques susmentionnés, notamment dans les conditions stringentes d'hybridation définies ci-dessus.

L'invention concerne également les grains d'amidon caractérisés en ce qu'ils comprennent un ou plusieurs polypeptides de fusion définis ci-dessus, lesdits grains d'amidon étant encore désignés par l'expression "grains d'amidon transformés" ou "glucosomes".

L'invention a plus particulièrement pour objet les grains d'amidon susmentionnés comprenant un polypeptide de fusion défini ci-dessus, ledit polypeptide de fusion contenant la GBSSI de *Chlamydomonas reinhardtii* d'environ 640 à 680 acides aminés décrite ci-dessus, dont l'extrémité aminoterminale correspond à l'enchaînement d'acides aminés suivant : ALDIVMVAAEVAPGGKTGGLGDV, ou ALDIVMVAAEVAPWSKTGGLGDV, et l'extrémité carboxyterminale correspond à l'enchaînement d'acides aminés représenté sur la figure 1, ou un fragment ou un polypeptide dérivé tels que décrits ci-dessus de la GBSSI de *Chlamydomonas reinhardtii.*

L'invention concerne plus particulièrement les grains d'amidon susmentionnés comprenant un polypeptide de fusion défini ci-dessus, ledit polypeptide de fusion contenant la séquence délimitée par les acides aminés situés aux positions 1 à 708 de la figure 2, codant pour la GBSSI de *Chlamydomonas reinhardtii* sous forme de pré-protéine de 708 acides aminés, ou tout fragment tel que défini ci-dessus de la séquence peptidique représentée sur la figure 2, notamment toute séquence dont l'acide aminé de l'extrémité aminoterminale correspond à celui situé en l'une des positions 1 à 58 de la figure 2, et dont l'acide aminé de l'extrémité carboxyterminale correspond à celui situé en l'une des positions 495 à 708 de la figure 2, tel que les fragments mentionnés ci-dessus

Avantageusement, les grains d'amidon susmentionnés sont caractérisés en ce qu'ils ont un diamètre compris entre environ 0,1 µm et quelques dizaines de µm, et en ce que la proportion en poids des polypeptides de fusion dans ces grains est comprise entre environ 0,1 % et 1 %.

L'invention a également pour objet toute composition pharmaceutique caractérisée en ce qu'elle comprend des grains d'amidon transformés tels que définis ci-dessus, le cas échéant en association avec un véhicule physiologiquement acceptable, lesdits grains contenant un ou plusieurs polypeptides de fusion tels que définis ci-dessus, le peptide d'intérêt dans lesdits polypeptides de fusion possédant un effet thérapeutique déterminé.

Avantageusement les compositions pharmaceutiques susmentionnées de l'invention se présentent sous une forme administrable par voie parentérale, notamment par voie intraveineuse, ou sous une forme administrable par voie orale.

De préférence, les compositions pharmaceutiques susmentionnées administrables par voie parentérale, sont caractérisées en ce que le diamètre des grains d'amidon est compris entre environ 0,1 µm et plusieurs µm, notamment entre environ 0,1 µm et 10 µm, et en ce que la proportion en poids des polypeptides de fusion dans ces grains est comprise entre environ 0,1 % et 1 %.

Des grains d'amidon tels que décrits ci-dessus dont les faibles diamètres sont compris entre environ 0,1 µm et environ 10 µm, et dans lesquels la proportion en poids des polypeptides de fusion est comprise entre environ 0,1 % et 1 %, sont avantageusement obtenus :
- à partir de plantes ou cellules de plantes transformées dans le cadre de la présente invention et choisies pour leur propriété à produire naturellement les grains d'amidon susmentionnés, lesdites plantes étant choisies notamment parmi le riz, l'amarante,
- ou à partir de parties de plantes transformées dans le cadre de la présente invention, lesdites parties de ces plantes produisant naturellement les grains d'amidon susmentionnés, telles que les feuilles des plantes,
- ou à partir de plantes ou cellules de plantes transformées dans le cadre de la présente invention, ces plantes étant choisies parmi des plantes comportant des mutations telles qu'elles produisent des grains d'amidon de faibles diamètres susmentionnés, notamment à partir des plantes mutantes décrites dans Buléon A. et al., 1998,
- ou à partir de plantes ou cellules de plantes transformées dans le cadre de la présente invention, ces plantes étant choisies parmi des plantes transformées à l'aide de séquences nucléotidiques antisens de tout ou partie du gène codant pour l'ADP-glucose pyrophosphorylase nécessaire à la synthèse d'ADP-glucose dans les cellules végétales, notamment à partir des plantes transformées décrites dans l'article de Müller-Röber B. et al., 1992.

Avantageusement, dans le cas de compositions pharmaceutiques susmentionnées administrables par voie parentérale, les grains d'amidon sont de préférence choisis parmi ceux de structure amorphe dans le cas où l'on souhaite obtenir une libération rapide du polypeptide de fusion qu'ils contiennent dans le sang du patient, ou, au contraire parmi ceux de structure cristalline lorsque l'on souhaite libérer progressivement le polypeptide de fusion dans le sang.

A titre d'illustration, des grains d'amidon amorphes peuvent être obtenus à partir de semences transformées selon l'invention en phase de germination, ou à partir de plantes mutantes particulières telles que décrites par Shannon J. et Garwood D., 1984, notamment à partir des cultivars mutants tels que "amylose extender" du maïs ou encore tous les cultivars mutants de plantes, algues ou micro-algues dont l'amidon est enrichi en amylose.

Les grains d'amidon selon l'invention de structure cristalline, présentent avantageusement environ 30 à 35 % de cristaux, et peuvent être obtenus à partir de semences de plantes, notamment de céréales, venant d'être récoltées et à maturité, ou à partir de plantes mutantes telles que décrites par Shannon J. et Garwood D., 1984, notamment à partir des cultivars mutants tels que "waxy" du maïs ou encore tous les cultivars mutants de plantes, algues ou micro-algues dont l'amidon est dépourvu d'amylose.

L'invention a également pour objet toute composition pharmaceutique caractérisée en ce qu'elle comprend un ou plusieurs polypeptides de fusion tels que définis ci-dessus, le cas échéant en association avec un véhicule physiologiquement acceptable, le peptide d'intérêt dans lesdits polypeptides de fusion possédant un effet thérapeutique déterminé.

L'invention concerne également toute composition alimentaire caractérisée en ce qu'elle comprend des grains d'amidon transformés tels que définis ci-dessus, lesdits grains contenant un ou plusieurs polypeptides de fusion tels que définis ci-dessus, le peptide d'intérêt dans lesdits polypeptides de fusion étant utilisable dans le domaine agroalimentaire.

L'invention a également pour objet toute composition alimentaire telle que décrite ci-dessus, caractérisée en ce qu'elle comprend un ou plusieurs polypeptides de fusion tels que définis ci-dessus, le peptide d'intérêt dans lesdits polypeptides de fusion étant utilisable dans le domaine agroalimentaire.

La présente invention concerne également tout procédé d'obtention de cellules végétales (de plantes, algues ou micro-algues), et, le cas échéant de plantes, algues ou micro-algues entières, transformées par au moins une séquence nucléotidique telle que définie ci-dessus, caractérisé en ce qu'il comprend:
- la transformation de cellules végétales, de manière à intégrer dans le génome de ces cellules, ou à maintenir de manière stable dans leur cytoplasme, une ou plusieurs séquences nucléotidiques recombinantes selon l'invention, et mise en *culture in vitro* de ces cellules transformées,
- le cas échéant, l'obtention de plantes transformées à partir des cellules transformées susmentionnées.

Selon un mode de réalisation du procédé susmentionné de l'invention, la transformation de cellules végétales peut être réalisée par transfert de la séquence nucléotidique recombinante de l'invention dans les protoplastes, notamment après incubation de ces derniers dans une solution de polyéthylène glycol (PEG) en présence de cations divalents (Ca²⁺) selon la méthode décrite dans l'article de Krens *et al.,* 1982.

La transformation des cellules végétales peut également être réalisée par électroporation notamment selon la méthode décrite dans l'article de Fromm *et al.,* 1986.

La transformation des cellules végétales peut également être réalisée par utilisation d'un canon à gène permettant la projection, à très grande vitesse, de particules métalliques recouvertes de séquences nucléotidiques recombinantes selon l'invention, délivrant ainsi des gènes à l'intérieur du noyau cellulaire, notamment selon la technique décrite dans l'article de Sanford, 1988.

Une autre méthode de transformation des cellules végétales, est celle de la micro-injection cytoplasmique ou nucléaire telle que décrite dans l'article de De La Penna *et al.,* 1987.

Selon un mode de réalisation particulièrement préféré du procédé susmentionné de l'invention, les cellules végétales sont transformées par mise en présence de ces dernières avec un hôte cellulaire transformé par un vecteur selon l'invention, tel que décrit ci-dessus, ledit hôte cellulaire étant susceptible d'infecter lesdites cellules végétales en permettant l'intégration dans le génome ou le maintien de manière stable dans le cytoplasme de ces dernières, des séquences nucléotidiques recombinantes de l'invention initialement contenues dans le génome du vecteur susmentionné.

Avantageusement, l'hôte cellulaire susmentionné utilisé est *Agrobacterium tumefaciens,* notamment selon les méthodes décrites dans les articles de Bevan, 1984 et d'An *et al.,* 1986, ou encore *Agrobacterium rhizogenes,* notamment selon la méthode décrite dans l'article de Jouanin *et al.,* 1987.

Parmi les cellules végétales susceptibles d'être transformées dans le cadre de la présente invention, on peut citer principalement les cellules de blé, maïs, pomme de terre, riz, orge, amarante, *Chlamydomonas reinhardtii, Chorella vulgaris.*

Selon un mode de réalisation du procédé susmentionné de l'invention, les cellules végétales transformées selon l'invention, sont cultivées *in vitro,* notamment en bioréacteurs selon la méthode décrite dans l'article de Brodelius, 1988, en milieu liquide, ou selon la méthode décrite dans l'article de Brodelius *et al.,* 1979, sous forme immobilisées, ou encore selon la méthode décrite dans l'article de Deno *et al.,* 1987, procédant par culture de racines transformées *in vitro.*

Selon un mode de réalisation préféré du procédé susmentionné de l'invention, la transformation de cellules végétales est suivie par une étape d'obtention de plantes transformées par mise en culture desdites cellules transformées dans un milieu approprié, et, le cas échéant, fécondation et récupération des semences des plantes obtenues à l'étape précédente, et mise en culture de ces semences pour l'obtention de plantes de génération suivante.

Les semences transformées selon l'invention sont récoltées à partir de plantes transformées susmentionnées, ces plantes étant soit celles de la génération T0, à savoir celles obtenues à partir de culture de cellules transformées de l'invention sur un milieu approprié, soit avantageusement celles des générations suivantes (T1, T2 etc.) obtenues par autofécondation des plantes de la génération précédente et dans lesquelles les séquences nucléotidiques recombinantes de l'invention se reproduisent selon les lois de Mendel, ou les lois de l'hérédité extrachromosomique.

L'invention concerne également un procédé de préparation de grains d'amidon transformés tels que décrits ci-dessus, caractérisé en ce qu'il comprend une étape d'extraction des grains d'amidon à partir de cellules végétales transformées ou de plantes, ou de parties, notamment fleurs, fruits, feuilles, tiges, racines, ou de fragments de ces plantes, transformées susmentionnées, notamment par sédimentation dans les conditions décrites ci-après.

De préférence, les grains d'amidon selon l'invention sont tels qu'obtenus par extraction à partir de plantes, algues ou micro-algues, transformées décrites ci-dessus, ou à partir de parties, ou de fragments de ces plantes, algues ou micro-algues, définies ci-dessus, notamment par sédimentation dans les conditions décrites ci-après.

Les plantes transformées utilisées pour la récupération des grains d'amidon sont celles de la génération T0, ou avantageusement celles des générations suivantes (T1, T2 etc.) susmentionnées.

L'invention concerne également un procédé de préparation de polypeptides de fusion tels que définis ci-dessus, caractérisé en ce qu'il comprend une étape de récupération, et le cas échéant de purification, des polypeptides de fusion à partir des grains d'amidon transformés susmentionnés, notamment dans les conditions décrites ci-après.

L'invention a également pour objet un procédé de préparation d'un peptide d'intérêt caractérisé en ce qu'il comprend la mise en oeuvre d'un procédé tel que décrit ci-dessus d'obtention de cellules végétales ou de plantes transformées selon l'invention, ledit procédé étant effectué par transformation de cellules végétales avec des séquences nucléotidiques susmentionnées codant pour un polypeptide de fusion contenant un site de clivage tel que décrit ci-dessus, et comprend une étape supplémentaire de clivage dudit polypeptide de fusion, à l'aide d'un réactif approprié, puis, le cas échéant, une étape de purification du polypeptide d'intérêt.

L'invention concerne également un procédé de biotransformation de grains d'amidon caractérisé en ce qu'il comprend les étapes suivantes :
- transformation de cellules végétales telles que définies ci-dessus à l'aide de cellules hôtes décrites ci-dessus contenant une ou plusieurs séquences nucléotidiques codant pour des enzymes susceptibles de transformer l'amidon susmentionnées,
- obtention de plantes, algues ou micro-algues transformées de manière à ce que leur génome contienne une ou plusieurs séquences nucléotidiques décrites ci-dessus, par culture *in vitro* des cellules végétales transformées susmentionnées,
- le cas échéant, fécondation et récupération des semences des plantes obtenues à l'étape précédente, et mise en culture de ces semences pour l'obtention de plantes de génération suivante,
- extraction des grains d'amidon à partir des plantes, algues ou micro-algues, ou de parties, notamment fleurs, fruits, feuilles, tiges, racines, ou de fragments de ces plantes, algues ou micro-algues transformées susmentionnées, notamment par sédimentation dans les conditions décrites ci-après,
- le cas échéant, chauffage desdits grains d'amidon à une température à laquelle le peptide d'intérêt du polypeptide de fusion susmentionné est susceptible d'être actif.

De préférence, lorsque les procédés décrits ci-dessus sont réalisés par transformation de cellules de plantes, ces dernières sont transformées avec des séquences recombinantes susmentionnées contenant la séquence nucléotidique de l'ADNc d'environ 2900 à 3100 paires de bases, et dont les 1696 paires de bases de l'extrémité 3' sont représentées sur la figure 1, ladite séquence nucléotidique codant pour la GBSSI de *Chlamydomonas reinhardtii* décrite ci-dessus, et plus particulièrement avec des séquences recombinantes susmentionnées contenant la séquence nucléotidique représentée sur la figure 2, ou contenant un fragment ou une séquence dérivée tels que décrits ci-dessus de la séquence nucléotidique représentée sur la figure 2. L'utilisation de telles séquences recombinantes contenant la séquence nucléotidique codant pour la GBSSI de *Chlamydomonas reinhardtii* décrite ci-dessus, permet avantageusement d'éviter l'apparition de phénomènes de co-suppression chez les plantes transformées ainsi obtenues.

L'invention a également pour objet un procédé de préparation d'anticorps reconnaissant spécifiquement une amidon synthétase liée au grain d'amidon, d'une plante, algue ou micro-algue déterminée, par immunisation d'un animal, notamment d'un lapin, avec de l'amidon provenant de ladite plante, algue ou micro-algue.

A ce titre, l'invention a plus particulièrement pour objet un procédé de préparation d'anticorps reconnaissant spécifiquement la GBSSI, d'une plante, algue ou micro-algue déterminée, par immunisation d'un animal, notamment d'un lapin, avec de l'amidon provenant de ladite plante, algue ou micro-algue.

L'invention a plus particulièrement pour objet encore un procédé de préparation d'anticorps reconnaissant spécifiquement une isoforme de GBSS autre que la GBSSI, d'une plante, algue ou micro-algue déterminée, par immunisation d'un animal, notamment d'un lapin, avec de l'amidon provenant de ladite plante, algue ou micro-algue comportant une mutation telle que l'expression de la GBSSI est réprimée, par exemple une mutation choisie parmi les suivantes : *sta2-29::ARG7* chez *Chlamydomonas reinhardtii* (décrite par *Delrue et al., 1992,* susmentionné), *amf* chez la pomme de terre (décrite par *Hovenkamp-Hermelink et al., 1987,* susmentionné), wx chez le maïs, le riz et le blé (décrite par *Tsaï., 1974,* susmentionné), *lam* chez le pois (décrite par *Denyer et al., 1995*, susmentionné).

L'invention a également pour objet un procédé d'obtention de l'amidon synthétase, telle que la GBSS, et plus particulièrement pour l'isoforme GBSSI, d'une plante, algue ou micro-algue déterminée, par criblage d'une banque d'ADNc préparée à partir de cellules de ladite plante, algue ou micro-algue déterminée, susceptibles de contenir cette enzyme, à l'aide d'un antisérum contenant des anticorps reconnaissant spécifiquement ladite enzyme codée par un ou plusieurs ADNc de la banque, lorsque ladite enzyme est exprimée par un vecteur de clonage approprié, ledit antisérum étant obtenu selon le procédé susmentionné.

L'invention divulgue également les séquences nucléotidiques codant pour une amidon-synthétase, ou pour une protéine dérivée, choisies parmi :
- la séquence nucléotidique de l'ADNc représenté sur la figure 2, correspondant à SEQ ID NO : 1 dans la liste de séquences ci-après, ladite séquence nucléotidique codant pour la GBSSI de *Chlamydomonas reinhardtii,*
- tout fragment tel que défini ci-dessus de la séquence nucléotidique SEQ ID NO : 1 représentée sur la figure 2, et plus particulièrement toute séquence dont le nucléotide de l'extrémité 5' correspond à celui situé en l'une des positions 1 à 186 de SEQ ID NO : 1, et dont le nucléotide de l'extrémité 3' correspond à celui situé en l'une des positions 1499 à 3117 de SEQ ID NO : 1, notamment :
   - la séquence SEQ ID NO : 2 délimitée par les nucléotides situés aux positions 15 à 2138 de SEQ ID NO : 1, codant pour la GBSSI de *Chlamydomonas reinhardtii* sous forme de pré-protéine de 708 acides aminés (SEQ ID NO : 3) délimitée par les acides aminés situés aux positions 1 et 708 de la séquence peptidique représentée sur la figure 2,
   - la séquence SEQ ID NO : 4 délimitée par les nucléotides situés aux positions 186 à 2138 de SEQ ID NO : 1, codant pour la GBSSI de *Chlamydomonas reinhardtii* sous forme de protéine mature de 651 acides aminés SEQ ID NO : 5) délimitée par les acides aminés situés aux positions 58 et 708 de la séquence peptidique représentée sur la figure 2,
   - la séquence SEQ ID NO : 6 délimitée par les nucléotides situés aux positions 186 à 1499 de SEQ ID NO : 1, codant pour un fragment de 438 acides aminés (SEQ ID NO : 7) délimité par les acides aminés situés aux positions 58 et 495 de la séquence peptidique de la GBSSI de *Chlamydomonas reinhardtii* représentée sur la figure 2,
   - la séquence SEQ ID NO : 8 délimitée par les nucléotides situés aux positions 186 à 1778 de SEQ ID NO : 1, codant pour un fragment de 531 acides aminés (SEQ ID NO : 9) délimité par les acides aminés situés aux positions 58 et 588 de la séquence peptidique de la GBSSI de *Chlamydomonas reinhardtii* représentée sur la figure 2,
- ou une séquence nucléotidique dérivée par dégénérescence du code génétique des séquences nucléotidiques susmentionnées, et codant pour la GBSSI de *Chlamydomonas reinhardtii* susmentionnée, ou pour un fragment peptidique susmentionné de cette dernière,
- ou une séquence nucléotidique dérivée d'une séquence ou d'un fragment nucléotidique susmentionnés, notamment par substitution, suppression ou addition d'un ou plusieurs nucléotides, et codant une séquence peptidique dérivée de la GBSSI de *Chlamydomonas reinhardtii* susmentionnée, ou dérivée d'un fragment peptidique susmentionné de cette dernière, et ayant la propriété de s'associer aux grains d'amidon, ladite séquence nucléotidique dérivée ayant de préférence une homologie d'au moins environ 50 %, et de préférence d'au moins environ 70 %, avec la séquence ou fragment nucléotidique susmentionnés,
- ou une séquence nucléotidique susceptible d'hybrider avec une des séquences ou fragments nucléotidiques susmentionnés, notamment dans les conditions stringentes d'hybridation définies ci-dessus.

L'invention a également pour objet les polypeptides choisis parmi :
- la séquence peptidique SEQ ID NO : 3 délimitée par les acides aminés situés aux positions 1 à 708 de la figure 2, correspondant à la GBSSI de *Chlamydomonas reinhardtii* sous forme de pré-protéine de 708 acides aminés,
- tout fragment tel que défini ci-dessus de la séquence peptidique SEQ ID NO : 3 représentée sur la figure 2, et plus particulièrement toute séquence dont l'acide aminé de l'extrémité aminoterminale correspond à celui situé en l'une des positions 1 à 58 de SEQ ID NO : 3, et dont l'acide aminé de l'extrémité carboxyterminale correspond à celui situé en l'une des positions 495 à 708 de SEQ ID NO : 3, notamment :
   - la séquence SEQ ID NO : 5 délimitée par les acides aminés situés aux positions 58 à 708 de SEQ ID NO : 3, correspondant à la GBSSI de *Chlamydomonas reinhardtii* sous forme de protéine mature de 651 acides aminés,
   - la séquence SEQ ID NO : 7 délimitée par les acides aminés situés aux positions 58 à 495 de SEQ ID NO : 3, correspondant à un fragment de 438 acides aminés de la séquence peptidique de la GBSSI de *Chlamydomonas reinhardtii* représentée sur la figure 2,
   - la séquence SEQ ID NO : 9 délimitée par les acides aminés situés aux positions 58 à 588 de SEQ ID NO : 3, correspondant à un fragment de 531 acides aminés de la séquence peptidique de la GBSSI de *Chlamydomonas reinhardtii* représentée sur la figure 2,
- ou une séquence peptidique dérivée d'une séquence ou d'un fragment peptidique susmentionnés, notamment par substitution, suppression ou addition d'un ou plusieurs acides aminés, et ayant la propriété de s'associer aux grains d'amidon, ladite séquence peptidique dérivée ayant de préférence une homologie d'au moins environ 60%, avantageusement d'au moins environ 80%, avec la séquence ou fragment peptidique susmentionnés,
la propriété que possède la GBSSI de *Chlamydomonas reinhardtii*, ou un fragment ou une protéine dérivée de cette dernière tels que définis ci-dessus, de pouvoir s'associer aux grains d'amidon, pouvant être mesurée selon la méthode décrite ci-dessus.

L'invention a également pour objet les anticorps, polyclonaux ou monoclonaux, dirigés contre les polypeptides susmentionnés.

L'invention sera davantage illustrée à l'aide de la description détaillée qui suit du clonage du gène codant pour la GBSSI de *Chlamydomonas reinhardtii*, et de l'obtention de grains d'amidon transformés contenant un polypeptide de fusion avec ladite GBSSI, ainsi qu'à l'aide de la figure 1 représentant la séquence nucléotidique et la séquence protéique déduite de l'insert ADNc du clone CD142 codant pour la GBSSI de *Chlamydomonas reinhardtii.* (la séquence soulignée correspond à une des trois régions hautement conservées à travers toutes les amidon- et glycogène- synthétases et intervient probablement dans la fixation du substrat ADP-glucose).

### I) Clonage des séquences d'ADNc (ADN complémentaire) et d'ADNg (ADN génomique) correspondant au gène de structure de la GBSSI de Chlamydomonas reinhardtii.

### A) Clonage de l'ADNc

La stratégie développée afin de cloner l'ADNc correspondant au gène de structure de la GBSSI de *Chlamydomonas reinhardtii* fait appel au criblage d'une banque d'expression à l'aide d'un antisérum polyclonal. L'antisérum est capable de reconnaître une séquence polypeptidique codée par un ADNc exprimé depuis un vecteur de clonage adéquat.

### a) Production de l'antisérum

Afin de produire un antisérum susceptible de reconnaître spécifiquement la GBSSI de *C*. *reinhardtii*, l'amidon provenant de la souche sauvage (137C) a été injecté à trois reprises à un lapin hybride néo-zélandais albinos. Lors d'une expérience similaire, c'est l'amidon résiduel d'une souche double mutante aux loci *STA2* et *STA3* (IJ2) qui a été injecté au lapin dans les mêmes conditions.

### Protocoles détaillés :

- Génotypes des souches de *C*. *reinhardtii :*
137C : *mt- nitl1 nit2*
IJ2 : *mt- nit1 nit2 sta2-29::ARG7 sta3-1*

La souche 137C constitue la souche de référence pour toutes les études du métabolisme de l'amidon effectuées chez C. *reinhardtii.*La souche IJ2 a été entièrement décrite par Maddelein et coll. en 1994. Dans cette souche double mutante aux loci *STA2* et *STA3,* les activités GBSSI et SSII sont simultanément absentes. La mutation au locus *STA2* a été générée par interruption génique à l'aide du plasmide pARG7 (Maddelein et coll., 1994) et conduit à la disparition totale de la GBSSI du grain d'amidon, alors que l'allèle mutant du gène *STA3* a été engendré par mutagenèse aux rayons X (Fontaine et coll., 1993).
- Conditions de culture, extraction et purification de l'amidon : les cellules sont mises en culture pendant 3 jours dans le milieu TAP en lumière continue (3000 lux) à partir d'un inoculum de 5x10⁴ cellules/ml. La culture principale est stoppée alors que la concentration cellulaire atteint environ 2x10⁶ cellules/ml.

**Composition du milieu TAP (valeurs pour un litre de milieu) :**

| | | | |
|---|---|---|---|
| NUL₄Cl | 0,40 g | ZnSO₄.7H₂O | 22 mg |
| Tris | 2,40 g | H₃BO₃ | 11,4 mg |
| KH₂PO₄ | 0,32 g | MnCl₂.4H₂O | 5,1 mg |
| K₂HPO₄ | 1,47 g | FeSO₄.7H₂O | 4,2 mg |
| CaCl₂.2H₂O | 0,05 g | MoO₃ | 1,8 mg |
| MgS0₄.7H₂O | 0,30 g | CoCl₂.6H₂O | 1,6 mg |
| EDTA | 50 mg | CuO₄.5H₂O | 1,6 mg |

| | | | |
|---|---|---|---|
| Le pH du milieu est ajusté à 7 par de l'acide acétique glacial | | | |

Le milieu TAP-N présente la même composition de base, mais ce milieu se distingue du premier par l'absence d'azote apporté sous forme de chlorure d'ammonium, remplacé par le chlorure de sodium à la même concentration ; c'est dans ces conditions de culture que les cellules accumulent une quantité d'amidon représentant jusqu'à vingt fois celle de cellules cultivées en milieu TAP. Dans ce cas, la culture est menée pendant 5 jours en lumière continue à partir d'une culture inoculée à 5x10⁵ cellules/ml.
Les cellules sont ensuite concentrées par centrifugation à 2-4x10⁸ cellules/ml (tampon Tris/acétate pH 7,5 50 mM ; EDTA 10 mM ; DTT 2,5 mM) puis soumises à l'action de la presse de French à 10000 psi. L'extrait obtenu en sortie de presse est centrifugé à 5000 g pendant 15 min à 4°C. Le culot qui contient l'amidon est remis en suspension dans un volume d'eau, auquel sont rajoutés neuf volumes de Percoll (Pharmacia, Uppsala, Suède) avant d'être centrifugé à 10000 g pendant 30 min à 4°C. Le Percoll forme un gradient de densité lors de la centrifugation. L'amidon qui est très dense (sa densité est de 1,3 à 1,5) sédimente au fond du tube alors que les lipides et autres débris cellulaires de faible densité forment une « capsule » à la surface du gradient de Percoll. Le culot d'amidon est ensuite rincé trois fois par de l'eau désionisée puis conservé à 4°C après l'avoir débarrassé du dernier surnageant de rinçage.
- Conditions d'immunisation du lapin, prélèvement et préparation de l'antisérum : le lapin utilisé lors de cette expérience est un lapin hybride néo-zélandais albinos. Trois injections successives espacées de trois semaines ont été effectuées avec 20 mg d'amidon purifié en suspension dans 500 µl d'eau. A cette suspension, sont rajoutés 500 µl de l'adjuvant de Freud standard. Le sang du lapin a été prélevé 3 semaines après l'ultime injection. Le sérum est préparé par simple centrifugation du sang après 24 heures de coagulation à 4°C. Les antiséra générés par les injections des amidons des souches 137C et IJ2 sont identifiés par les dénominations « antisérum SA137C » et « antisérum SAIJ2 » respectivement dans ce qui suit.

### b) Préparation et criblage de la banque d'ADNc La banque d'ADNc a été produite à partir des ARNm purifiés de la souche sauvage de C. reinhardtii.C'est le vecteur d'expression λ ZAP qui a été utilisé

### Protocoles détaillés :

- Préparation des ARN totaux de *C*. *reinhardtii* : cette méthode est une adaptation de celle utilisée pour extraire les ARN des feuilles d'*Arabidopsis thaliana*. Les cellules d'une culture de 1-2 x 10⁶ cellules/ml sont récoltées par centrifugation à 3500 g pendant 15 min à 4°C. Les cellules sont ensuite réparties en aliquotes d'un volume d'environ 200 µl. A ce stade, les cellules sont congelées dans l'azote liquide et peuvent être conservées à -80°C pour plusieurs mois. A ces 200 µl de cellules congelées sont ajoutés 400 µl du tampon « Z6 » de composition suivante :

| | | |
|---|---|---|
| Tampon Z6 : | MES/NaOH pH 7,0 | 20 mM |
| | EDTA | 20 mM |
| | Guanidine-HCl | 6 M |
| | β-mercaptoéthanol | 100 *µ*M. |

Le mélange est très fortement agité pendant plusieurs minutes, puis 400 µl du mélange phénol/chloroforme/alcool isoamylique (25v/24v/lv) sont additionnés et le mélange est de nouveau fortement agité pendant plusieurs minutes. Le tout est centrifugé à 13000 g pendant 10 min à 4°C. Après avoir récupéré puis estimé le volume du surnageant, on y ajoute 1/20 de volume d'acide acétique à 1 M ainsi que 0,7 volumes d'éthanol 100%. On laisse le temps aux acides nucléiques de précipiter à -20°C pendant au moins 30 min. Après centrifugation à 13000 g pendant 15 min à 4°C, le culot est resuspendu dans 400 µl d'acétate de sodium à 3 M pH 5,6 puis centrifugé 10 min à 13000 g à 4°C. Le culot est alors rincé deux fois avec de l'éthanol à 70%, séché et enfin dissout dans 50 µl d'eau traitée au DEPC. La quantité d'acides nucléiques est estimée au spectrophotomètre à 260 nm (DO₂₆₀=1 équivaut à environ 40 µg/ml d'acides nucléiques).
- Construction d'une banque d'ADNc dans le vecteur λ ZAP : les ARN dotés d'une queue polyA (les ARNm en particulier) sont isolés de la préparation d'ARN totaux grâce au kit « polyATtract mRNA isolation systems » commercialisé par Promega (Madison, WI, USA). La synthèse des ADNc, la ligation dans le vecteur ZAP et l'empaquetage dans les capsides sont effectués avec l'aide du kit « cDNA synthesis kit, ZAP-cDNA synthesis kit and ZAP-cDNA gigapack II gold cloning kit » commercialisé par Stratagene (La Jolla, CA, USA). Le protocole suivi correspond au manuel d'instruction fourni avec le kit.
- Criblage immunologique d'une banque d'ADNc dans un vecteur d'expression : le criblage de la banque d'expression d'ADNc λ ZAP de *C. reinhardtii* a été réalisé par l'utilisation de l'antisérum préalablement obtenu (voir supra). Environ 100000 plaques de lyse sont étalées par la technique du Top-agar sur plusieurs boites de pétri contenant du milieu de croissance bactérien et l'antibiotique adapté. Après 3 heures d'incubation à 37°C, des filtres de nitrocellulose (Protan BA 85, Schleicher & Schuell, Dassel, Allemagne) préalablement plongés dans une solution d'IPTG 10 mM et séchés, sont appliqués à la surface du Top-agar. Les boîtes sont à nouveau incubées pendant 3 heures à 37°C avant d'être placées 30 min à 4°C. Les filtres de nitrocellulose sont alors délicatement retirés de la surface gélosée. Lors du criblage de la banque λZAP c'est la souche *d'E. coli* XL1-blue qui a été utilisée. Le protocole de révélation des filtres est alors le même que celui utilisé lors de l'étude en Western Blot (voir le chapitre concernant le Western Blot).

Les plages de lyse positives sont soumises à deux séries successives de criblage par le même antisérum afin de confirmer leur caractère positif, mais aussi pour les purifier. Lorsqu'une plage de lyse se révèle positive à la fin des trois tours de criblage, la séquence du plasmide pBluescript SK+ contenant l'insert d'intérêt est excisée du phage λ *in vivo*. C'est le phage « ExAssist helper phage » qui est utilisé pour la cotransfection de la souche SOLR avec le phage λ ZAP. On obtient ainsi un phagemide qui est utilisé pour l'infection de la souche XL1-Blue MRF' conduisant à la restitution du plasmide double brin pBluescript SK+ porteur de l'ADNc d'intérêt.

Un tel criblage mené avec l'antisérum SA137C a conduit à l'obtention d'un unique clone positif après trois tours de criblage. Nous avons dénommé ce clone par « CD 142 ». L'insert du clone CD142 possède une taille de 1696 pb (voir la séquence sur la figure 1).

### c) Analyse de la séquence de l'insert du clone CD142

Lorsqu'on interroge les banques de séquences protéiques avec celle déduite du clone d'ADNc « CD142 », les similitudes les plus importantes sont obtenues avec les GBSSI des plantes supérieures. Cette première indication sur l'origine de cet ADNc est renforcée par la présence d'une extension de 119 aminoacides (environ 14 kDa) en position carboxy-terminale de la séquence codante, par rapport aux principales GBSSI des plantes supérieures. En effet, le poids moléculaire de la GBSSI de C. *reinhardtii,* estimé par SDS-PAGE, est en moyenne de 10 à 15 kDa supérieur à ceux des protéines correspondantes chez les végétaux. L'extension de 119 aminoacides pourrait expliquer cette différence de poids moléculaire entre les GBSSI d'origines différentes. Prise séparément, cette extension de la séquence codante ne partage aucune similitude avec d'autres types de séquences polypeptidiques déjà connues.

La présence du codon stop UAA en position 717 signale le début d'une très longue région non codante de 946 pb. Très fréquentes dans les gènes nucléaires de Chlamydomonas, ces régions non codantes en position 3' terminale, semblent être surtout destinées à stabiliser le messager.

### B) Clonage de l'ADNg

L'ADNg relatif au clone CD142 a été isolé après criblage d'une banque indexée d'ADNg dans des cosmides (Zhang et coll., 1994). Construite dans un vecteur cosmidique dérivé de c2RB, cette banque d'ADNg est contenue dans 120 plaques de microtitration de 96 puits. Chaque puits (sauf deux afin de faciliter l'orientation de la plaque) contient un clone bactérien transportant un unique cosmide. L'ensemble de la banque représente ainsi 11280 clones dont la taille moyenne des inserts avoisine 38 kb. Le génome nucléaire de C. *reinhardtii* y est donc statistiquement représenté environ quatre fois.

Le criblage de cette banque avec une sonde correspondant au clone CD142 a conduit à l'isolement un clone d'ADN génomique dénommé 18B1. L'insert présent dans cet unique cosmide a été analysé plus en détail. Après restriction par NotI puis hybridation avec la sonde CD142, seule une bande d'environ 9 kb reste positive, indiquant que toute l'information correspondant au clone CD142 est présente dans ce fragment. La séquence génomique correspondant au clone CD142 est présentée ci dessous.

### Protocoles détaillés :

- Préparation des filtres de nylon pour le criblage : les filtres de nylon (Hybond N, Amersham Buchler, Braunschweig, Allemagne) sont délicatement déposés sur une boîte de pétri contenant un milieu de croissance bactérien riche supplémenté avec l'antibiotique adéquat (dans le cas présent, c'est l'ampicilline qui est utilisée). Chaque clone *d'E. coli* contenu dans la banque est alors répliqué directement sur le filtre de Nylon à l'aide d'un appareil à réplique et les boîtes ainsi préparées sont incubées une nuit à 37°C. Les filtres sont ensuite ôtés de la surface gélosée puis soumis au traitement suivant :
(1) 2 min sur une solution de dénaturation (NaOH 0,5 M ; NaCl 1,5 M)
(2) 2 min sur une solution de neutralisation (Tris/HCl pH 7,0 0,5 M ; NaCl 1,5 M)
(3) 2 min sur une solution de rinçage (tampon 2 x SSC)

Les filtres sont finalement incubés dans une étuve à sec pendant 2 heures à 80°C.
- Préhybridation et hybridation des filtres : la préhybridation est effectuée dans le tampon d'hybridation à 42°C pendant au moins 4 heures. L'hybridation est accomplie à 42°C une nuit complète en présence de la sonde nucléotidique marquée par le ³²P. Le lavage de la membrane s'effectue à 60°C dans la solution de lavage adaptée à la stringence que l'on veut appliquer. Le temps et la fréquence de changement des bains de lavage varient selon la stringence et les niveaux de radioactivité détectés sur la membrane. En général, les bains sont renouvelés toutes les 10 min et le lavage commence avec un tampon de lavage de faible stringence pour terminer par un tampon de plus forte stringence. Un film Kodak X-OMAT AR est finalement exposé aux filtres à -80°C afin de révéler les clones positifs.
Composition des solutions et tampons :
Tampon SSC x 20 : Dissoudre 175,3 g de NaCl et 88,2 g de citrate de sodium dans 800 ml d'eau. Ajuster le pH à 7,0 avec quelques gouttes d'une solution de NaOH 10N. Compléter avec de l'eau jusqu'à 1 litre.
Tampon d'hybridation :

| | |
|---|---|
| Formamide | 50% |
| Denhardt's | x 5 |
| SDS | 0,5% |
| Tampon Phosphate de Na pH 7,0 | 50 mM |
| ADN de sperme de saumon | 100 *µ*g/ml |
| Sérum albumine bovine | 0,5% |

Réactif de Denhardt's x 100 (quantité pour 500 ml dans l'eau) :

| | | | |
|---|---|---|---|
| Ficoll 400 | | 10 g | |
| PolyVinylPyrrolidone | 40 (PVP40) | | 10 g |
| BSA | | 10 g | |

Tampon Phosphate pH 7,0 à 1 M (quantité pour 100 ml de tampon) :

| | | |
|---|---|---|
| Na₂HPO₄ | 1M | 57,7 ml |
| NaH₂PO₄ | 1M | 42,3 ml |

Tampons de lavage :

| | |
|---|---|
| faible stringence : | SSC x 2 ; SDS 0,2% |
| stringence moyenne : | SSC x 1 ; SDS 0,5% |
| forte stringence : | SSC x 0,5 ; SDS 0,5% |
| | SSC x 0,1 ; SDS 0,5% |

- Préparation et marquage d'une sonde nucléotidique au ³²P : le fragment servant de sonde nucléotidique est généralement inséré dans le site de clonage multiple d'un plasmide bactérien. Il est donc d'abord nécessaire de le digérer, avec les endonucléases de restriction adéquates puis de séparer le fragment d'intérêt du reste du plasmide par électrophorèse sur gel d'agarose 1 % tamponné avec du tampon TAE x 1. La bande correspondant au fragment d'intérêt est ensuite découpée du gel et l'extraction de l'ADN effectuée avec le kit « The GENECLEAN II Kit » commercialisé par BIO *101 Inc.* (La Jolla, CA, USA). Le morceau d'agarose est tout d'abord dissout dans une solution d'iodure de sodium 6 M. Lorsque la dissolution est achevée, les molécules d'ADN sont alors captées par une matrice de silice dénommée « Glassmilk ». Les molécules d'ADN en présence de l'agent chaotropique NaI s'adsorbent spécifiquement sur les billes de silice. Après avoir éliminé les sels et l'agarose dissout, les molécules d'ADN sont éluées des billes de silices en présence d'eau stérile.

Le marquage d'une sonde nucléotidique au ³²P est menée à bien grâce au kit « Random primed DNA labelling kit » de Boehringer (Mannheim, Allemagne). Le principe est l'amorçage aléatoire de la réaction d'élongation par l'ADN polymérase de Klenow par utilisation d'un mélange d'héxanucléotides représentant toutes les combinaisons de séquences possibles. L'incorporation de l'élément radioactif s'effectue à partir de [α-³²P]-dCTP (3000 Ci/mmol) dont 50 µCi sont utilisées pour chaque réaction de marquage. La sonde radiomarquée est finalement ajoutée à la solution d'hybridation après dénaturation à 95°C pendant 4 min.

### C) Le locus STA2 chez C. reinhardtii représente le gène de structure de la GBSSI

L'analyse qui suit démontre de manière formelle que le gène *STA2* de *C. reinhardtii* correspond au gène de structure de la GBSSI et que le clone CD142 représente un ADNc issu de ce locus. En effet, des analyses de restriction de l'ADN génomique digéré par l'endonucléase BamHI révèlent une modification profonde du profil de restriction dans la souche BAFR1 mutante au locus *STA2* générée par interruption génique (Delrue et coll. 1992). La même modification est aussi observée dans la souche IJ2 double mutante aux loci *STA2* et *STA3* que Maddelein et coll. (1994) ont généré par croisement de la souche BAFR1 avec une souche mutante *sta3-1*.

De plus, cette modification du profil de restriction dans la descendance méiotique de la souche IJ2 fusionnée à la souche de *C*. *smithii* « CS9 » a pu être suivie dans le croisement suivant :

| | | |
|---|---|---|
| **CS9** | **X** | **IJ2** |
| (*C*. *smithii*) | | (*C*. *reinhardtii*) |
| +/+ | | *sta2-29::ARG7*/*sta3-1* |
| | ↓ | |
| | +/+ | 25% |
| *sta2-29::ARG7*/*sta3-*1 | | 25% descendance |
| *sta2-29::ARG7*/+ | | 25% méiotique |
| | *+*/*sta*3-1 | 25% |

352 ségrégeants issus de ce croisement ont été purifiés, amplifiés et leur phénotype d'accumulation d'amidon analysé. 54 recombinants méiotiques ont fait l'objet d'une analyse de restriction dont 21 de génotype *sta2-29::ARG7*/+, 19 de génotype +/s*ta3*-1 et 14 sauvages. En ce qui concerne les 21 ségrégeants de génotype *sta2-29::ARG7*/+ leur profil de restriction, obtenu par digestion avec BamHI et hybridé avec la sonde CD142, présente toujours la même modification que la souche parentale IJ2. On en déduit donc que le gène *STA2* et la sonde CD142 sont très fortement liés génétiquement. Il n'y a maintenant plus de doute quant à la nature du clone CD142 qui représente le gène de structure de la GBSSI (le locus *STA2*).

### Protocoles détaillés :

Réalisation des croisements : avant de réaliser la fusion de cellules de polarités sexuelles opposées, il est nécessaire de les mettre dans un état propice à leur fusion. Ainsi les cellules doivent d'abord être différenciées en gamètes avant qu'elles ne soient mises en contact direct. La gamétogenèse est induite chez *Chlamydomonas* en soumettant les cellules à une carence azotée et en présence d'une source lumineuse intense (5000 lux). Pour cela des cellules fraîches cultivées sur milieu gélosé riche (culture de moins de 5 jours) sont mises en suspension dans 2 ml de milieu TAP-N et laissées au minimum 12 heures en lumière forte sans agitation. Avant la mise en contact l'état des cellules est examiné au microscope optique. Après différenciation en gamètes, les cellules sont plus petites et surtout beaucoup plus actives que lors d'une culture non carencée. Des quantités équivalentes de cellules de chaque polarité sexuelle sont mélangées. La fusion est menée toujours en lumière intense. Après une heure de contact, des fusions cellulaires sont déjà visibles au microscope optique. L'analyse des ségrégeants méiotiques va consister à déposer les produits de la fusion cellulaire sur un milieu riche à 4% agar. Les boîtes ainsi obtenues sont incubées en lumière atténuée pendant 15 heures puis entreposées à l'obscurité totale pendant au moins une semaine. Ceci permet la maturation des zygotes et leur « enkystement » dans l'agar à 4%. Après cette période d'incubation à l'obscurité, les boîtes sont remises à la lumière et les étapes suivantes sont réalisées le plus rapidement possible. Afin d'éliminer le plus grand nombre de cellules végétatives non fusionnées, on gratte très légèrement la surface de la gélose avec une lame de rasoir. Par observation à la loupe binoculaire, une région regroupant une cinquantaine de zygotes est repérée et ceux-ci sont transférés sur une boîte fraîche de milieu riche à 1,5% d'agar. Afin de s'assurer de la disparition complète des cellules végétatives résiduelles la boîte est soumise à des vapeurs de chloroforme pendant 45 secondes à une minute (les zygotes peuvent résister, contrairement aux autres cellules, à ce temps moyen d'exposition aux vapeurs de chloroforme). La présence de lumière va déclencher de façon irréversible l'entrée en méiose des zygotes. Lors de leur germination (facilité par l'humidité plus importante du milieu contenant 1,5% d'agar) les zygotes vont libérer quatre cellules filles haploïdes (une tétrade) qui vont croître par divisions mitotiques et former des colonies sur boîte. L'analyse des produits de méiose peut-être menée de deux manières. La première consiste à étudier de manière aléatoire au moins 200 ségrégeants issus du croisement. Après purification des ségrégeants, les caractères de ces derniers peuvent être étudiés par répliques sur différents milieux sélectifs.

Techniques d'extraction d'ADN génomique : le protocole d'extraction de l'ADN total utilisé est celui décrit par Rochaix et coll. (1991) : en voici le détail :
(1) Centrifuger10 ml de culture cellulaire à environ 3-5 x 10⁶ cellules/ml pendant 10 min à 3500 g en falcon de 15 ml.
(2) Le culot de cellules est ensuite resuspendu dans 350 µl du tampon suivant :

| | |
|---|---|
| TRIS/HCl pH 8,0 | 20 mM |
| EDTA | 50 mM |
| NaCl | 100 mM. |

(3) Ajouter 50 µl de protéinase K d'une solution stock à 2 mg/ml (à défaut, on peut utiliser de la pronase à 10 mg/ml).
(4) Ajouter 25 µl de SDS à 20% et incuber 2 heures à 55°C.
(5) Ajouter 2 µl de diethylpyrocarbonate (DEPC) et incuber pendant 15 min à 70°C.
(6) Refroidir le tube brièvement dans la glace et ajouter 50 µl d'une solution d'acétate de potassium à 5 M.
(7) Mélanger en agitant le tube correctement et laisser reposer sur la glace au moins 30 min (il est possible de stopper l'extraction à ce moment là et de la reprendre le lendemain si on laisse les tubes dans la glace en chambre froide).
(8) Transférer dans un tube « eppendorf » de 1,5 ml et centrifuger 15 min dans une minifuge (à environ 13000 g).
(9) Récupérer le surnageant en le transférant dans un nouveau tube « eppendorf ».
(10) Extraire le surnageant par un volume du mélange suivant :

| | |
|---|---|
| Phénol (saturé par le TE : Tris/HCl pH 8,0 10 mM, EDTA 1 mM) | 25 vol |
| Chloroforme | 24 vol |
| Alcool isoamylique | 1 vol |

(11) Après extraction, ajouter 1 ml d'éthanol 100% à température de la pièce. On doit voir apparaître un précipité sous forme de « cheveux d'ange » si l'extraction est réussie. A partir de ce moment, il faut manipuler avec soin et douceur afin d'éviter que les molécules d'ADN ne se brisent.
(12) Centrifuger 5 min dans une minifuge (environ 13000 g).
(13) Rincer le culot avec de l'éthanol à 70% et centrifuger 3 min en minifuge.
(14) Recommencer l'opération (13) une ou deux fois afin d'éliminer les sels correctement.
(15) Sécher le culot en le plaçant 5 min à 37°C, puis le dissoudre dans 50 µl de TE contenant de la RNase de pancréas de boeuf à 1 µg/ml.

Hybridations moléculaires et analyses par Southern Blot : 25 µg d'ADN sont digérés totalement avec la ou les endonucléases de restriction adéquates. Les produits de la restriction sont ensuite séparés par électrophorèse en gel d'agarose 0,8%, TBE x 1. Puis le gel est incubé successivement 15 min dans la solution de dépurination et 30 min dans la solution de dénaturation. L'ADN dénaturé est ensuite transféré sur membrane de nylon « Porablot NYplus » (Macherey-Nagel GmbH, Düren, Allemagne) par capillarité avec le tampon SSC x 20. Après le transfert, la membrane est incubée à 80°C sous vide d'air pendant 2 heures afin de fixer les fragments d'ADN à la surface de la membrane de nylon. La préhybridation est effectuée dans le tampon d'hybridation à 42°C pendant au moins 4 heures. L'hybridation est accomplie à 42°C une nuit complète en présence de la sonde marquée précédemment préparée. Le lavage de la membrane s'effectue à 60°C dans le tampon de lavage adapté à la stringence que l'on veut appliquer au lavage. Le temps et la fréquence de changement des bains de lavage varient selon la stringence et les niveaux de radioactivité présents sur la membrane. En général, les bains sont renouvelés toutes les 10 min et le lavage commence avec un tampon de lavage de faible stringence pour finir avec un tampon de plus forte stringence. Un film Kodak X-OMAT AR est finalement exposé à la membrane à-80°C afin de révéler les zones d'hybridation.

### II) Etude de la liaison de la GBSSI au grain d'amidon.

### A) Analyse de l'allèle mutant sta2-1

Parmi tous les allèles mutants générés au locus *STA2* chez *C. reinhardtii* un seul conduit à la production d'une GBSSI tronquée de 58 kDa en lieu et place de la protéine sauvage de 76 kDa. C'est le cas de l'allèle *sta2-1* de la souche 18B. Delrue et coll. (1992) par micro-séquençage de la GBSSI extraite d'un gel de polyacrylamide a pu démontrer que les séquences peptidiques amino-terminales des protéines de la souche sauvage (137C) et de la souche mutante (18B) sont identiques.

### Séquences amino-terminales :

➢ GBSSI de la souche 137C : ALDIVMVAAEVAPGGKTGGLGDV
➢ GBSSI de la souche 18B : ALDIVMVAAEVAPGGKTGGLGDV

La protéine produite par l'allèle mutant *sta2-1* est donc tronquée en position carboxy-terminale et le Kₘ pour l'ADP-glucose est accrue d'un facteur 6. L'absence de cette séquence carboxy-terminale ne modifie cependant pas les propriétés de fixation de la protéine sur le grain comme le montre la figure 1.

### Protocole détaillé :

Technique d'extraction des protéines du grain d'amidon et SDS-PAGE : les protéines sont extraites de 0,3 à 1 mg d'amidon avec 60 µl de tampon d'extraction : β-mercaptoéthanol 5% (v/v) ; SDS 2% (p/v) à 100°C pendant 5 min. Après centrifugation à 13000 g pendant 10 min, le surnageant est récupéré et l'opération est renouvelée une fois avec le culot. Les deux surnageants sont réunis et l'échantillon peut être chargé dans les puits du gel après avoir rajouté 10 µl du tampon de chargement suivant : Tris 50 mM, glycine 384 mM, 20% glycérol, SDS 0,1%, bleu de bromophénol 0,001%. La migration est accomplie à température ambiante, à 150 V pendant 1h30 (jusqu'à ce que le bleu de bromophénol quitte le gel). Les protéines sont révélées par coloration au bleu de Coomassie ou par immunodétection (voir plus loin le chapitre spécifique au Western Blot). Lors de la coloration au bleu de Coomassie, le gel est incubé 30 min. dans la solution suivante : 2 g de Coomassie Brilliant Blue R250, 0,5 g de Coomassie Brillant Blue G250, 425 ml d'éthanol, 50 ml de méthanol, 100 ml d'acide acétique qsp 1000 ml avec de l'eau. Le gel est ensuite décoloré à l'aide des solutions suivantes :
➢ 15 à 30 min dans le décolorant I : 450 ml d'éthanol, 50 ml d'acide acétique, qsp 1000 ml avec de l'eau.
➢ une nuit dans le décolorant II: 80 ml d'acide acétique, 50 ml de méthanol, qsp 1000 ml avec de l'eau ; ce décolorant II permet d'éliminer la coloration non spécifique du gel.
➢ le décolorant III (240 ml d'acide acétique, 200 ml de méthanol, qsp 1000 ml avec de l'eau) permet la décoloration totale du gel si nécessaire.

### B) Estimation de la quantité de protéines liées au grain

La quantité de protéines liées au grain d'amidon a été estimée dans différentes conditions de culture et dans des fonds génétiques variables. Pour cela, les cellules ont été placées en conditions d'accumulation massive d'amidon (milieu carencé en azote) ou en conditions de croissance mixotrophe (présence d'azote). Les protéines extraites du grain ont ensuite été déposées sur un gel de polyacrylamide en conditions dénaturantes (présence de SDS). Après la migration, les protéines sont révélées par coloration au bleu de Coomassie. Au cours de cette expérience la souche I7 mutante au locus *STA1* a été utilisée. Cette mutation a été décrite en détail par Van den Koornhuyse et coll. (1996) puis par Van de Wal et coll. (1998). Le locus *STAI* correspond au gène de structure de la grande sous-unité régulatrice de l'ADP-glucose pyrophosphorylase. La mutation *sta1-1* engendrée lors d'une mutagenèse aux rayons X conduit à l'insensibilité de l'enzyme à l'acide 3-phosphoglycérique, son activateur allostérique. Dès lors, la souche I7 accumule moins de 5% de la quantité normale d'amidon. L'estimation de la quantité de GBSSI liée au grain est d'environ 0,1 % du poids d'amidon en condition de carence azotée pour les souches 137C et 18B. Cette valeur atteint 1% dans les conditions de mixotrophie. Dans le cas de la souche I7, quelque soit les conditions de culture, la GBSSI représente plus de 1% du poids du grain d'amidon. Les techniques employées lors de cette analyse sont les mêmes que celles détaillées au paragraphe précédent.

### C) Analyse de la réponse immunitaire en Western Blot

Afin de tester l'antigénicité des antiséra SA137C et SAIJ2 précédemment obtenus chez le lapin, les protéines extraites de 100 µg d'amidon frais provenant de différentes souches cultivées dans des conditions de culture variables ont été soumises à une analyse par la technique d'immunotransfert (Western Blotting). La réponse immunitaire engendrée vis à vis de la GBSSI lors de l'injection de l'amidon de la souche sauvage (137C) s'avère très spécifique et forte (les protéines étant extraites de seulement 100 µg d'amidon frais) même dans le cas de la protéine tronquée chez le mutant *sta2-1.* La quantité de protéines liées au grain d'amidon semble plus importante chez le mutant I7 lors d'une culture carencée en azote comme le montre la présence d'une bande de masse supérieure à la GBSSI révélée par l'antisérum SA137C. Ce fait est confirmé par l'analyse en Western blot réalisée avec l'antisérum SAIJ2 où la réponse immunitaire la plus forte est détectée avec les protéines extraites de l'amidon de la souche I7 cultivée en carence azotée.

A des fins de contrôle, nous avons effectué le même type d'expérience en utilisant l'antisérum PA55 obtenu par Abel et coll. (1995). Cet antisérum engendré chez le lapin est dirigé contre un peptide dont la séquence consensus correspond à la région carboxy-terminale fortement conservée dans toutes les amidon-synthétases de plantes supérieures, qu'elles soient solubles ou liées au grain d'amidon. Cet antisérum reconnaît spécifiquement la GBSSI de *C. reinhardtii* lorsqu'elle est présente dans le grain. D'autre part, l'antisérum PA55 reconnaît aussi la protéine tronquée produite par le mutant 18B (*sta2-1*). Il semble donc que la séquence fortement conservée en position carboxy-terminale soit toujours présente dans la protéine tronquée.

### Protocoles détaillés :

Technique d'extraction des protéines du grain d'amidon et SDS-PAGE : ces techniques sont les mêmes que celles décrites au paragraphe précédent hormis la coloration au bleu de Coomassie qui est omise dans ce cas.

Technique de transfert et de révélation par les antiséra : lorsque la migration sur SDS-PAGE est terminée, le gel est incubé 30 min dans le tampon « Western » x 1 contenant 20% de méthanol. Les protéines sont ensuite électrotransférées sur une membrane de nitrocellulose (Protan BA 85 Schleicher & Schuell, Dassel, Allemagne) à l'aide d'un appareil d'électrotransfert (Multiphor II, LKB-Pharmacia, Bromma, Suède) à 4°C dans les conditions suivantes: 45 min à 250 mA avec le tampon utilisé précédemment. Après cette étape de transfert des protéines sur la membrane de nitrocellulose, cette dernière est incubée 1 heure à température ambiante dans le tampon TBST contenant 3% de BSA. La membrane est ensuite rincer trois fois dans le tampon TBST avant d'être incubée une nuit à 4°C dans l'antisérum primaire de lapin dilué dans le tampon TBS. La membrane est de nouveau rincée trois fois par le tampon TBST puis est incubée une heure à température ambiante avec l'anticorps secondaire biotinylé dilué à 1/500 dans le TBS dirigé contre l'antisérum de lapin. Après trois nouveaux rinçages dans le tampon TBST, la membrane est incubée 30 min à température ambiante avec le complexe streptavidine-phosphatase alcaline dilué à 1/3000 dans le tampon TBS. Finalement, après 3 rinçages dans le tampon TBST, les membranes sont révélées par incubation dans un tampon de diéthanolamine contenant les substrats de la phosphatase alcaline : NBT et BCIP (le temps d'incubation varie selon l'intensité de la réaction). Le kit de détection utilisé est celui proposé par Amersham Buchler (Braunschweig, Allemagne): « Blotting détection kit for rabbit antibodies »

**Composition des solutions et tampons :**

| | | |
|---|---|---|
| Tampon « Western » x 10: | Glycine | 390 mM |
| | Tris | 480 mM |
| | SDS | 0,375% |
| Tampon TBS (Tris Buffer Saline) | Tris/HCl pH 7,5 | 20 mM |
| | NaCl | 500 mM |

Tampon TBST (Tris Buffer Saline Tween): TBS + 0,1% (v/v) Tween 20

NBT: Nitro-Blue Tetrazolium en solution dans le diméthylformamide 70%

BCIP: 5-Bromo-4-Chloro-3-Indolyl Phosphate en solution dans le diméthylformamide.

### III) Ciblage des protéines de fusion dans le grain d'amidon

L'extension carboxy-terminale spécifique de la GBSSI de *C. reinhardtii* n'est pas requise pour le ciblage de la protéine au grain d'amidon *in vivo* comme nous avons pu le démontrer au cours des expériences précédentes. Cette extension d'environ 16 kDa peut être remplacée par une séquence peptidique d'intérêt, autorisant ainsi son ciblage au coeur même du grain d'amidon.

Les différents types de vecteurs susceptibles d'être construits afin d'appliquer cette méthode chez les plantes supérieures, sont constitués :
- d'un gène de sélection et une origine de réplication bactériens afin de pouvoir amplifier le plasmide dans une souche bactérienne appropriée.
- d'un gène de sélection qui permettra une sélection aisée des plantes transformantes
- d'une fusion traductionnelle entre la séquence codante de la GBSSI et une séquence polypeptidique d'intérêt. Deux types de fusions traductionnelles principales peuvent être considérées : dans le premier cas, c'est là séquence tronquée de 58 kDa de la GBSSI qui est fusionnée à la séquence d'intérêt ; dans le deuxième cas, c'est la séquence complète de la GBSSI qui est employée.
- la fusion pourra être placée sous le contrôle d'un promoteur végétal constitutif fort, ou encore d'un promoteur végétal inductible, immédiatement suivi d'un peptide de transit adapté favorisant la translocation de la protéine de fusion vers le chloroplaste.

### IV) Protocole de dosage de l'activité amidon-synthétase liée au grain :

20 µg d'amidon sont ajoutés à 100 µl du mélange réactionnel suivant :

| | | |
|---|---|---|
| Glycylglycine/NaOH pH 9,0 | | 50 mM |
| (NH₄)₂S_{O4} | | 100 mM |
| β-n ercaptoéthanol | | 5 mM |
| MgCl₂ | | 5 mM |
| Sérum albumine bovine | | 0,5 mg/ml |
| ADP-glucose | | 0,2 mM |
| [U ⁴C]ADP-glucose (235 mCi/mmol | 2,66 µg | |
| Citrate trisodique | | 0 ou 0,5 M (précisé selon |
| le cas) | | |

La réaction est menée à 30°C pendant 15 min. puis arrêtée par addition de 3 ml d'éthanol à 70%. Le précipité obtenu est filtré sous vide sur filtre "Whatman Glass Fiber" (Whatman, Maidstone, Angleterre), rincé par 4 x 5 ml d'éthanol 70%. Le comptage radioactif est réalisé avec un compteur Beckman après avoir introduit les filtres dans des fioles de comptage contenant 3,5 ml de liquide de scintillation.

### V) méthodes d'extraction et de purification d'amidon sont les suivantes :

- cas d'une algue verte unicellulaire telle que *Chlamydomonas reinhardtii* (voir la méthode décrite ci-dessus)
- cas de graines, de tubercules ou tout autre organe de plante supérieure :
   l'organe ou le type prélevé sur la plante est correctement homogénéisé (après broyage). Le broyat ainsi obtenu est rincé avec de l'eau au travers d'un tissu filtrant (tel que le Miracloth Calbiochem, La Jolla, CA, USA). Le filtrat est ensuite laissé au repos pendant deux heures afin de permettre aux grains d'amidon de sédimenter. Le sédiment est rincé une première fois avec plusieurs volumes d'eau puis une deuxième fois avec plusieurs volumes d'une solution de NaCl à 0,1M. Le sédiment est une nouvelle fois filtré puis rincé deux fois avec de l'éthanol avant d'être séché.

### BIBLIOGRAPHIE

Abel G., Springer F., Willmitzer L. and Kossmann J., (1996), The Plant Journal, 10 (6) : 981-991
An et al. (1986), Plant Physiol., 81, 301-305
Baba T., Nishihara M., Mizuno K., Kawasaki T., Shimada H., Kobayashi E., Ohnishi S., Tanaka K. and Arai Y., (1993), Plant Physiology, 103 : 565-573
Bevan M. (1984), Nucleic Acids. Res., 12, 8711-8721
Brodelius et al. (1979), FEBS Letters, 103, 93-97
Brodelius (1988), In: Moo-Young M. (Ed), Bioreactor Immobilized Enzymes and Cells: Fundamentals and Applications, Elsevier, Londres
Buléon A., Colonna P., Planchot V., Ball S. (1998), International Journal of Biological Macromolecules, 23 : 85-112
De La Penna et al. (1987), Nature, 325, 274-276
Delrue B., Fontaine T., Routier F., Decq A., Wieruszeski J-M, van den Koornhuyse N., Maddelein M-L., Fournet B. and Bail S. (1992), Journal of Bacteriology, 174 (11) : 3612-3620
Deno et al (1987), J. Plant. Physiol., 131, 315-322
Denyer K., Clarke B., Hylton C., Tatge H. and Smith A., (1996), Plant, Cell and Environnent, 18 : 1019-1026
Denyer K., Hylton C. and Smith A., (1995), Planta, 196 : 256-265
Dry I., Smith A., Edwards A., Battacharyya M., Dunn P. and Martin C. (1992), The Plant Journal, 2 (2) : 193-202
Edwards A., Marshall J., Sidebottom C., Visser R., Smith A. and Martin C. (1995), The Plant Journal, 8 (2) : 283-294
Fontaine T., D'Hulst C., Maddelein M-L, Routier F., Marianne Pépin T., Decq A., Wieruszeski J-M., Delrue B., van den Koornhuyse N., Bossu J-P., Fournet B. and Ball S., (1993), The Journal of Biological Chemistry, 268 (22) : 16223-16230
Gao M., Wanat J., Stinard P.S., James M.G., Myers A.M. (1998) Plant Cell, 10(3) : 399-412
Hovenkamp-Hermelink J., Jacobsen E., Ponstein A., Visser R., Vos-Scheperkeuter G., Bijmolt E., de Vries J., Witholt B. and Feenstra W. (1987), Theorical and Applied Genetics, 75 : 217-221
Jouanin et al. (1987), Plant Sci., 53, 53-63
MacDonald F. and Preiss J., (1985), Plant Physiology, 78 : 849-852
Maddelein M-L., Libessart N., Bellanger F., Delrue B., D'Hulst C., van den Koornhuyse N., Fontaine T., Wieruszeski J-M., Decq A. and Ball S., (1994), The Journal of Biological Chemistry, 269 (40) : 25150-25157
Marshall J., Sidebottom C., Debet M., Martin C., Smith A. and Edwards A., (1996), The Plant Cell, 8 : 1121-1135
Mu C., Harn C., Ko Y-T., Singletary G., Keeling P. and Wasserman B., (1994), The Plant Journal, 6 (2) : 151-159
Müller-Rôber B., Sonnewald U. and Willmitzer L. (1992), The EMBO Journal, 11(4) : 1229-1238
Rochaix J., Mayfield S., Goldschlmidt-Clermont M. and Erickson J., (1991), Plant Molecular Biology : a Practical Approach, pp 253-275, ed. Shaw C., IRL Press, Oxford
Sanford J.C. (1988), Trends in Biotechnology, 6, 299-302
Shannon J. and Garwood D. (1984), In Starch: Chemistry and Technology, 2nd ed., Whistler R., Bemiller J., Paschall E., eds., Academic Press, San Diego, California : 26-86
Smith A., (1990), Planta, 182 : 599-604
Tsai C-Y., (1974), Biochemical Genetics, 11 (2) : 83-95
Van den Koornhuyse N., Libessart N., Delrue B., Zabawinski C., Decq A., Iglesias A., Carton A., Preiss J. and Ball S., (1996), The Journal of Biological Chemistry, 271(27) : 16281-16287
Van de Wal et al (1998), The Journal of Biological Chemistry, 273(35) : 22232-22240
Zhang H., Herman P. and Weeks D., (1994), Plant Molecular Biology, 24 : 663-672

## Revendications

1. Utilisation d'une séquence nucléotidique recombinante insérée dans un vecteur recombinant en un site non essentiel pour sa réplication comprenant, dans le sens 5'→3', une séquence nucléotidique codant pour une adénosine diphosphate glucose α-1, 4-glucane α-4-glucosyltransférase ou amidon-synthétase EC 2.4.1.21, ou pour une protéine dérivée de cette enzyme, par suppression, ou substitution d'un ou plusieurs acides aminés, la séquence nucléotidique de ladite protéine dérivée ayant une homologie d'au moins 60% avec la séquence nucléotidique susmentionnés, ladite enzyme ou protéine dérivée ayant la propriété de migrer vers les sites de biosynthèse des grains d'amidon dans les cellules végétales et de s'associer aux grains d'amidon,
ladite séquence nucléotidique étant choisie parmi les séquences SEQ ID NO 6 ou SEQ ID NO 8,
ladite séquence, codant pour l'enzyme ou protéine susmentionnée, étant placée en amont d'une séquence nucléotidique codant pour un peptide ou polypeptide d'intérêt,
pour la transformation de cellules végétales dans le cadre de la mise en oeuvre d'un procédé de préparation de grains d'amidon contenant un polypeptide de fusion codé par ladite séquence nucléotidique recombinante, ledit polypeptide de fusion comprenant l'enzyme ou protéine susmentionnée en position N-terminale et ledit peptide ou polypeptide d'intérêt en position C-terminale.

2. Utilisation selon la revendication 1, pour la mise en oeuvre d'un procédé de préparation du polypeptide de fusion tel que défini dans la revendication 1, à partir des grains d'amidon contenant ledit polypeptide de fusion.

3. Utilisation selon la revendication 1 ou 2, pour la mise en oeuvre d'un procédé de préparation d'un peptide ou polypeptide d'intérêt à partir du polypeptide de fusion tel que défini dans la revendication 1.

4. Utilisation selon la revendication 1, pour la préparation d'une composition pharmaceutique comprenant des grains d'amidon contenant des polypeptides de fusion tels que définis dans la revendication 1, ou d'une composition pharmaceutique contenant des polypeptides de fusion tels que définis dans la revendication 1, lesdits grains d'amidon ou polypeptides de fusion étant le cas échéant en association avec un véhicule physiologiquement acceptable, le peptide ou polypeptide d'intérêt dans lesdits polypeptides de fusion possédant un effet thérapeutique déterminé.

5. Utilisation selon la revendication 1, pour la préparation d'une composition alimentaire comprenant des grains d'amidon contenant des polypeptides de fusion tels que définis dans la revendication 1, le peptide ou polypeptide d'intérêt dans lesdits polypeptides de fusion étant utilisable dans le domaine agroalimentaire.

6. Séquence nucléotidique recombinante insérée dans un vecteur recombinant en un site non essentiel pour sa réplication, **caractérisée en ce qu'**elle comprend, dans le sens 5'→3', une séquence nucléotidique codant pour une adénosine diphosphate glucose α-1, 4-glucane α-4-glucosyltransférase ou amidon-synthétase EC 2.4.1.21, ladite enzyme ayant la propriété de migrer vers les sites de biosynthèse des grains d'amidon dans les cellules végétales et de s'associer aux grains d'amidon, ladite séquence nucléotidique codant pour l'enzyme ou protéine susmentionnée étant placée en amont d'une séquence nucléotidique codant pour un peptide ou polypeptide d'intérêt, la dite séquence recombinante étant choisie parmi :
- un fragment correspondant à:
. la séquence SEQ ID NO : 6 codant pour un fragment de 438 acides aminés (SEQ ID NO : 7) de la GBSSI de *Chlamydomonas reinhardtii*, ou
. la séquence SEQ ID NO : 8 codant pour un fragment de 531 acides aminés (SEQ ID NO : 9) de la GBSSI de *Chlamydomonas reinhardtii*,
- ou une séquence nucléotidique dérivée par dégénérescence du code génétique des séquences nucléotidiques correspondant à la séquence SEQ ID NO : 6 ou SEQ ID NO : 8, et codant pour la GBSSI de *Chlamydomonas reinhardtii* susmentionnée, ou pour un fragment peptidique susmentionné de cette dernière, ayant la propriété de s'associer au grain d'amidon,
- ou une séquence nucléotidique dérivée d'une séquence ou d'un fragment nucléotidique correspondant à la séquence SEQ ID NO : 6 ou SEQ ID NO : 8, par substitution ou suppression d'un ou plusieurs nucléotides, et codant une séquence peptidique dérivée de la GBSSI de *Chlamydomonas reinhardtii* susmentionnée, et ayant la propriété de s'associer aux grains d'amidon, ladite séquence nucléotidique dérivée ayant une homologie d'au moins 70%, avec la séquence ou fragment nucléotidique susmentionnés.

7. Séquence nucléotidique recombinante selon la revendication 6, **caractérisée en ce que** la séquence nucléotidique codant pour un peptide ou polypeptide d'intérêt est choisie parmi :
- celles codant des peptides biologiquement actifs, notamment des peptides d'intérêt thérapeutique ou utilisables dans le domaine agroalimentaire, ou
- celles codant des enzymes susceptibles de transformer l'amidon, telles que les enzymes interagissant avec les α-glucanes dont les hydrolases diverses, les phosphorylases, les α-1,4 glucanotransférases, les enzymes de branchement, les amylases, et notamment les hydrolases thermostables issues de bactéries extrémophiles telles que les archaebactéries actives à des températures supérieures à 40°C.

8. Séquence nucléotidique recombinante selon l'une des revendications 6 à 7, **caractérisée en ce qu'**elle comprend une séquence nucléotidique codant un site de clivage, ladite séquence nucléotidique étant placée entre la séquence nucléotidique codant pour une amidon-synthétase, ou une protéine dérivée de cette dernière, et la séquence nucléotidique codant le polypeptide d'intérêt.

9. Cellules végétales transgéniques, choisies parmi les cellules de plantes, d'algues ou de micro-algues, capables de fabriquer de l'amidon, lesdites cellules comprenant une séquence nucléotidique recombinante selon l'une des revendications 6 à 8 intégrée dans son génome ou maintenue de manière stable dans son cytoplasme.

10. Plantes, algues ou micro-algues transgéniques, ou parties, notamment fleurs, fruits, feuilles, tiges, racines, semences, ou fragments de ces plantes, algues ou micro-algues, comprenant une séquence nucléotidique recombinante selon l'une des revendications 6 à 8 intégrée dans le génome ou maintenue de manière stable dans le cytoplasme des cellules les composant.

11. Polypeptide de fusion **caractérisé en ce qu'**il comprend :
- en position N-terminale une amidon-synthétase, ladite amidon-synthétase ayant la propriété de migrer vers les sites de biosynthèse des grains d'amidon dans les cellules végétales et de s'associer aux grains d'amidon,
- et, en position C-terminale, un peptide ou polypeptide d'intérêt,
la partie C-terminale de la séquence en acides aminés de l'amidon-synthétase étant ainsi liée à la partie N-terminale de la séquence peptidique d'intérêt, ledit polypeptide de fusion étant codé par une séquence nucléotidique recombinante selon l'une des revendications 6 à 8.

12. Polypeptide de fusion selon la revendication 11, **caractérisé en ce que** l'amidon-synthétase est :
- un polypeptide :
. de séquence SEQ ID NO : 7 correspondant à un fragment de 438 acides aminés de la séquence peptidique de la GBSSI de *Chlamydomonas reinhardtii,* ou
. de séquence SEQ ID NO : 9 correspondant à un fragment de 531 acides aminés de la séquence peptidique de la GBSSI de *Chlamydomonas reinhardtii*,
- ou une séquence peptidique dérivée d'une séquence ou d'un fragment peptidique correspondant à la séquence SEQ ID NO : 7 ou SEQ ID NO : 9, par substitution ou suppression d'un ou plusieurs acides aminés, et ayant la propriété de s'associer aux grains d'amidon, ladite séquence peptidique dérivée ayant une homologie d'au moins environ 60%, avantageusement d'au moins environ 80%, avec la séquence ou fragment peptidique correspondant à la séquence SEQ ID NO : 7 ou SEQ ID NO : 9.

13. Polypeptide de fusion selon la revendication 11 ou 12, **caractérisé en ce qu'**il comprend un site de clivage placé entre d'une part l'amidon-synthétase, ou une protéine dérivée de cette dernière, et, d'autre part, le polypeptide d'intérêt.

14. Grains d'amidon **caractérisés en ce qu'**ils comprennent un ou plusieurs polypeptides de fusion définis dans l'une des revendications 11 à 13.

15. Composition pharmaceutique **caractérisée en ce qu'**elle comprend des grains d'amidon selon la revendication 14, le cas échéant en association avec un véhicule physiologiquement acceptable, lesdits grains contenant un ou plusieurs polypeptides de fusion tels que définis dans l'une des revendications 11 à 13, le peptide d'intérêt dans lesdits polypeptides de fusion possédant un effet thérapeutique déterminé.

16. Composition pharmaceutique selon la revendication 15, **caractérisée en ce qu'**elle se présente sous une forme administrable par voie parentérale, notamment par voie intraveineuse, ou sous une forme administrable par voie orale, le diamètre des grains d'amidon étant compris entre environ 0,1 µm et quelques dizaines de µm, et la proportion en poids des polypeptides de fusion dans ces grains étant comprise entre environ 0,1% et 1%.

17. Composition pharmaceutique **caractérisée en ce qu'**elle comprend un ou plusieurs polypeptides de fusion tels que définis dans l'une des revendications 11 à 13, le cas échéant en association avec un véhicule physiologiquement acceptable, le peptide d'intérêt dans lesdits polypeptides de fusion possédant un effet thérapeutique déterminé.

18. Composition alimentaire **caractérisée en ce qu'**elle comprend des grains d'amidon selon la revendication 16, lesdits grains contenant un ou plusieurs polypeptides de fusion tels que définis dans l'une des revendications 11 à 13, le peptide d'intérêt dans lesdits polypeptides de fusion étant utilisable dans le domaine agroalimentaire.

19. Procédé de préparation de grains d'amidon selon la revendication 14, **caractérisé en ce qu'**il comprend les étapes suivantes :
- transformation de cellules végétales, à l'aide d'un hôte cellulaire, tel que *Agrobacterium tumefaciens,* transformé par un vecteur recombinant, notamment du type plasmide, cosmide ou phage, contenant une séquence nucléotidique recombinante selon l'une des revendications 6 à 8,
- obtention de plantes, algues ou micro-algues transformées de manière à ce que leur génome contienne une ou plusieurs séquences nucléotidiques selon l'une des revendications 6 à 8, par culture *in vitro* des cellules hôtes transformées susmentionnées,
- extraction des grains d'amidon à partir des plantes, algues ou micro-algues, ou de parties, notamment fleurs, fruits, feuilles, tiges, racines, ou de fragments de ces plantes, algues ou micro-algues transformées susmentionnées, notamment par sédimentation.

20. Procédé de préparation de polypeptides de fusion selon l'une des revendications 11 à 13, **caractérisé en ce qu'**il comprend la mise en oeuvre du procédé selon la revendication 19, ledit procédé comprenant une étape supplémentaire de récupération, et le cas échéant de purification, des polypeptides de fusion à partir des grains d'amidon.

21. Procédé de préparation d'un peptide d'intérêt **caractérisé en ce qu'**il comprend la mise en oeuvre du procédé selon la revendication 19 ou la revendication 20, ledit procédé étant effectué par transformation des cellules hôtes avec des séquences nucléotidiques selon la revendication 8, et comprend une étape supplémentaire de clivage du polypeptide de fusion obtenu, à l'aide d'un réactif approprié, puis, le cas échéant, une étape de purification du polypeptide d'intérêt.

22. Procédé de biotransformation de grains d'amidon **caractérisé en ce qu'**il comprend les étapes suivantes :
- transformation de cellules végétales, à l'aide d'un hôte cellulaire, tel que *Agrobacterium tumefaciens,* transformé par un vecteur recombinant, notamment du type plasmide, cosmide ou phage, contenant une séquence nucléotidique recombinante selon la revendication 4, codant des enzymes susceptibles de transformer l'amidon,
- obtention de plantes, algues ou micro-algues transformées de manière à ce que leur génome contienne une ou plusieurs séquences nucléotidiques susmentionnées, par culture *in vitro* des cellules hôtes transformées susmentionnées,
- extraction des grains d'amidon à partir des plantes, algues ou micro-algues, ou de parties, notamment fleurs, fruits, feuilles, tiges, racines, ou de fragments de ces plantes, algues ou micro-algues transformées susmentionnées, notamment par sédimentation,
- le cas échéant, chauffage desdits grains d'amidon à une température à laquelle le peptide d'intérêt du polypeptide de fusion est susceptible d'être actif.

## Patentansprüche

1. Verwendung einer rekombinanten Nukleotidsequenz, die in einen rekombinanten Vektor an einer für ihre Replikation nicht essenziellen Stelle eingefügt ist, umfassend, in 5'→3' -Richtung, eine Nukleotidsequenz, die für eine Adenosindiphosphat-glucose-α-1,-4-glucan-α-4-glycosyltransferase oder Stärkesynthetase EC 2.4.1.21, oder für ein Protein kodiert, das von diesem Enzym durch Suppression oder Substitution einer oder mehrerer Aminosäuren abgeleitet ist, wobei die Nukleotidsequenz dieses abgeleiteten Proteins eine Homologie von mindestens 60 % mit der oben genannten Nukleotidsequenz aufweist, wobei das Enzym oder das abgeleitete Protein die Eigenschaft aufweist, zu den Biosynthesestellen der Stärkekörner in den Pflanzenzellen zu migrieren und sich mit den Stärkekörnern zu assoziieren,
wobei die Nukleotidsequenz aus den Sequenzen SEQ ID NO: 6 oder SEQ ID NO: 8 ausgewählt ist,
wobei die Sequenz, die für das Enzym oder das oben genannte Protein kodiert, stromaufwärts einer Nukleotidsequenz angeordnet ist, die für ein interessierendes Peptid oder Polypeptid kodiert,
zur Transformation von Pflanzenzellen im Rahmen der Durchführung eines Verfahrens zur Herstellung von Stärkekörnern, die ein Fusionspolypeptid enthalten, das von dieser rekombinanten Nukleotidsequenz kodiert wird, wobei das Fusionspolypeptid das Enzym oder das oben genannte Protein an N-terminaler Position und das interessierende Peptid oder Polypeptid an C-terminaler Position umfasst.

2. Verwendung nach Anspruch 1 zur Durchführung eines Verfahrens zur Herstellung des Fusionspolypeptids, wie in Anspruch 1 definiert aus Stärkekörnern, die das Fusionspolypeptid enthalten.

3. Verwendung nach Anspruch 1 oder 2 zur Durchführung eines Verfahrens zur Herstellung eines interessierenden Peptids oder Polypeptids aus dem Fusionspolypeptid gemäß Anspruch 1.

4. Verwendung nach Anspruch 1 zur Herstellung einer pharmazeutischen Zusammensetzung, die Stärkekörner umfasst, die Fusionspolypeptide gemäß Anspruch 1 enthalten, oder einer pharmazeutischen Zusammensetzung, die Fusionspolypeptide gemäß Anspruch 1 enthält, wobei die Stärkekörner oder Fusionspolypeptide gegebenenfalls mit einem physiologisch verträglichen Vehikel assoziiert sind, wobei das interessierende Peptid oder Polypeptid in den Fusionspolypeptiden eine bestimmte therapeutische Wirkung aufweist.

5. Verwendung nach Anspruch 1 zur Herstellung einer Nahrungsmittelzusammensetzung, die Stärkekörner umfasst, die Fusionspolypeptide gemäß Anspruch 1 enthalten, wobei das interessierende Peptid oder Polypeptid in den Fusionspolypeptiden im Agrar- und Nahrungsmittelsektor verwendet werden kann.

6. Rekombinante Nukleotidsequenz, die in einen rekombinanten Vektor an einer für ihre Replikation nicht essenziellen Stelle eingefügt ist, **dadurch gekennzeichnet, dass** sie in 5'→3'-Richtung eine Nukleotidsequenz umfasst, die für eine Adenosindiphosphat-glucose-α-1,4-glucan-α-4-glucosyltransferase oder Stärkesynthetase EC 2.4.1.21 kodiert, wobei das Enzym die Eigenschaft aufweist, zu den Biosynthesestellen der Stärkekörner in den Pflanzenzellen zu migrieren und sich mit den Stärkekörnern zu assoziieren, wobei die für das Enzym oder das oben genannte Protein kodierende Nukleotidsequenz stromaufwärts einer Nukleotidsequenz angeordnet ist, die für ein interessierendes Peptid oder Polypeptid kodiert, wobei die rekombinante Sequenz ausgewählt ist aus:
- einem Fragment, das Folgendem entspricht:
der Sequenz SEQ ID NO: 6, die für ein Fragment mit 438 Aminosäuren (SEQ ID. NO: 7) der GBSSI von *Chlamydomonas reinhardtii* kodiert, oder
der Sequenz SEQ ID NO: 8, die für ein Fragment mit 531 Aminosäuren (SEQ ID NO: 9) der GBSSI von *Chlamydomonas reinhardtii* kodiert,
- oder einer Nukleotidsequenz, die durch Degeneration des genetischen Codes von Nukleotidsequenzen abgeleitet ist, die der Sequenz SEQ ID NO: 6 oder SEQ ID NO: 8 entsprechen und für die oben genannte GBSSI von *Chlamydomonas reinhardtii* oder für ein oben genanntes Peptidfragment von letztgenannter kodieren, die die Eigenschaft aufweist, sich mit dem Stärkekorn zu assoziieren,
- oder einer Nukleotidsequenz, die von einer Nukleotidsequenz oder einem Nukleotidfragment, die bzw. das der Sequenz SEQ ID NO: 6 oder SEQ ID NO: 8 entspricht, durch Substitution oder Suppression eines oder mehrerer Nukleotide abgeleitet ist und eine Peptidsequenz kodiert, die von der oben genannten GBSSI von *Chlamydomonas reinhardtii* abgeleitet ist, und die die Eigenschaft aufweist, sich mit den Stärkekörnern zu assoziieren, wobei die abgeleitete Nukleotidsequenz eine Homologie von mindestens 70 % mit der Nukleotidsequenz oder dem oben genannten Nukleotidfragment aufweist.

7. Rekombinante Nukleotidsequenz nach Anspruch 6, **dadurch gekennzeichnet, dass** die Nukleotidsequenz, die für ein interessierendes Peptid oder Polypeptid kodiert, ausgewählt ist aus:
- jenen, die biologisch aktive Peptide kodieren, insbesondere Peptide, die therapeutisch interessant sind oder im Agrar- und Nahrungsmittelsektor verwendet werden können, oder
- jenen, die Enzyme kodieren, die geeignet sind, Stärke zu transformieren, wie beispielsweise die Enzyme, die mit den α-Glucanen interagieren, darunter die verschiedenen Hydrolasen, die Phosphorylasen, die α-1,4-Glucanotransferasen, die Verzweigungsenzyme, die Amylasen und insbesondere die thermostabilen Hydrolasen, die aus extremophilen Bakterien, wie beispielsweise den Archaebakterien, stammen, die bei Temperaturen oberhalb von 40 °C aktiv sind.

8. Rekombinante Nukleotidsequenz nach einem der Ansprüche 6 bis 7, **dadurch gekennzeichnet, dass** sie ,eine Nukleotidsequenz umfasst, die eine Spaltungsstelle kodiert, wobei die Nukleotidsequenz zwischen der Nukleotidsequenz, die für eine Stärkesynthetase oder ein von dieser abgeleitetes Protein kodiert, und der Nukleotidsequenz, die für das interessierende Polypeptid kodiert, angeordnet ist.

9. Transgene Pflanzenzellen, ausgewählt aus Pflanzen-, Algen- oder Mikroalgenzellen, die in der Lage sind, Stärke herzustellen, wobei die Zellen eine rekombinante Nukleotidsequenz nach einem der Ansprüche 6 bis 8 umfassen, die in ihr Genom integriert ist oder in ihrem Zytoplasma stabil erhalten wird.

10. Transgene Pflanzen, Algen oder Mikröalgen oder Teile, insbesondere Blüten, Früchte, Blätter, Stängel, Wurzeln, Samen oder Fragmente dieser Pflanzen, Algen oder Mikroalgen, die eine rekombinante Nukleotidsequenz nach einem der Ansprüche 6 bis 8 umfassen, die in das Genom integriert ist oder in dem Zytoplasma der Zellen aus denen sie bestehen stabil erhalten wird.

11. Fusionspolypeptid, **dadurch gekennzeichnet, dass** es Folgendes umfasst:
- eine Stärkesynthetase an N-terminaler Position, wobei die Stärkesynthetase die Eigenschaft aufweist, zu den Biosynthesestellen der Stärkekörner in den Pflanzenzellen zu migrieren und sich mit den Stärkekörnern assoziieren,
- und, an C-terminaler Position, ein interessierendes Peptid oder Polypeptid,
wobei der C-terminale Teil der Aminosäuresequenz der Stärkesynthetase auf diese Weise mit dem N-terminalen Teil der interessierenden Peptidsequenz verbunden ist, wobei das Fusionspolypeptid von einer rekombinanten Nukleotidsequenz nach einem der Ansprüche 6, bis 8 kodiert wird.

12. Fusionspolypeptid nach Anspruch 11, **dadurch gekennzeichnet, dass** die Stärkesynthetase Folgendes ist:
- ein Polypeptid:
. mit der Sequenz SEQ ID NO: 7, die einem Fragment mit 438 Aminosäuren der Peptidsequenz der GBSSI von *Chlamydomonas reinhardtii* entspricht,
oder
. mit der Sequenz SEQ ID NO: 9, die einem Fragment mit 531 Aminosäuren der Peptidsequenz der GBSSI von *Chlamydomonas reinhardtii* entspricht,
- oder eine Peptidsequenz, die von einer Peptidsequenz oder einem Peptidfragment, die/das der Sequenz SEQ ID NO: 7 oder SEQ ID NO: 9 entspricht, durch Substitution oder Suppression einer oder mehrerer Aminosäuren abgeleitet ist und die Eigenschaft aufweist, sich mit Stärkekörnern zu assoziieren, wobei die abgeleitete Peptidsequenz ein Homologie von mindestens etwa 60 %, vorteilhafterweise von mindestens etwa 80 % mit der Peptidsequenz oder dem Peptidfragment aufweist, die/das der Sequenz SEQ ID NO: 7 oder SEQ ID NO: 9 entspricht.

13. Fusionspolypeptid nach Anspruch 11 oder 12, **dadurch gekennzeichnet, dass** es eine Spaltungsstelle umfasst, die zwischen der Stärkesynthetase oder einem von dieser abgeleiteten Protein einerseits und dem interessierenden Polypeptid andererseits angeordnet ist

14. Stärkekörner, **dadurch gekennzeichnet, dass** sie ein oder mehrere Fusionspolypeptide gemäß einem der Ansprüche 11 bis 13 umfassen.

15. Pharmazeutische Zusammensetzung, **dadurch gekennzeichnet, dass** sie Stärkekörner nach Anspruch 14, gegebenenfalls in Assoziation mit einem physiologisch verträglichen Vehikel umfasst, wobei die Körner ein oder mehrere Fusionspolypeptide gemäß einem der Ansprüche 11 bis 13 enthalten, wobei das interessierende Peptid in diesen Fusionspolypeptiden eine bestimmte therapeutische Wirkung aufweist.

16. Pharmazeutische Zusammensetzung nach Anspruch 15, **dadurch gekennzeichnet, dass** sie in einer parenteral, insbesondere intravenös verabreichbaren Form oder in einer oral verabreichbaren Form vorliegt, wobei der Durchmesser der Stärkekörner im Bereich zwischen etwa 0,1 µm und einigen Dutzend µm liegt und der Gewichtsanteil der Fusionspolypeptide in diesen Körnern im Bereich zwischen etwa 0,1 % und 1 % liegt.

17. Pharmazeutische Zusammensetzung, **dadurch gekennzeichnet, dass** sie ein oder mehrere Fusionspolypeptide gemäß einem der Ansprüche 11 bis 13, gegebenenfalls in Assoziation mit einem physiologisch verträglichen Vehikel enthält, wobei das interessierende Peptid in diesen Fusionspolypeptiden eine bestimmte therapeutische Wirkung aufweist.

18. Nahrungsmittelzusammensetzung, **dadurch gekennzeichnet, dass** sie Stärkekörner nach Anspruch 16 umfasst, wobei die Körner ein oder mehrere Fusionspolypeptide gemäß einem der Ansprüche 11 bis 13 enthalten, wobei das interessierende Peptid in diesen Fusionspolypeptiden im Agrar- und Nahrungsmittelsektor verwendbar ist.

19. Verfahren zur Herstellung von Stärkekörnern nach Anspruch 14, **dadurch gekennzeichnet, dass** es die folgenden Schritte umfasst:
- Transformieren von Pflanzenzellen mithilfe eines zellulären Wirts, wie beispielsweise *Agrobacterium, tumefaciens,* das mit einem rekombinanten Vektor, insbesondere einem Plasmid-, Cosmid- oder Phagenvektor, transformiert wurde, der eine rekombinante Nukleotidsequenz nach einem der Ansprüche 6 bis 8 enthält,
- Erhalten von Pflanzen, Algen oder Mikroalgen, die derart transformiert wurden, dass ihr Genom eine oder mehrere der Nukleotidsequenzen nach einem der Ansprüche 6 bis 8 enthält, durch *In-vitro*-Kultur der oben genannten transformierten Wirtszellen,
- Extrahieren von Stärkekörnern aus Pflanzen, Algen oder Mikroalgen oder Teilen, insbesondere Blüten, Früchten, Blättern, Stengeln, Wurzeln oder Fragmenten dieser oben genannten transformierten Pflanzen, Algen oder Mikroalgen, insbesondere durch Sedimentation

20. Verfahren zur Herstellung von Fusionspolypeptiden nach einem der Ansprüche 11 bis 13, **dadurch gekennzeichnet, dass** es die Durchführung des Verfahrens nach Anspruch 19 umfasst, wobei das Verfahren einen zusätzlichen Schritt zur Gewinnung und gegebenenfalls zur Reinigung der Fusionspolypeptide aus Stärkekörnern umfasst.

21. Verfahren zur Herstellung eines interessierenden Polypeptids, **dadurch gekennzeichnet, dass** es die Durchführung des Verfahrens nach Anspruch 19 oder Anspruch 20 umfasst, wobei das Verfahren durch Transformation der Wirtszellen mit Nukleotidsequenzen nach Anspruch 8 durchgeführt wird und einen zusätzlichen Schritt zur Spaltung des erhaltenen Fusionspolypeptids mithilfe eines geeigneten Reagens, dann gegebenenfalls einen Schritt zur Reinigung des interessierenden Polypeptids, umfasst.

22. Verfahren zur Biotransformation von Stärkekörnern, **dadurch gekennzeichnet, dass** es die folgenden Schritte umfasst:
- Transformieren von Pflanzenzellen mithilfe eines zellulären Wirts, wie beispielsweise *Agrobacterium tumefaciens*, das mit einem rekombinanten Vektor, insbesondere einem Plasmid-, Cosmid- oder Phagenvektor, transformiert wurde, der eine rekombinante Nukleotidsequenz nach Anspruch 4 enthält, die Enzyme kodiert, die für die Transformation der Stärke geeignet sind,
- Erhalten von Pflanzen, Algen oder Mikroalgen, die derart transformiert wurden, dass ihr Genom eine oder mehrere der oben genannten Nukleotidsequenzen enthält, durch *In-vitro-*Kultur der oben genannten transformierten Wirtszellen,
- Extrahieren von Stärkekörnern aus Pflanzen, Algen oder Mikroalgen oder Teilen, insbesondere Blüten, Früchten, Blättern, Stengeln, Wurzeln oder Fragmenten dieser oben genannten transformierten Pflanzen, Algen oder Mikroalgen, insbesondere durch Sedimentation,
- gegebenenfalls Erhitzen der Stärkekörner auf eine Temperatur, bei der das interessierende Peptid des Fusionspolypeptids aktiv sein kann.

## Claims

1. Use of a recombinant nucleotide sequence inserted into a recombinant vector in a site that is non-essential for its replication comprising, in the 5' → 3' direction, a nucleotide sequence coding for an adenosine diphosphate glucose α-1, 4-glucane α-4-glucosyltransferase or starch-synthase EC 2.4.1.21, or a protein derived from this enzyme, by suppression, or substitution of one or more amino acids, the nucleotide sequence of the said derived protein having a homology of at least 60% with the aforementioned sequence, the said enzyme or derived protein having the property of migrating to the sites of biosynthesis of starch granules in plant cells and of attaching to the starch granules, the said nucleotide sequence being chosen from sequences SEQ ID NO 6 or SEQ ID NO 8, the said sequence, coding for the aforementioned enzyme or protein, being positioned upstream of a nucleotide sequence coding for a peptide or a polypeptide of interest, for the transformation of plant cells during the implementation of a method for the preparation of starch granules containing a fusion polypeptide encoded by the said recombinant nucleotide sequence, the said fusion polypeptide comprising the aforementioned enzyme or protein in the N-terminal position and the said peptide or polypeptide of interest in the C-terminal position.

2. Use according to Claim 1, for implementing a method for the preparation of the fusion polypeptide as defined in Claim 1, starting from the starch granules containing the said fusion polypeptide.

3. Use according to Claim 1 or 2, for implementing a method for the preparation of a peptide or a polypeptide of interest starting from the fusion polypeptide as defined in Claim 1.

4. Use according to Claim 1, for the preparation of a pharmaceutical preparation comprising the starch granules containing fusion polypeptides as defined in Claim 1, or a pharmaceutical composition containing fusion polypeptides as defined in Claim 1, the said starch granules or fusion polypeptides being if necessary in combination with a physiologically acceptable vehicle, the peptide or polypeptide of interest in the said fusion polypeptides possessing a defined therapeutic effect.

5. Use according to Claim 1, for the preparation of a food composition comprising starch granules containing fusion polypeptides as defined in Claim 1, the peptide or polypeptide of interest in the said fusion polypeptides can be used in the agricultural and food industry.

6. A Recombinant nucleotide sequence inserted into a recombinant vector in a site that is non-essential for its replication, **characterized in that** the nucleotide sequence comprises, in the 5' → 3' direction, a nucleotide sequence coding for an adenosine diphosphate glucose α-1, 4-glucane α-4-glucosyltransferase or starch-synthase EC 2.4.1.21, the said enzyme having the property of migrating to the sites of biosynthesis of the starch granules in plant cells and of attaching to the starch granules, being positioned upstream of a nucleotide sequence coding for a peptide or a polypeptide of interest, the said recombinant nucleotide sequence being chosen from :
• a fragment corresponding to:
- the sequence SEQ ID NO : 6 coding for a fragment of 438 amino acids (SEQ ID NO : 7) of the GBSSI of *Chlamydomonas reinhardtii*, or
- the sequence SED ID NO : 8 coding for a fragment of 531 amino acids (SEQ ID NO : 9) of the GBSSI of *Chlamydomonas reinhardtii*,
• or a nucleotide sequence derived by degeneration of the genetic code of the nucleotide sequence corresponding to the sequence SEQ ID NO : 6 or SEQ ID NO : 8, and coding for the aforementioned GBSSI of *Chlamydomonas reinhardtii,* or for an aforementioned peptide fragment of the latter, having the property of attaching to the starch granule,
• or a nucleotide sequence derived from a sequence or a nucleotide fragment corresponding to the sequence SEQ ID NO : 6 or SEQ ID NO : 8, by substitution or suppression of one or more nucleotides, and coding for a peptide sequence derived from the aforementioned *Chlamydomonas reinhardtii*, and having the property of attaching to starch granules, the said derived nucleotide sequence having a homology of at least 70%, with the aforementioned sequence or nucleotide fragment.

7. A recombinant nucleotide sequence according to Claim 6, **characterized in that** the nucleotide sequence coding for a peptide or a polypeptide of interest is chosen from:
• those coding biologically active peptides, especially peptides of therapeutic interest or that can be used in agricultural and food industry, or
• those coding enzymes potentially able to transform starch, such as enzymes that interact with α-glucanes including various hydrolase, phosphorylases, α-4-glucosyltransferase, branching enzymes, amylases, and especially heat-resistant hydrolase obtained from extremophiles such as a rchaebacteria that are active at temperature above 40°C.

8. A recombinant nucleotide sequence according to one of Claims 6 to 7, **characterized in that** the nucleotide sequence comprises a nucleotide sequence coding a cleavage site, the said nucleotide sequence being positioned upstream in the nucleotide sequence coding for a starch-synthase, or a protein derived from the latter, and a nucleotide sequence coding the polypeptide of interest.

9. Transgenic plant cells, chosen from plant cells, algae or micro-algae, able to produce starch, the said cells comprising a recombinant nucleotide sequence according to one of Claims 6 to 8 integrated in its genome or maintained in a stable manner in its cytoplasm.

10. Plants, algae or micro-algae, or parts, especially flowers, fruits, leaves, stems, roots or fragments of these plants, algae or micro-algae, comprising a recombinant nucleotide sequence according to one of Claims 6 to 8 integrated in the genome or maintained in a stable manner in the cytoplasm of the cells of which they are composed.

11. A fusion polypeptide **characterized in that** the fusion polypeptide comprises:
• in the N-position a starch-synthase, the said starch-synthase having the property of migrating to the biosynthesis sites of starch granule in plant cells and of attaching to the starch granules,
• and, in the C-terminal position, a peptide or a polypeptide of interest, the C-terminal part of the sequence in amino acids of the starch-synthase, being **in that** way linked to the N-terminal part of the peptide sequence of interest, the said fusion polypeptide being encoded by a recombinant nucleotide sequence according to one of Claims 6 to 8.

12. A fusion polypeptide according to Claim 11, **characterized in that** the starch-synthase is:
• a polypeptide:
- of the sequence SEQ ID NO : 7 corresponding to a fragment of 438 amino acids of the nucleotide sequence of the GBSSI of *Chlamydomonas reinhardtii,* or
- of the sequence SEQ ID NO : 9 corresponding to a fragment of 531 amino acids of the nucleotide sequence of the GBSSI of *Chlamydomonas reinhardtii,*
• or of peptide sequence derived from a sequence or a peptide fragment corresponding to the sequence SEQ ID NO : 7 or SEQ ID NO : 9, by substitution or suppression of one or more amino acids, and having the property of attaching to starch granules, the said derived peptide sequence having a homology of at least about 60%, advantageously at least about 80%, with the sequence or peptide fragment corresponding to the sequence SEQ ID N'O : 7 or SEQ ID NO: 9.

13. A fusion polypeptide according to Claim, 11 or 12, **characterized in that** the fusion polypeptide comprises a cleavage site positioned between, on the one hand, the starch-synthase, or a protein derived from the latter, and, on the other end, the polypeptide of interest.

14. Starch granules **characterized in that** the starch granules comprise one or more fusion polypeptides defined in one of Claims 11 to 13.

15. A pharmaceutical composition **characterized in that** it comprises starch granules according to Claim 14, if necessary in combination with a physiologically acceptable vehicle, the said granules containing one or more fusion polypeptides as defined in one of Claim 11 to 13, the peptide of interest in the said fusion polypeptides possessing a defined therapeutic effect.

16. A pharmaceutical composition according to Claim 15, **characterized in that** it is in a form that can be administered parenterally, especially intravenously, or in a form that can be administered orally, the diameter of the starch granules being between about 0.1 µm and several tens of µm, and the proportion by weight of the fusion polypeptides in these granules being between about 0.1% and 1%.

17. A pharmaceutical composition **characterized in that** it comprises one or more fusion polypeptides as defined in one of Claims 11 to 13, if necessary in combination with a physiologically acceptable vehicle, the peptide of interest in the said fusion polypeptides possessing a defined therapeutic effect.

18. A food composition **characterized in that** it comprises starch granules according to Claim 16, the said granules containing one or more fusion polypeptides as defined in one of Claims 11 to 13, the peptide of interest in the said fusion polypeptides being usable in the food processing field.

19. A method of preparation of starch granules according to Claim 14, **characterized in that** it comprises the following steps:
• transformation of plant cells, by means of a cellular host, such as *Agrobacterium tumefaciens,* transformed by a recombinant vector, especially of the plasmid type, cosmid or phage, containing a recombinant nucleotide sequence according to one of Claims 6 to 8,
• Obtaining plant, algae or micro-algae transformed in such a way that their genome contains one or more nucleotide sequences according to one of Claims 6 to 8, by *in vitro* culture of the aforementioned transformed host cells,
• Extraction of starch granules from the plants, algae or micro-algae, or from parts, especially flowers, fruits, leaves, steams, roots, or fragments of these aforementioned transformed plants, algae or micro-algae, especially by sedimentation.

20. A method of preparation of fusion polypeptides according to one of Claims 11 to 13, **characterized in that** it includes the implementation of the method according to Claim 19, the said method comprising an additional step of recovery, and if necessary of purification, of the fusion polypeptides from the starch granules.

21. A method of preparation of a peptide of interest **characterized in that** it includes the implementation of the method according to Claim 19 or Claim 20, the said method being carried out by transformation of host cells with nucleotide sequences according to Claim 8, and comprises an additional step of cleavage of the fusion polypeptide obtained, by means of a suitable reagent, then, if necessary, a purification step of the polypeptide of interest.

22. A method of biotransformation of starch granules **characterized in that** it comprises the following steps:
• transformation of plant cells, by means of a cellular host, such as *Agrobacterium tumefaciens,* transformed by a recombinant vector, especially of the plasmid, cosmid or phage type, containing a recombinant nucleotide sequence according to Claim 4, coding to enzymes potentially able to transform starch,
• obtaining plants, algae or micro-algae transformed in such a way that their genome contains one or more aforementioned nucleotide sequences, by *in vitro* culture of the aforementioned transformed host cells,
• extraction of starch granules from the plants, algae or micro-algae, or from parts, especially flowers, fruits, leaves, steams, roots, or fragment of these aforementioned transformed plants, algae or micro-algae, especially by sedimentation.
• If necessary, heating of the said starch granules to a temperature at which the peptide of interest of the fusion polypeptide is capable of being active.


### LISTE DE SEQUENCES

<110> CNRS
<120> GRAINS D'AMIDON CONTENANT UN POLYPEPTIDE RECOMBINANT D'INTERET, LEUR PROCEDE D'OBTENTION, ET LEURS UTILISATIONS
<130> WOB 99AB CNR AMYL
<140>
   <141>
<150> FR9906494
   <151> 1999-05-21
<160> 9
<170> PatentIn Ver. 2.1
<210> 1
   <211> 3117
   <212> ADN
   <213> Chlamydomonas reinhardtii
<400> 1
   gccaggcgga gcgtatggct gttgcctcta ccagccgccc cagcagcgcg cgtcctatcg 60
   tcatcaatgc cgcgtcgttc ggtgtcaaga agaccgcgaa ccagctgctg cgtgagcttg 120
   ctcgtggctc cgcacgcaag tccacctcgc gctcggctgt tactggtgcc actggtgcca 180
   cttgcgcgct ggacatcgtg atggttgctg ctgaggtcgc cccttggtcc aagacgggcg 240
   gcctgggcga tgtgactggt ggcctgccta ttgagctggt caagcgcggc caccgcgtca 300
   tgaccattgc ccctcgctac gaccagtacg ctgacgcctg ggacacctcg gtggtcgtgg 360
   acatcatggg cgagaaggtc cgctacttcc actccatcaa gaagggcgtg caccgcgtgt 420
   ggattgacca cccctggttc ctggccaagg tctggggcaa gaccggctcc aagctgtacg 480
   gcccccgctc cggcgctgac tacctggaca accacaagcg cttcgccctg ttctgcaagg 540
   ccgctattga ggctgcccgc gtgctgccct tcggccccgg cgaggactgc gtcttcgtgg 600
   ccaacgactg gcactccgcc ctggtgcccg tcctgctgaa ggacgagtac cagcccaagg 660
   gccagttcac caaggccaag tcggtgctgg ctatccacaa catcgccttc cagggccgca 720
   tgtgggagga ggctttcaag gacacgaagc tgccccaggc cgcctttgac aagctggcct 780
   tctcggacgg ctatgccaag gtttacactg aggccacccc catggaggag gacgagaagc 840
   ccccgctgac gggaaagacc tacaagaaga tcaactggct gaagggtggc attatcgccg 900
   ccgacaagct ggtgactgtg tcgcccaact acgcgaccga gatcgctgcc gatgccgccg 960
   gcggtgtgga gctggacacc gtcatccgcg ccaagggcat tgagggcatt gtgaacggca 1020
   tggacattga ggagtggaac cccaagaccg acaagttcct gtctgcgccc tacgaccaga 1080
   acagcgtcta cgccggcaag gccgccgcca aggaggccct gcaggccgag ctgggcctgc 1140
   ctgtggaccc caccgccccc ctgttcgcct tcatcggccg cctggaggag cagaagggtg 1200
   tggacatcat cctggccgcc ctgcccaaga tcctggccac ccccaaggtg cagatcgcca 1260
   tcctgggtac cggcaaggcc gcctacgaga agctggtgaa cgccatcggc accaagtaca 1320
   agggccgcgc caagggcgtg gtcaagttct cggcgcccct ggcgcacatg ctcaccgccg 1380
   gcgccgactt catgctggtg ccctcgcgct tcgagccctg cggcctgatc cagctgcacg 1440
   ccatgcacta cggtaccgtg cccgtggtag cctccaccgg cggcctggtc gacaccgtca 1500
   aggagggcgt caccggcttc cacatgggcg ccctgaaccc cgacaagctg gacgaggctg 1560
   acgccgacgc cctggccgcc accgtgcgcc gtgccagcga ggtgtttgcg ggcggccgct 1620
   accccgagat ggtggccaac tgcatcagcc aggacctgtc ctggtccaag cccgcccaga 1680
   agtgggaggg cctgctggag gaggtggtgt acggcaaggg cggcgtggcc accgccaaga 1740
   aggaggagat caaggtgccc gttgccgaga agatccccgg cgacctgccc gccgtgtcct 1800
   acgcccccaa caccctgaag cccgtgtccg cctccgtgga gggcaacggc gccgccgcgc 1860
   ccaaggtcgg caccaccgcc cccgccatgg gcgcgtggcg cgcgaccacc ccctcgggcc 1920
   cctcgcccgc cgccgccacc cccaaggtga ccacctacaa gcccgccctg cccgccaccg 1980
   ccaagcccaa gaccgctggc ctcaagctgg ccggtgaggc ctccaccacc tcgacctcgg 2040
   agaacggcgc tgcctccaac ggcaacggca acggtgcctc ggcctccaag acctcggctg 2100
   ccaagcccct ggtctccgcc gccacccgca agtccgccta aagcggcagt agccgcagag 2160
   gcggcgacag catgagcggc tcgaccaaag ctgtggcagg aacggctgta gcagcggcag 2220
   gcggccgcca ccggcgagga gcaggcttgc ggcagcgagg gcgatgagct tagcggccgt 2280
   gagcatggca ggcggaaacg tgtgtactga aatgtggtgc atgagagtgt cgtgctgtaa 2340
   tgaagtcggt tttgcgagac cggagaaacg ccggtttggt tttgtagtgc agggcctgtg 2400
   gtttcggttt tgcccaagtc caaaagaaga gtaacgaaac tgtagcagta gcagagcact 2460
   tgcgcggcgc ggcgaccacg ccggcccgtg cgcagcctgt cctgccctca gccttgtgat 2520
   tcggcggcaa gagggcgggt ctgtacactc catccattcc aggatttttg caggctgcct 2580
   gagagtttgc cattttgtgg gacgtgagcg gcgggacggc cgcgccgggc tctcctaccg 2640
   cctccggcaa cggagaagtg ggaggcgctg tagcccggtg accccccaat gtagaggatg 2700
   ggatacataa gagcgtgtgg aatggtggta aaagaggagg ggcctgggtc gcccctcgat 2760
   ggttttgttg aggtgcagac ggcaccgtcg gcgtcaaagg ccctcgcaag gcccgggtgc 2820
   cttgggctca tttttggtgc ccgtcgatga tgagagattg gccagcggtt ttttgaggct 2880
   ggctcgaagc gagggtttgt ggaagtggag cgaggagggt tggagaaaga ggcggacatg 2940
   cttgactgga ggtacacaaa gtggagcgtg cgacggcacg gaggcattgg cggactattg 3000
   acccagtagt gtggaaagta gttggacctg aattctttga gagtaccgcg cattaatccg 3060
   tgagagagta acaaagatgg cacctgaaaa aaaaaaaaaa aaaaaaaaaa aaaaaaa    3117
<210> 2
   <211> 2124
   <212> ADN
   <213> Séquence artificielle
<220>
   <223> Description de la séquence artificielle: fragment de la séquence complète de l'ADNc codant la GBSSI de Chlamydomonas reinhardtii
<220>
   <221> CDS
   <222> (1)..(2124)
<400> 2
   atg gct gtt gcc tct acc agc cgc ccc agc agc gcg cgt cct atc gtc    48
   Met Ala Val Ala Ser Thr Ser Arg Pro Ser Ser Ala Arg Pro Ile Val
    1               5                  10                  15
   atc aat gcc gcg tcg ttc ggt gtc aag aag acc gcg aac cag ctg ctg    96
   Ile Asn Ala Ala Ser Phe Gly Val Lys Lys Thr Ala Asn Gln Leu Leu
               20                  25                  30
   cgt gag ctt gct cgt ggc tcc gca cgc aag tcc acc tcg cgc tcg gct   144
   Arg Glu Leu Ala Arg Gly Ser Ala Arg Lys Ser Thr Ser Arg Ser Ala
           35                  40                  45
   gtt act ggt gcc act ggt gcc act tgc gcg ctg gac atc gtg atg gtt   192
   Val Thr Gly Ala Thr Gly Ala Thr Cys Ala Leu Asp Ile Val Met Val
       50                  55                  60
   gct gct gag gtc gcc cct tgg tcc aag acg ggc ggc ctg ggc gat gtg   240
   Ala Ala Glu Val Ala Pro Trp Ser Lys Thr Gly Gly Leu Gly Asp Val
   65                  70                  75                  80
   act ggt ggc ctg cct att gag ctg gtc aag cgc ggc cac cgc gtc atg   288
   Thr Gly Gly Leu Pro Ile Glu Leu Val Lys Arg Gly His Arg Val Met
                   85                  90                  95
   acc att gcc cct cgc tac gac cag tac gct gac gcc tgg gac acc tcg   336
   Thr Ile Ala Pro Arg Tyr Asp Gln Tyr Ala Asp Ala Trp Asp Thr Ser
               100                 105                 110
   gtg gtc gtg gac atc atg ggc gag aag gtc cgc tac ttc cac tcc atc    384
   Val Val Val Asp Ile Met Gly Glu Lys Val Arg Tyr Phe His Ser Ile
           115                 120                 125
   aag aag ggc gtg cac cgc gtg tgg att gac cac ccc tgg ttc ctg gcc    432
   Lys Lys Gly Val His Arg Val Trp Ile Asp His Pro Trp Phe Leu Ala
       130                 135                 140
   aag gtc tgg ggc aag acc ggc tcc aag ctg tac ggc ccc cgc tcc ggc    480
   Lys Val Trp Gly Lys Thr Gly Ser Lys Leu Tyr Gly Pro Arg Ser Gly
   145                 150                 155                 160
   gct gac tac ctg gac aac cac aag cgc ttc gcc ctg ttc tgc aag gcc    528
   Ala Asp Tyr Leu Asp Asn His Lys Arg Phe Ala Leu Phe Cys Lys Ala
                   165                 170                 175
   gct att gag gct gcc cgc gtg ctg ccc ttc ggc ccc ggc gag gac tgc    576
   Ala Ile Glu Ala Ala Arg Val Leu Pro Phe Gly Pro Gly Glu Asp Cys
               180                 185                 190
   gtc ttc gtg gcc aac gac tgg cac tcc gcc ctg gtg ccc gtc ctg ctg    624
   Val Phe Val Ala Asn Asp Trp His Ser Ala Leu Val Pro Val Leu Leu
           195                 200                 205
   aag gac gag tac cag ccc aag ggc cag ttc acc aag gcc aag tcg gtg    672
   Lys Asp Glu Tyr Gln Pro Lys Gly Gln Phe Thr Lys Ala Lys Ser Val
       210                 215                 220
   ctg gct atc cac aac atc gcc ttc cag ggc cgc atg tgg gag gag gct    720
   Leu Ala Ile His Asn Ile Ala Phe Gln Gly Arg Met Trp Glu Glu Ala
   225                 230                 235                 240
   ttc aag gac acg aag ctg ccc cag gcc gcc ttt gac aag ctg gcc ttc    768
   Phe Lys Asp Thr Lys Leu Pro Gln Ala Ala Phe Asp Lys Leu Ala Phe
                   245                 250                 255
   tcg gac ggc tat gcc aag gtt tac act gag gcc acc ccc atg gag gag    816
   Ser Asp Gly Tyr Ala Lys Val Tyr Thr Glu Ala Thr Pro Met Glu Glu
               260                 265                 270
   gac gag aag ccc ccg ctg acg gga aag acc tac aag aag atc aac tgg    864
   Asp Glu Lys Pro Pro Leu Thr Gly Lys Thr Tyr Lys Lys Ile Asn Trp
           275                 280                 285
   ctg aag ggt ggc att atc gcc gcc gac aag ctg gtg act gtg tcg ccc    912
   Leu Lys Gly Gly Ile Ile Ala Ala Asp Lys Leu Val Thr Val Ser Pro
       290                 295                 300
   aac tac gcg acc gag atc gct gcc gat gcc gcc ggc ggt gtg gag ctg    960
   Asn Tyr Ala Thr Glu Ile Ala Ala Asp Ala Ala Gly Gly Val Glu Leu
   305                 310                 315                 320
   gac acc gtc atc cgc gcc aag ggc att gag ggc att gtg aac ggc atg    1008
   Asp Thr Val Ile Arg Ala Lys Gly Ile Glu Gly Ile Val Asn Gly Met
                   325                 330                 335
   gac att gag gag tgg aac ccc aag acc gac aag ttc ctg tct gcg ccc    1056
   Asp Ile Glu Glu Trp Asn Pro Lys Thr Asp Lys Phe Leu Ser Ala Pro
               340                 345                 350
   tac gac cag aac agc gtc tac gcc ggc aag gcc gcc gcc aag gag gcc    1104
   Tyr Asp Gln Asn Ser Val Tyr Ala Gly Lys Ala Ala Ala Lys Glu Ala
           355                 360                 365
   ctg cag gcc gag ctg ggc ctg cct gtg gac ccc acc gcc ccc ctg ttc    1152
   Leu Gln Ala Glu Leu Gly Leu Pro Val Asp Pro Thr Ala Pro Leu Phe
       370                 375                 380
   gcc ttc atc ggc cgc ctg gag gag cag aag ggt gtg gac atc atc ctg    1200
   Ala Phe Ile Gly Arg Leu Glu Glu Gln Lys Gly Val Asp Ile Ile Leu
   385                 390                 395                 400
   gcc gcc ctg ccc aag atc ctg gcc acc ccc aag gtg cag atc gcc atc    1248
   Ala Ala Leu Pro Lys Ile Leu Ala Thr Pro Lys Val Gln Ile Ala Ile
                   405                 410                 415
   ctg ggt acc ggc aag gcc gcc tac gag aag ctg gtg aac gcc atc ggc    1296
   Leu Gly Thr Gly Lys Ala Ala Tyr Glu Lys Leu Val Asn Ala Ile Gly
               420                 425                 430
   acc aag tac aag ggc cgc gcc aag ggc gtg gtc aag ttc tcg gcg ccc    1344
   Thr Lys Tyr Lys Gly Arg Ala Lys Gly Val Val Lys Phe Ser Ala Pro
           435                 440                 445
   ctg gcg cac atg ctc acc gcc ggc gcc gac ttc atg ctg gtg ccc tcg    1392
   Leu Ala His Met Leu Thr Ala Gly Ala Asp Phe Met Leu Val Pro Ser
       450                 455                 460
   cgc ttc gag ccc tgc ggc ctg atc cag ctg cac gcc atg cac tac ggt    1440
   Arg Phe Glu Pro Cys Gly Leu Ile Gln Leu His Ala Met His Tyr Gly
   465                 470                 475                 480
   acc gtg ccc gtg gta gcc tcc acc ggc ggc ctg gtc gac acc gtc aag    1488
   Thr Val Pro Val Val Ala Ser Thr Gly Gly Leu Val Asp Thr Val Lys
                   485                 490                 495
   gag ggc gtc acc ggc ttc cac atg ggc gcc ctg aac ccc gac aag ctg    1536
   Glu Gly Val Thr Gly Phe His Met Gly Ala Leu Asn Pro Asp Lys Leu
               500                 505                 510
   gac gag gct gac gcc gac gcc ctg gcc gcc acc gtg cgc cgt gcc agc    1584
   Asp Glu Ala Asp Ala Asp Ala Leu Ala Ala Thr Val Arg Arg Ala Ser
           515                 520                 525
   gag gtg ttt gcg ggc ggc cgc tac ccc gag atg gtg gcc aac tgc atc    1632
   Glu Val Phe Ala Gly Gly Arg Tyr Pro Glu Met Val Ala Asn Cys Ile
       530                 535                 540
   agc cag gac ctg tcc tgg tcc aag ccc gcc cag aag tgg gag ggc ctg    1680
   Ser Gln Asp Leu Ser Trp Ser Lys Pro Ala Gln Lys Trp Glu Gly Leu
   545                 550                 555                 560
   ctg gag gag gtg gtg tac ggc aag ggc ggc gtg gcc acc gcc aag aag    1728
   Leu Glu Glu Val Val Tyr Gly Lys Gly Gly Val Ala Thr Ala Lys Lys
                   565                 570                 575
   gag gag atc aag gtg ccc gtt gcc gag aag atc ccc ggc gac ctg ccc    1776
   Glu Glu Ile Lys Val Pro Val Ala Glu Lys Ile Pro Gly Asp Leu Pro
               580                 585                 590
   gcc gtg tcc tac gcc ccc aac acc ctg aag ccc gtg tcc gcc tcc gtg    1824
   Ala Val Ser Tyr Ala Pro Asn Thr Leu Lys Pro Val Ser Ala Ser Val
           595                 600                 605
   gag ggc aac ggc gcc gcc gcg ccc aag gtc ggc acc acc gcc ccc gcc    1872
   Glu Gly Asn Gly Ala Ala Ala Pro Lys Val Gly Thr Thr Ala Pro Ala
       610                 615                 620
   atg ggc gcg tgg cgc gcg acc acc ccc tcg ggc ccc tcg ccc gcc gcc    1920
   Met Gly Ala Trp Arg Ala Thr Thr Pro Ser Gly Pro Ser Pro Ala Ala
   625                 630                 635                 640
   gcc acc ccc aag gtg acc acc tac aag ccc gcc ctg ccc gcc acc gcc    1968
   Ala Thr Pro Lys Val Thr Thr Tyr Lys Pro Ala Leu Pro Ala Thr Ala
                   645                 650                 655
   aag ccc aag acc gct ggc ctc aag ctg gcc ggt gag gcc tcc acc acc    2016
   Lys Pro Lys Thr Ala Gly Leu Lys Leu Ala Gly Glu Ala Ser Thr Thr
               660                 665                 670
   tcg acc tcg gag aac ggc gct gcc tcc aac ggc aac ggc aac ggt gcc    2064
   Ser Thr Ser Glu Asn Gly Ala Ala Ser Asn Gly Asn Gly Asn Gly Ala
           675                 680                 685
   tcg gcc tcc aag acc tcg gct gcc aag ccc ctg gtc tcc gcc gcc acc    2112
   Ser Ala Ser Lys Thr Ser Ala Ala Lys Pro Leu Val Ser Ala Ala Thr
       690                 695                 700
   cgc aag tcc gcc                                                    2124
   Arg Lys Ser Ala
   705
<210> 3
   <211> 708
   <212> PRT
   <213> Séquence artificielle
   <223> Description de la séquence artificielle: fragment de la séquence complète de l'ADNc codant la GBSSI de Chlamydomonas reinhardtii
<400> 3
   Met Ala Val Ala Ser Thr Ser Arg Pro Ser Ser Ala Arg Pro Ile Val
   1               5                   10                  15
   Ile Asn Ala Ala Ser Phe Gly Val Lys Lys Thr Ala Asn Gln Leu Leu
               20                  25                  30
   Arg Glu Leu Ala Arg Gly Ser Ala Arg Lys Ser Thr Ser Arg Ser Ala
           35                  40                  45
   Val Thr Gly Ala Thr Gly Ala Thr Cys Ala Leu Asp Ile Val Met Val
       50                  55                  60
   Ala Ala Glu Val Ala Pro Trp Ser Lys Thr Gly Gly Leu Gly Asp Val
   65                  70                  75                  80
   Thr Gly Gly Leu Pro Ile Glu Leu Val Lys Arg Gly His Arg Val Met
                   85                  90                  95
   Thr Ile Ala Pro Arg Tyr Asp Gln Tyr Ala Asp Ala Trp Asp Thr Ser
   100 105 110
   Val Val Val Asp Ile Met Gly Glu Lys Val Arg Tyr Phe His Ser Ile
   115 120 125
   Lys Lys Gly Val His Arg Val Trp Ile Asp His Pro Trp Phe Leu Ala
   130 135 140
   Lys Val Trp Gly Lys Thr Gly Ser Lys Leu Tyr Gly Pro Arg Ser Gly
   145 150 155 160
   Ala Asp Tyr Leu Asp Asn His Lys Arg Phe Ala Leu Phe Cys Lys Ala
   165 170 175
   Ala Ile Glu Ala Ala Arg Val Leu Pro Phe Gly Pro Gly Glu Asp Cys
   180 185 190
   Val Phe Val Ala Asn Asp Trp His Ser Ala Leu Val Pro Val Leu Leu
   195 200 205
   Lys Asp Glu Tyr Gln Pro Lys Gly Gln Phe Thr Lys Ala Lys Ser Val
   210 215 220
   Leu Ala Ile His Asn Ile Ala Phe Gln Gly Arg Met Trp Glu Glu Ala
   225 230 235 240
   Phe Lys Asp Thr Lys Leu Pro Gln Ala Ala Phe Asp Lys Leu Ala Phe
   245 250 255
   Ser Asp Gly Tyr Ala Lys Val Tyr Thr Glu Ala Thr Pro Met Glu Glu
   260 265 270
   Asp Glu Lys Pro Pro Leu Thr Gly Lys Thr Tyr Lys Lys Ile Asn Trp
   275 280 285
   Leu Lys Gly Gly Ile Ile Ala Ala Asp Lys Leu Val Thr Val Ser Pro
   290 295 300
   Asn Tyr Ala Thr Glu Ile Ala Ala Asp Ala Ala Gly Gly Val Glu Leu
   305 310 315 320
   Asp Thr Val Ile Arg Ala Lys Gly Ile Glu Gly Ile Val Asn Gly Met
   325 330 335
   Asp Ile Glu Glu Trp Asn Pro Lys Thr Asp Lys Phe Leu Ser Ala Pro
   340 345 350
   Tyr Asp Gln Asn Ser Val Tyr Ala Gly Lys Ala Ala Ala Lys Glu Ala
   355 360 365
   Leu Gln Ala Glu Leu Gly Leu Pro Val Asp Pro Thr Ala Pro Leu Phe
   370 375 380
   Ala Phe Ile Gly Arg Leu Glu Glu Gln Lys Gly Val Asp Ile Ile Leu
   385 390 395 400
   Ala Ala Leu Pro Lys Ile Leu Ala Thr Pro Lys Val Gln Ile Ala Ile
   405 410 415
   Leu Gly Thr Gly Lys Ala Ala Tyr Glu Lys Leu Val Asn Ala Ile Gly
               420                 425                 430
   Thr Lys Tyr Lys Gly Arg Ala Lys Gly Val Val Lys Phe Ser Ala Pro
           435                 440                 445
   Leu Ala His Met Leu Thr Ala Gly Ala Asp Phe Met Leu Val Pro Ser
       450                 455                 460
   Arg Phe Glu Pro Cys Gly Leu Ile Gln Leu His Ala Met His Tyr Gly
   465                 470                 475                 480
   Thr Val Pro Val Val Ala Ser Thr Gly Gly Leu Val Asp Thr Val Lys
                   485                 490                 495
   Glu Gly Val Thr Gly Phe His Met Gly Ala Leu Asn Pro Asp Lys Leu
               500                 505                 510
   Asp Glu Ala Asp Ala Asp Ala Leu Ala Ala Thr Val Arg Arg Ala Ser
           515                 520                 525
   Glu Val Phe Ala Gly Gly Arg Tyr Pro Glu Met Val Ala Asn Cys Ile
       530                 535                 540
   Ser Gln Asp Leu Ser Trp Ser Lys Pro Ala Gln Lys Trp Glu Gly Leu
   545                 550                 555                 560
   Leu Glu Glu Val Val Tyr Gly Lys Gly Gly Val Ala Thr Ala Lys Lys
                   565                 570                 575
   Glu Glu Ile Lys Val Pro Val Ala Glu Lys Ile Pro Gly Asp Leu Pro
               580                 585                 590
   Ala Val Ser Tyr Ala Pro Asn Thr Leu Lys Pro Val Ser Ala Ser Val
           595                 600                 605
   Glu Gly Asn Gly Ala Ala Ala Pro Lys Val Gly Thr Thr Ala Pro Ala
       610                 615                 620
   Met Gly Ala Trp Arg Ala Thr Thr Pro Ser Gly Pro Ser Pro Ala Ala
   625                 630                 635                 640
   Ala Thr Pro Lys Val Thr Thr Tyr Lys Pro Ala Leu Pro Ala Thr Ala
                   645                 650                 655
   Lys Pro Lys Thr Ala Gly Leu Lys Leu Ala Gly Glu Ala Ser Thr Thr
               660                 665                 670
   Ser Thr Ser Glu Asn Gly Ala Ala Ser Asn Gly Asn Gly Asn Gly Ala
           675                 680                 685
   Ser Ala Ser Lys Thr Ser Ala Ala Lys Pro Leu Val Ser Ala Ala Thr
       690                 695                 700
   Arg Lys Ser Ala
   705
<210> 4
   <211> 1953
   <212> ADN
   <213> Séquence artificielle
<220>
   <223> Description de la séquence artificielle: fragment de l'ADNc complet codant la GBSSI de Chlamydomonas reinhardtii et codant la protéine GBSSI mature
<220>
   <221> CDS
   <222> (1)..(1953)
<400> 4
   gcg ctg gac atc gtg atg gtt gct gct gag gtc gcc cct tgg tcc aag    48
   Ala Leu Asp Ile Val Met Val Ala Ala Glu Val Ala Pro Trp Ser Lys
   1               5                   10                  15
   acg ggc ggc ctg ggc gat gtg act ggt ggc ctg cct att gag ctg gtc    96
   Thr Gly Gly Leu Gly Asp Val Thr Gly Gly Leu Pro Ile Glu Leu Val
               20                  25                  30
   aag cgc ggc cac cgc gtc atg acc att gcc cct cgc tac gac cag tac    144
   Lys Arg Gly His Arg Val Met Thr Ile Ala Pro Arg Tyr Asp Gln Tyr
           35                  40                  45
   gct gac gcc tgg gac acc tcg gtg gtc gtg gac atc atg ggc gag aag    192
   Ala Asp Ala Trp Asp Thr Ser Val Val Val Asp Ile Met Gly Glu Lys
       50                  55                  60
   gtc cgc tac ttc cac tcc atc aag aag ggc gtg cac cgc gtg tgg att    240
   Val Arg Tyr Phe His Ser Ile Lys Lys Gly Val His Arg Val Trp Ile
   65                  70                  75                  80
   gac cac ccc tgg ttc ctg gcc aag gtc tgg ggc aag acc ggc tcc aag    288
   Asp His Pro Trp Phe Leu Ala Lys Val Trp Gly Lys Thr Gly Ser Lys
                   85                  90                  95
   ctg tac ggc ccc cgc tcc ggc gct gac tac ctg gac aac cac aag cgc    336
   Leu Tyr Gly Pro Arg Ser Gly Ala Asp Tyr Leu Asp Asn His Lys Arg
               100                 105                 110
   ttc gcc ctg ttc tgc aag gcc gct att gag gct gcc cgc gtg ctg ccc    384
   Phe Ala Leu Phe Cys Lys Ala Ala Ile Glu Ala Ala Arg Val Leu Pro
           115                 120                 125
   ttc ggc ccc ggc gag gac tgc gtc ttc gtg gcc aac gac tgg cac tcc    432
   Phe Gly Pro Gly Glu Asp Cys Val Phe Val Ala Asn Asp Trp His Ser
       130                 135                 140
   gcc ctg gtg ccc gtc ctg ctg aag gac gag tac cag ccc aag ggc cag    480
   Ala Leu Val Pro Val Leu Leu Lys Asp Glu Tyr Gln Pro Lys Gly Gln
   145                 150                 155                 160
   ttc acc aag gcc aag tcg gtg ctg gct atc cac aac atc gcc ttc cag    528
   Phe Thr Lys Ala Lys Ser Val Leu Ala Ile His Asn Ile Ala Phe Gln
                   165                 170                 175
   ggc cgc atg tgg gag gag gct ttc aag gac acg aag ctg ccc cag gcc     576
   Gly Arg Met Trp Glu Glu Ala Phe Lys Asp Thr Lys Leu Pro Gln Ala
               180                 185                 190
   gcc ttt gac aag ctg gcc ttc tcg gac ggc tat gcc aag gtt tac act     624
   Ala Phe Asp Lys Leu Ala Phe Ser Asp Gly Tyr Ala Lys Val Tyr Thr
           195                 200                 205
   gag gcc acc ccc atg gag gag gac gag aag ccc ccg ctg acg gga aag     672
   Glu Ala Thr Pro Met Glu Glu Asp Glu Lys Pro Pro Leu Thr Gly Lys
       210                 215                 220
   acc tac aag aag atc aac tgg ctg aag ggt ggc att atc gcc gcc gac     720
   Thr Tyr Lys Lys Ile Asn Trp Leu Lys Gly Gly Ile Ile Ala Ala Asp
   225                 230                 235                 240
   aag ctg gtg act gtg tcg ccc aac tac gcg acc gag atc gct gcc gat     768
   Lys Leu Val Thr Val Ser Pro Asn Tyr Ala Thr Glu Ile Ala Ala Asp
                   245                 250                 255
   gcc gcc ggc ggt gtg gag ctg gac acc gtc atc cgc gcc aag ggc att     816
   Ala Ala Gly Gly Val Glu Leu Asp Thr Val Ile Arg Ala Lys Gly Ile
               260                 265                 270
   gag ggc att gtg aac ggc atg gac att gag gag tgg aac ccc aag acc     864
   Glu Gly Ile Val Asn Gly Met Asp Ile Glu Glu Trp Asn Pro Lys Thr
           275                 280                 285
   gac aag ttc ctg tct gcg ccc tac gac cag aac agc gtc tac gcc ggc     912
   Asp Lys Phe Leu Ser Ala Pro Tyr Asp Gln Asn Ser Val Tyr Ala Gly
       290                 295                 300
   aag gcc gcc gcc aag gag gcc ctg cag gcc gag ctg ggc ctg cct gtg     960
   Lys Ala Ala Ala Lys Glu Ala Leu Gln Ala Glu Leu Gly Leu Pro Val
   305                 310                 315                 320
   gac ccc acc gcc ccc ctg ttc gcc ttc atc ggc cgc ctg gag gag cag     1008
   Asp Pro Thr Ala Pro Leu Phe Ala Phe Ile Gly Arg Leu Glu Glu Gln
                   325                 330                 335
   aag ggt gtg gac atc atc ctg gcc gcc ctg ccc aag atc ctg gcc acc     1056
   Lys Gly Val Asp Ile Ile Leu Ala Ala Leu Pro Lys Ile Leu Ala Thr
               340                 345                 350
   ccc aag gtg cag atc gcc atc ctg ggt acc ggc aag gcc gcc tac gag     1104
   Pro Lys Val Gln Ile Ala Ile Leu Gly Thr Gly Lys Ala Ala Tyr Glu
           355                 360                 365
   aag ctg gtg aac gcc atc ggc acc aag tac aag ggc cgc gcc aag ggc     1152
   Lys Leu Val Asn Ala Ile Gly Thr Lys Tyr Lys Gly Arg Ala Lys Gly
       370                 375                 380
   gtg gtc aag ttc tcg gcg ccc ctg gcg cac atg ctc acc gcc ggc gcc     1200
   Val Val Lys Phe Ser Ala Pro Leu Ala His Met Leu Thr Ala Gly Ala
   385                 390                 395                 400
   gac ttc atg ctg gtg ccc tcg cgc ttc gag ccc tgc ggc ctg atc cag     1248
   Asp Phe Met Leu Val Pro Ser Arg Phe Glu Pro Cys Gly Leu Ile Gln
                   405                 410                 415
   ctg cac gcc atg cac tac ggt acc gtg ccc gtg gta gcc tcc acc ggc     1296
   Leu His Ala Met His Tyr Gly Thr Val Pro Val Val Ala Ser Thr Gly
               420                 425                 430
   ggc ctg gtc gac acc gtc aag gag ggc gtc acc ggc ttc cac atg ggc     1344
   Gly Leu Val Asp Thr Val Lys Glu Gly Val Thr Gly Phe His Met Gly
           435                 440                 445
   gcc ctg aac ccc gac aag ctg gac gag gct gac gcc gac gcc ctg gcc     1392
   Ala Leu Asn Pro Asp Lys Leu Asp Glu Ala Asp Ala Asp Ala Leu Ala
       450                 455                 460
   gcc acc gtg cgc cgt gcc agc gag gtg ttt gcg ggc ggc cgc tac ccc     1440
   Ala Thr Val Arg Arg Ala Ser Glu Val Phe Ala Gly Gly Arg Tyr Pro
   465                 470                 475                 480
   gag atg gtg gcc aac tgc atc agc cag gac ctg tcc tgg tcc aag ccc     1488
   Glu Met Val Ala Asn Cys Ile Ser Gln Asp Leu Ser Trp Ser Lys Pro
                   485                 490                 495
   gcc cag aag tgg gag ggc ctg ctg gag gag gtg gtg tac ggc aag ggc     1536
   Ala Gln Lys Trp Glu Gly Leu Leu Glu Glu Val Val Tyr Gly Lys Gly
               500                 505                 510
   ggc gtg gcc acc gcc aag aag gag gag atc aag gtg ccc gtt gcc gag     1584
   Gly Val Ala Thr Ala Lys Lys Glu Glu Ile Lys Val Pro Val Ala Glu
           515                 520                 525
   aag atc ccc ggc gac ctg ccc gcc gtg tcc tac gcc ccc aac acc ctg     1632
   Lys Ile Pro Gly Asp Leu Pro Ala Val Ser Tyr Ala Pro Asn Thr Leu
       530                 535                 540
   aag ccc gtg tcc gcc tcc gtg gag ggc aac ggc gcc gcc gcg ccc aag     1680
   Lys Pro Val Ser Ala Ser Val Glu Gly Asn Gly Ala Ala Ala Pro Lys
   545                 550                 555                 560
   gtc ggc acc acc gcc ccc gcc atg ggc gcg tgg cgc gcg acc acc ccc     1728
   Val Gly Thr Thr Ala Pro Ala Met Gly Ala Trp Arg Ala Thr Thr Pro
                   565                 570                 575
   tcg ggc ccc tcg ccc gcc gcc gcc acc ccc aag gtg acc acc tac aag     1776
   Ser Gly Pro Ser Pro Ala Ala Ala Thr Pro Lys Val Thr Thr Tyr Lys
               580                 585                 590
   ccc gcc ctg ccc gcc acc gcc aag ccc aag acc gct ggc ctc aag ctg     1824
   Pro Ala Leu Pro Ala Thr Ala Lys Pro Lys Thr Ala Gly Leu Lys Leu
           595                 600                 605
   gcc ggt gag gcc tcc acc acc tcg acc tcg gag aac ggc gct gcc tcc     1872
   Ala Gly Glu Ala Ser Thr Thr Ser Thr Ser Glu Asn Gly Ala Ala Ser
       610                 615                 620
   aac ggc aac ggc aac ggt gcc tcg gcc tcc aag acc tcg gct gcc aag     1920
   Asn Gly Asn Gly Asn Gly Ala Ser Ala Ser Lys Thr Ser Ala Ala Lys
   625                 630                 635                 640
   ccc ctg gtc tcc gcc gcc acc cgc aag tcc gcc                         1953
   Pro Leu Val Ser Ala Ala Thr Arg Lys Ser Ala
                   645                 650
<210> 5
   <211> 651
   <212> PRT
   <213> Séquence artificielle
   <223> Description de la séquence artificielle: fragment de l'ADNc complet codant la GBSSI de Chlamydomonas reinhardtii et codant la protéine GBSSI mature
<400> 5
   Ala Leu Asp Ile Val Met Val Ala Ala Glu Val Ala Pro Trp Ser Lys
   1               5                   10                  15
   Thr Gly Gly Leu Gly Asp Val Thr Gly Gly Leu Pro Ile Glu Leu Val
               20                  25                  30
   Lys Arg Gly His Arg Val Met Thr Ile Ala Pro Arg Tyr Asp Gln Tyr
           35                  40                  45
   Ala Asp Ala Trp Asp Thr Ser Val Val Val Asp Ile Met Gly Glu Lys
       50                  55                  60
   Val Arg Tyr Phe His Ser Ile Lys Lys Gly Val His Arg Val Trp Ile
   65                  70                  75                  80
   Asp His Pro Trp Phe Leu Ala Lys Val Trp Gly Lys Thr Gly Ser Lys
                   85                  90                  95
   Leu Tyr Gly Pro Arg Ser Gly Ala Asp Tyr Leu Asp Asn His Lys Arg
               100                 105                 110
   Phe Ala Leu Phe Cys Lys Ala Ala Ile Glu Ala Ala Arg Val Leu Pro
           115                 120                 125
   Phe Gly Pro Gly Glu Asp Cys Val Phe Val Ala Asn Asp Trp His Ser
       130                 135                 140
   Ala Leu Val Pro Val Leu Leu Lys Asp Glu Tyr Gln Pro Lys Gly Gln
   145                 150                 155                 160
   Phe Thr Lys Ala Lys Ser Val Leu Ala Ile His Asn Ile Ala Phe Gln
                   165                 170                 175
   Gly Arg Met Trp Glu Glu Ala Phe Lys Asp Thr Lys Leu Pro Gln Ala
               180                 185                 190
   Ala Phe Asp Lys Leu Ala Phe Ser Asp Gly Tyr Ala Lys Val Tyr Thr
           195                 200                 205
   Glu Ala Thr Pro Met Glu Glu Asp Glu Lys Pro Pro Leu Thr Gly Lys
       210                 215                 220
   Thr Tyr Lys Lys Ile Asn Trp Leu Lys Gly Gly Ile Ile Ala Ala Asp
   225                 230                 235                 240
   Lys Leu Val Thr Val Ser Pro Asn Tyr Ala Thr Glu Ile Ala Ala Asp
                   245                 250                 255
   Ala Ala Gly Gly Val Glu Leu Asp Thr Val Ile Arg Ala Lys Gly Ile
               260                 265                 270
   Glu Gly Ile Val Asn Gly Met Asp Ile Glu Glu Trp Asn Pro Lys Thr
           275                 280                 285
   Asp Lys Phe Leu Ser Ala Pro Tyr Asp Gln Asn Ser Val Tyr Ala Gly
       290                 295                 300
   Lys Ala Ala Ala Lys Glu Ala Leu Gln Ala Glu Leu Gly Leu Pro Val
   305                 310                 315                 320
   Asp Pro Thr Ala Pro Leu Phe Ala Phe Ile Gly Arg Leu Glu Glu Gln
                   325                 330                 335
   Lys Gly Val Asp Ile Ile Leu Ala Ala Leu Pro Lys Ile Leu Ala Thr
               340                 345                 350
   Pro Lys Val Gln Ile Ala Ile Leu Gly Thr Gly Lys Ala Ala Tyr Glu
           355                 360                 365
   Lys Leu Val Asn Ala Ile Gly Thr Lys Tyr Lys Gly Arg Ala Lys Gly
       370                 375                 380
   Val Val Lys Phe Ser Ala Pro Leu Ala His Met Leu Thr Ala Gly Ala
   385                 390                 395                 400
   Asp Phe Met Leu Val Pro Ser Arg Phe Glu Pro Cys Gly Leu Ile Gln
                   405                 410                 415
   Leu His Ala Met His Tyr Gly Thr Val Pro Val Val Ala Ser Thr Gly
               420                 425                 430
   Gly Leu Val Asp Thr Val Lys Glu Gly Val Thr Gly Phe His Met Gly
           435                 440                 445
   Ala Leu Asn Pro Asp Lys Leu Asp Glu Ala Asp Ala Asp Ala Leu Ala
       450                 455                 460
   Ala Thr Val Arg Arg Ala Ser Glu Val Phe Ala Gly Gly Arg Tyr Pro
   465                 470                 475                 480
   Glu Met Val Ala Asn Cys Ile Ser Gln Asp Leu Ser Trp Ser Lys Pro
                   485                 490                 495
   Ala Gln Lys Trp Glu Gly Leu Leu Glu Glu Val Val Tyr Gly Lys Gly
               500                 505                 510
   Gly Val Ala Thr Ala Lys Lys Glu Glu Ile Lys Val Pro Val Ala Glu
           515                 520                 525
   Lys Ile Pro Gly Asp Leu Pro Ala Val Ser Tyr Ala Pro Asn Thr Leu
       530                 535                 540
   Lys Pro Val Ser Ala Ser Val Glu Gly Asn Gly Ala Ala Ala Pro Lys
   545                 550                 555                 560
   Val Gly Thr Thr Ala Pro Ala Met Gly Ala Trp Arg Ala Thr Thr Pro
                   565                 570                 575
   Ser Gly Pro Ser Pro Ala Ala Ala Thr Pro Lys Val Thr Thr Tyr Lys
               580                 585                 590
   Pro Ala Leu Pro Ala Thr Ala Lys Pro Lys Thr Ala Gly Leu Lys Leu
             595                 600                 605
   Ala Gly Glu Ala Ser Thr Thr Ser Thr Ser Glu Asn Gly Ala Ala Ser
       610                 615                 620
   Asn Gly Asn Gly Asn Gly Ala Ser Ala Ser Lys Thr Ser Ala Ala Lys
   625                 630                 635                 640
   Pro Leu Val Ser Ala Ala Thr Arg Lys Ser Ala
                   645                 650
<210> 6
   <211> 1314
   <212> ADN
   <213> Séquence artificielle
<220>
   <223> Description de la séquence artificielle: fragment de l'ADNc complet codant la GBSSI de Chlamydomonas reinhardtii
<220>
   <221> CDS
   <222> (1)..(1314)
<400> 6
   gcg ctg gac atc gtg atg gtt gct gct gag gtc gcc cct tgg tcc aag      48
   Ala Leu Asp Ile Val Met Val Ala Ala Glu Val Ala Pro Trp Ser Lys
     1               5                  10                  15
   acg ggc ggc ctg ggc gat gtg act ggt ggc ctg cct att gag ctg gtc      96
   Thr Gly Gly Leu Gly Asp Val Thr Gly Gly Leu Pro Ile Glu Leu Val
                20                  25                  30
   aag cgc ggc cac cgc gtc atg acc att gcc cct cgc tac gac cag tac     144
   Lys Arg Gly His Arg Val Met Thr Ile Ala Pro Arg Tyr Asp Gln Tyr
            35                  40                  45
   gct gac gcc tgg gac acc tcg gtg gtc gtg gac atc atg ggc gag aag     192
   Ala Asp Ala Trp Asp Thr Ser Val Val Val Asp Ile Met Gly Glu Lys
        50                  55                  60
   gtc cgc tac ttc cac tcc atc aag aag ggc gtg cac cgc gtg tgg att     240
   Val Arg Tyr Phe His Ser Ile Lys Lys Gly Val His Arg Val Trp Ile
    65                  70                  75                  80
   gac cac ccc tgg ttc ctg gcc aag gtc tgg ggc aag acc ggc tcc aag     288
   Asp His Pro Trp Phe Leu Ala Lys Val Trp Gly Lys Thr Gly Ser Lys
                    85                  90                  95
   ctg tac ggc ccc cgc tcc ggc gct gac tac ctg gac aac cac aag cgc     336
   Leu Tyr Gly Pro Arg Ser Gly Ala Asp Tyr Leu Asp Asn His Lys Arg
               100                 105                 110
   ttc gcc ctg ttc tgc aag gcc gct att gag gct gcc cgc gtg ctg ccc     384
   Phe Ala Leu Phe Cys Lys Ala Ala Ile Glu Ala Ala Arg Val Leu Pro
           115                 120                 125
   ttc ggc ccc ggc gag gac tgc gtc ttc gtg gcc aac gac tgg cac tcc     432
   Phe Gly Pro Gly Glu Asp Cys Val Phe Val Ala Asn Asp Trp His Ser
       130                 135                 140
   gcc ctg gtg ccc gtc ctg ctg aag gac gag tac cag ccc aag ggc cag     480
   Ala Leu Val Pro Val Leu Leu Lys Asp Glu Tyr Gln Pro Lys Gly Gln
   145                 150                 155                 160
   ttc acc aag gcc aag tcg gtg ctg gct atc cac aac atc gcc ttc cag     528
   Phe Thr Lys Ala Lys Ser Val Leu Ala Ile His Asn Ile Ala Phe Gln
                   165                 170                 175
   ggc cgc atg tgg gag gag gct ttc aag gac acg aag ctg ccc cag gcc     576
   Gly Arg Met Trp Glu Glu Ala Phe Lys Asp Thr Lys Leu Pro Gln Ala
               180                 185                 190
   gcc ttt gac aag ctg gcc ttc tcg gac ggc tat gcc aag gtt tac act     624
   Ala Phe Asp Lys Leu Ala Phe Ser Asp Gly Tyr Ala Lys Val Tyr Thr
           195                 200                 205
   gag gcc acc ccc atg gag gag gac gag aag ccc ccg ctg acg gga aag     672
   Glu Ala Thr Pro Met Glu Glu Asp Glu Lys Pro Pro Leu Thr Gly Lys
       210                 215                 220
   acc tac aag aag atc aac tgg ctg aag ggt ggc att atc gcc gcc gac     720
   Thr Tyr Lys Lys Ile Asn Trp Leu Lys Gly Gly Ile Ile Ala Ala Asp
   225                 230                 235                 240
   aag ctg gtg act gtg tcg ccc aac tac gcg acc gag atc gct gcc gat     768
   Lys Leu Val Thr Val Ser Pro Asn Tyr Ala Thr Glu Ile Ala Ala Asp
                   245                 250                 255
   gcc gcc ggc ggt gtg gag ctg gac acc gtc atc cgc gcc aag ggc att     816
   Ala Ala Gly Gly Val Glu Leu Asp Thr Val Ile Arg Ala Lys Gly Ile
               260                 265                 270
   gag ggc att gtg aac ggc atg gac att gag gag tgg aac ccc aag acc     864
   Glu Gly Ile Val Asn Gly Met Asp Ile Glu Glu Trp Asn Pro Lys Thr
           275                 280                 285
   gac aag ttc ctg tct gcg ccc tac gac cag aac agc gtc tac gcc ggc     912
   Asp Lys Phe Leu Ser Ala Pro Tyr Asp Gln Asn Ser Val Tyr Ala Gly
       290                 295                 300
   aag gcc gcc gcc aag gag gcc ctg cag gcc gag ctg ggc ctg cct gtg     960
   Lys Ala Ala Ala Lys Glu Ala Leu Gln Ala Glu Leu Gly Leu Pro Val
   305                 310                 315                 320
   gac ccc acc gcc ccc ctg ttc gcc ttc atc ggc cgc ctg gag gag cag    1008
   Asp Pro Thr Ala Pro Leu Phe Ala Phe Ile Gly Arg Leu Glu Glu Gln
                   325                 330                 335
   aag ggt gtg gac atc atc ctg gcc gcc ctg ccc aag atc ctg gcc acc    1056
   Lys Gly Val Asp Ile Ile Leu Ala Ala Leu Pro Lys Ile Leu Ala Thr
               340                 345                 350
   ccc aag gtg cag atc gcc atc ctg ggt acc ggc aag gcc gcc tac gag    1104
   Pro Lys Val Gln Ile Ala Ile Leu Gly Thr Gly Lys Ala Ala Tyr Glu
           355                 360                 365
   aag ctg gtg aac gcc atc ggc acc aag tac aag ggc cgc gcc aag ggc    1152
   Lys Leu Val Asn Ala Ile Gly Thr Lys Tyr Lys Gly Arg Ala Lys Gly
       370                 375                 380
   gtg gtc aag ttc tcg gcg ccc ctg gcg cac atg ctc acc gcc ggc gcc    1200
   Val Val Lys Phe Ser Ala Pro Leu Ala His Met Leu Thr Ala Gly Ala
   385                 390                 395                 400
   gac ttc atg ctg gtg ccc tcg cgc ttc gag ccc tgc ggc ctg atc cag    1248
   Asp Phe Met Leu Val Pro Ser Arg Phe Glu Pro Cys Gly Leu Ile Gln
                   405                 410                 415
   ctg cac gcc atg cac tac ggt acc gtg ccc gtg gta gcc tcc acc ggc    1296
   Leu His Ala Met His Tyr Gly Thr Val Pro Val Val Ala Ser Thr Gly
               420                 425                 430
   ggc ctg gtc gac acc gtc                                            1314
   Gly Leu Val Asp Thr Val
           435
<210> 7
   <211> 438
   <212> PRT
   <213> Séquence artificielle
   <223> Description de la séquence artificielle: fragment de l'ADNc complet codant la GBSSI de Chlamydomonas reinhardtii
<400> 7
   Ala Leu Asp Ile Val Met Val Ala Ala Glu Val Ala Pro Trp Ser Lys
   1               5                   10                  15
   Thr Gly Gly Leu Gly Asp Val Thr Gly Gly Leu Pro Ile Glu Leu Val
               20                  25                  30
   Lys Arg Gly His Arg Val Met Thr Ile Ala Pro Arg Tyr Asp Gln Tyr
           35                  40                  45
   Ala Asp Ala Trp Asp Thr Ser Val Val Val Asp Ile Met Gly Glu Lys
       50                  55                  60
   Val Arg Tyr Phe His Ser Ile Lys Lys Gly Val His Arg Val Trp Ile
   65                  70                  75                  80
   Asp His Pro Trp Phe Leu Ala Lys Val Trp Gly Lys Thr Gly Ser Lys
                   85                  90                  95
   Leu Tyr Gly Pro Arg Ser Gly Ala Asp Tyr Leu Asp Asn His Lys Arg
               100                 105                 110
   Phe Ala Leu Phe Cys Lys Ala Ala Ile Glu Ala Ala Arg Val Leu Pro
           115                 120                 125
   Phe Gly Pro Gly Glu Asp Cys Val Phe Val Ala Asn Asp Trp His Ser
       130                 135                 140
   Ala Leu Val Pro Val Leu Leu Lys Asp Glu Tyr Gln Pro Lys Gly Gln
   145                 150                 155                 160
   Phe Thr Lys Ala Lys Ser Val Leu Ala Ile His Asn Ile Ala Phe Gln
   165 170 175
   Gly Arg Met Trp Glu Glu Ala Phe Lys Asp Thr Lys Leu Pro Gln Ala
   180 185 190
   Ala Phe Asp Lys Leu Ala Phe Ser Asp Gly Tyr Ala Lys Val Tyr Thr
   195 200 205
   Glu Ala Thr Pro Met Glu Glu Asp Glu Lys Pro Pro Leu Thr Gly Lys
   210 215 220
   Thr Tyr Lys Lys Ile Asn Trp Leu Lys Gly Gly Ile Ile Ala Ala Asp
   225 230 235 240
   Lys Leu Val Thr Val Ser Pro Asn Tyr Ala Thr Glu Ile Ala Ala Asp
   245 250 255
   Ala Ala Gly Gly Val Glu Leu Asp Thr Val Ile Arg Ala Lys Gly Ile
   260 265 270
   Glu Gly Ile Val Asn Gly Met Asp Ile Glu Glu Trp Asn Pro Lys Thr
   275 280 285
   Asp Lys Phe Leu Ser Ala Pro Tyr Asp Gln Asn Ser Val Tyr Ala Gly
   290 295 300
   Lys Ala Ala Ala Lys Glu Ala Leu Gln Ala Glu Leu Gly Leu Pro Val
   305 310 315 320
   Asp Pro Thr Ala Pro Leu Phe Ala Phe Ile Gly Arg Leu Glu Glu Gln
   325 330 335
   Lys Gly Val Asp Ile Ile Leu Ala Ala Leu Pro Lys Ile Leu Ala Thr
   340 345 350
   Pro Lys Val Gln Ile Ala Ile Leu Gly Thr Gly Lys Ala Ala Tyr Glu
   355 360 365
   Lys Leu Val Asn Ala Ile Gly Thr Lys Tyr Lys Gly Arg Ala Lys Gly
   370 375 380
   Val Val Lys Phe Ser Ala Pro Leu Ala His Met Leu Thr Ala Gly Ala
   385 390 395 400
   Asp Phe Met Leu Val Pro Ser Arg Phe Glu Pro Cys Gly Leu Ile Gln
   405 410 415
   Leu His Ala Met His Tyr Gly Thr Val Pro Val Val Ala Ser Thr Gly
   420 425 430
   Gly Leu Val Asp Thr Val
   435
<210> 8
   <211> 1593
   <212> ADN
   <213> Séquence artificielle
<220>
   <223> Description de la séquence artificielle: fragment de l'ADNc complet codant la GBSSI de Chlamydomonas reinhardtii
<220>
   <221> CDS
   <222> (1)..(1593)
<400> 8
   gcg ctg gac atc gtg atg gtt gct gct gag gtc gcc cct tgg tcc aag      48
   Ala Leu Asp Ile Val Met Val Ala Ala Glu Val Ala Pro Trp Ser Lys
   1               5                   10                  15
   acg ggc ggc ctg ggc gat gtg act ggt ggc ctg cct att gag ctg gtc      96
   Thr Gly Gly Leu Gly Asp Val Thr Gly Gly Leu Pro Ile Glu Leu Val
               20                  25                  30
   aag cgc ggc cac cgc gtc atg acc att gcc cct cgc tac gac cag tac     144
   Lys Arg Gly His Arg Val Met Thr Ile Ala Pro Arg Tyr Asp Gln Tyr
           35                  40                  45
   gct gac gcc tgg gac acc tcg gtg gtc gtg gac atc atg ggc gag aag     192
   Ala Asp Ala Trp Asp Thr Ser Val Val Val Asp Ile Met Gly Glu Lys
       50                  55                  60
   gtc cgc tac ttc cac tcc atc aag aag ggc gtg cac cgc gtg tgg att     240
   Val Arg Tyr Phe His Ser Ile Lys Lys Gly Val His Arg Val Trp Ile
   65                  70                  75                  80
   gac cac ccc tgg ttc ctg gcc aag gtc tgg ggc aag acc ggc tcc aag     288
   Asp His Pro Trp Phe Leu Ala Lys Val Trp Gly Lys Thr Gly Ser Lys
                   85                  90                  95
   ctg tac ggc ccc cgc tcc ggc gct gac tac ctg gac aac cac aag cgc     336
   Leu Tyr Gly Pro Arg Ser Gly Ala Asp Tyr Leu Asp Asn His Lys Arg
               100                 105                 110
   ttc gcc ctg ttc tgc aag gcc gct att gag gct gcc cgc gtg ctg ccc     384
   Phe Ala Leu Phe Cys Lys Ala Ala Ile Glu Ala Ala Arg Val Leu Pro
           115                 120                 125
   ttc ggc ccc ggc gag gac tgc gtc ttc gtg gcc aac gac tgg cac tcc     432
   Phe Gly Pro Gly Glu Asp Cys Val Phe Val Ala Asn Asp Trp His Ser
       130                 135                 140
   gcc ctg gtg ccc gtc ctg ctg aag gac gag tac cag ccc aag ggc cag     480
   Ala Leu Val Pro Val Leu Leu Lys Asp Glu Tyr Gln Pro Lys Gly Gln
   145                 150                 155                 160
   ttc acc aag gcc aag tcg gtg ctg gct atc cac aac atc gcc ttc cag     528
   Phe Thr Lys Ala Lys Ser Val Leu Ala Ile His Asn Ile Ala Phe Gln
                   165                 170                 175
   ggc cgc atg tgg gag gag gct ttc aag gac acg aag ctg ccc cag gcc     576
   Gly Arg Met Trp Glu Glu Ala Phe Lys Asp Thr Lys Leu Pro Gln Ala
               180                 185                 190
   gcc ttt gac aag ctg gcc ttc tcg gac ggc tat gcc aag gtt tac act     624
   Ala Phe Asp Lys Leu Ala Phe Ser Asp Gly Tyr Ala Lys Val Tyr Thr
           195                 200                 205
   gag gcc acc ccc atg gag gag gac gag aag ccc ccg ctg acg gga aag     672
   Glu Ala Thr Pro Met Glu Glu Asp Glu Lys Pro Pro Leu Thr Gly Lys
       210                 215                 220
   acc tac aag aag atc aac tgg ctg aag ggt ggc att atc gcc gcc gac     720
   Thr Tyr Lys Lys Ile Asn Trp Leu Lys Gly Gly Ile Ile Ala Ala Asp
   225                 230                 235                 240
   aag ctg gtg act gtg tcg ccc aac tac gcg acc gag atc gct gcc gat     768
   Lys Leu Val Thr Val Ser Pro Asn Tyr Ala Thr Glu Ile Ala Ala Asp
                   245                 250                 255
   gcc gcc ggc ggt gtg gag ctg gac acc gtc atc cgc gcc aag ggc att     816
   Ala Ala Gly Gly Val Glu Leu Asp Thr Val Ile Arg Ala Lys Gly Ile
               260                 265                 270
   gag ggc att gtg aac ggc atg gac att gag gag tgg aac ccc aag acc     864
   Glu Gly Ile Val Asn Gly Met Asp Ile Glu Glu Trp Asn Pro Lys Thr
           275                 280                 285
   gac aag ttc ctg tct gcg ccc tac gac cag aac agc gtc tac gcc ggc     912
   Asp Lys Phe Leu Ser Ala Pro Tyr Asp Gln Asn Ser Val Tyr Ala Gly
       290                 295                 300
   aag gcc gcc gcc aag gag gcc ctg cag gcc gag ctg ggc ctg cct gtg     960
   Lys Ala Ala Ala Lys Glu Ala Leu Gln Ala Glu Leu Gly Leu Pro Val
   305                 310                 315                 320
   gac ccc acc gcc ccc ctg ttc gcc ttc atc ggc cgc ctg gag gag cag     1008
   Asp Pro Thr Ala Pro Leu Phe Ala Phe Ile Gly Arg Leu Glu Glu Gln
                   325                 330                 335
   aag ggt gtg gac atc atc ctg gcc gcc ctg ccc aag atc ctg gcc acc     1056
   Lys Gly Val Asp Ile Ile Leu Ala Ala Leu Pro Lys Ile Leu Ala Thr
               340                 345                 350
   ccc aag gtg cag atc gcc atc ctg ggt acc ggc aag gcc gcc tac gag     1104
   Pro Lys Val Gln Ile Ala Ile Leu Gly Thr Gly Lys Ala Ala Tyr Glu
           355                 360                 365
   aag ctg gtg aac gcc atc ggc acc aag tac aag ggc cgc gcc aag ggc     1152
   Lys Leu Val Asn Ala Ile Gly Thr Lys Tyr Lys Gly Arg Ala Lys Gly
       370                 375                 380
   gtg gtc aag ttc tcg gcg ccc ctg gcg cac atg ctc acc gcc ggc gcc     1200
   Val Val Lys Phe Ser Ala Pro Leu Ala His Met Leu Thr Ala Gly Ala
   385                 390                 395                 400
   gac ttc atg ctg gtg ccc tcg cgc ttc gag ccc tgc ggc ctg atc cag     1248
   Asp Phe Met Leu Val Pro Ser Arg Phe Glu Pro Cys Gly Leu Ile Gln
                   405                 410                 415
   ctg cac gcc atg cac tac ggt acc gtg ccc gtg gta gcc tcc acc ggc     1296
   Leu His Ala Met His Tyr Gly Thr Val Pro Val Val Ala Ser Thr Gly
               420                 425                 430
   ggc ctg gtc gac acc gtc aag gag ggc gtc acc ggc ttc cac atg ggc     1344
   Gly Leu Val Asp Thr Val Lys Glu Gly Val Thr Gly Phe His Met Gly
           435                 440                 445
   gcc ctg aac ccc gac aag ctg gac gag gct gac gcc gac gcc ctg gcc    1392
   Ala Leu Asn Pro Asp Lys Leu Asp Glu Ala Asp Ala Asp Ala Leu Ala
       450                 455                 460
   gcc acc gtg cgc cgt gcc agc gag gtg ttt gcg ggc ggc cgc tac ccc    1440
   Ala Thr Val Arg Arg Ala Ser Glu Val Phe Ala Gly Gly Arg Tyr Pro
   465                 470                 475                 480
   gag atg gtg gcc aac tgc atc agc cag gac ctg tcc tgg tcc aag ccc    1488
   Glu Met Val Ala Asn Cys Ile Ser Gln Asp Leu Ser Trp Ser Lys Pro
                   485                 490                 495
   gcc cag aag tgg gag ggc ctg ctg gag gag gtg gtg tac ggc aag ggc    1536
   Ala Gln Lys Trp Glu Gly Leu Leu Glu Glu Val Val Tyr Gly Lys Gly
               500                 505                 510
   ggc gtg gcc acc gcc aag aag gag gag atc aag gtg ccc gtt gcc gag    1584
   Gly Val Ala Thr Ala Lys Lys Glu Glu Ile Lys Val Pro Val Ala Glu
           515                 520                 525
   aag atc ccc                                                        1593
   Lys Ile Pro
       530
<210> 9
   <211> 531
   <212> PRT
   <213> Séquence artificielle
   <223> Description de la séquence artificielle: fragment de l'ADNc complet codant la GBSSI de Chlamydomonas reinhardtii
<400> 9
   Ala Leu Asp Ile Val Met Val Ala Ala Glu Val Ala Pro Trp Ser Lys
   1               5                   10                  15
   Thr Gly Gly Leu Gly Asp Val Thr Gly Gly Leu Pro Ile Glu Leu Val
               20                  25                  30
   Lys Arg Gly His Arg Val Met Thr Ile Ala Pro Arg Tyr Asp Gln Tyr
           35                  40                  45
   Ala Asp Ala Trp Asp Thr Ser Val Val Val Asp Ile Met Gly Glu Lys
       50                  55                  60
   Val Arg Tyr Phe His Ser Ile Lys Lys Gly Val His Arg Val Trp Ile
   65                  70                  75                  80
   Asp His Pro Trp Phe Leu Ala Lys Val Trp Gly Lys Thr Gly Ser Lys
                   85                  90                  95
   Leu Tyr Gly Pro Arg Ser Gly Ala Asp Tyr Leu Asp Asn His Lys Arg
               100                 105                 110
   Phe Ala Leu Phe Cys Lys Ala Ala Ile Glu Ala Ala Arg Val Leu Pro
           115                 120                 125
   Phe Gly Pro Gly Glu Asp Cys Val Phe Val Ala Asn Asp Trp His Ser
       130                 135                 140
   Ala Leu Val Pro Val Leu Leu Lys Asp Glu Tyr Gln Pro Lys Gly Gln
   145 150 155 160
   Phe Thr Lys Ala Lys Ser Val Leu Ala Ile His Asn Ile Ala Phe Gln
   165 170 175
   Gly Arg Met Trp Glu Glu Ala Phe Lys Asp Thr Lys Leu Pro Gln Ala
   180 185 190
   Ala Phe Asp Lys Leu Ala Phe Ser Asp Gly Tyr Ala Lys Val Tyr Thr
   195 200 205
   Glu Ala Thr Pro Met Glu Glu Asp Glu Lys Pro Pro Leu Thr Gly Lys
   210 215 220
   Thr Tyr Lys Lys Ile Asn Trp Leu Lys Gly Gly Ile Ile Ala Ala Asp
   225 230 235 240
   Lys Leu Val Thr Val Ser Pro Asn Tyr Ala Thr Glu Ile Ala Ala Asp
   245 250 255
   Ala Ala Gly Gly Val Glu Leu Asp Thr Val Ile Arg Ala Lys Gly Ile
   260 265 270
   Glu Gly Ile Val Asn Gly Met Asp Ile Glu Glu Trp Asn Pro Lys Thr
   275 280 285
   Asp Lys Phe Leu Ser Ala Pro Tyr Asp Gln Asn Ser Val Tyr Ala Gly
   290 295 300
   Lys Ala Ala Ala Lys Glu Ala Leu Gln Ala Glu Leu Gly Leu Pro Val
   305 310 315 320
   Asp Pro Thr Ala Pro Leu Phe Ala Phe Ile Gly Arg Leu Glu Glu Gln
   325 330 335
   Lys Gly Val Asp Ile Ile Leu Ala Ala Leu Pro Lys Ile Leu Ala Thr
   340 345 350
   Pro Lys Val Gln Ile Ala Ile Leu Gly Thr Gly Lys Ala Ala Tyr Glu
   355 360 365
   Lys Leu Val Asn Ala Ile Gly Thr Lys Tyr Lys Gly Arg Ala Lys Gly
   370 375 380
   Val Val Lys Phe Ser Ala Pro Leu Ala His Met Leu Thr Ala Gly Ala
   385 390 395 400
   Asp Phe Met Leu Val Pro Ser Arg Phe Glu Pro Cys Gly Leu Ile Gln
   405 410 415
   Leu His Ala Met His Tyr Gly Thr Val Pro Val Val Ala Ser Thr Gly
   420 425 430
   Gly Leu Val Asp Thr Val Lys Glu Gly Val Thr Gly Phe His Met Gly
   435 440 445
   Ala Leu Asn Pro Asp Lys Leu Asp Glu Ala Asp Ala Asp Ala Leu Ala
   450 455 460
   Ala Thr Val Arg Arg Ala Ser Glu Val Phe Ala Gly Gly Arg Tyr Pro
   465                 470                 475                 480
   Glu Met Val Ala Asn Cys Ile Ser Gln Asp Leu Ser Trp Ser Lys Pro
                   485                 490                 495
   Ala Gln Lys Trp Glu Gly Leu Leu Glu Glu Val Val Tyr Gly Lys Gly
               500                 505                 510
   Gly Val Ala Thr Ala Lys Lys Glu Glu Ile Lys Val Pro Val Ala Glu
           515                 520                 525
   Lys Ile Pro
       530